(19) Europäisches Patentamt
European Patent Office
Office européen des brevets

(11) Publication number: **0 335 476 A2**

(12) # EUROPEAN PATENT APPLICATION

(21) Application number: 89201162.8

(22) Date of filing: 07.02.85

(30) Priority: 08.02.84 US 578115
08.02.84 US 587121
09.02.84 US 578122
07.09.84 US 648759
20.09.84 US 653515

(43) Date of publication of application: 04.10.89 Bulletin 89/40

(60) Publication number of the earlier application in accordance with Art.76 EPC: 0 170 697

(84) Designated Contracting States: AT BE CH DE FR GB LI LU NL SE

(51) Int. Cl.4: C12N 15/00 , C12P 21/00

(71) Applicant: CETUS CORPORATION
1400 Fifty-Third Street
Emeryville California 94608(US)

(72) Inventor: Gelfand, David
6208 Chelton Drive
Oakland California 94611(US)
Inventor: Lawyer, Frances Cook
6641 Saroni Drive
Oakland California 94611(US)
Inventor: Horn, Glenn
3 Admiral Drive No. F370
Emeryville California 94608(US)
Inventor: Greenfield, Lawrence
555 Pierce Street Unit 435
Albany California 94706(US)
Inventor: Nitecki, Danute
2296 Virginia Street
Berkeley California 94709(US)
Inventor: Kaplan, Donald
3301 Noeske Drive
Midland Michigan 48640(US)
Inventor: Piatak, Michael, Jr.
1120 Alfred Avenue
Walnut Creek California 94596(US)

(74) Representative: Bizley, Richard Edward et al
BOULT, WADE & TENNANT 27 Furnival Street
London EC4A 1PQ(GB)

(54) **Recombinant methods for the production of ricin a, ricin b, ricin or diphtheria toxin (dt)a or ab' fragment, suitable hosts and vectors therefor, and conjugates comprising ricin toxin a chain or diphtheria toxin.**

(57) Recombinant methods for the production of Ricin A, Ricin B, Ricin of Diphtheria toxin (DT)A or AB′ fragment are described together with suitable hosts and vectors. Immunotoxin conjugates comprising Ricin toxin A chain or Diphtheria toxin are also described.

EP 0 335 476 A2

```
                                                                       Msp I                                                                 Mbo I
                                                                       CCGGCGTTGCG TATCCAGTGGCTACACTCAGGTTGTAATGA TTGGGATGATGTACCTGATCTGAGAGCGAT

TAAAAACTCATTGAGGAGTAGGTCCCGATT GGTTTTTGCTAGTGAAGCTTAGCTAGCTTT CCCCATGTAACCAATCTATCAAAAAAGGGC ATTGATTTCAGAGCACCCTTATAATTAGGA

TAGCTTTACCTAATTATTTTATGAGTCCTG GTAAGGGGATACGTTGTGAGCAGAAAACTG TTTGCGTCAATCTTAATAGGGGCGCTACTG GGGATAGGGGCCCCACCTTCAGCCCATGCA
                     TerValLeu ValArgGlyTyrValValSerArgLysLeu PheAlaSerIleLeuIleGlyAlaLeuLeu GlyIleGlyAlaProProSerAlaHisAla
                           -31                            -21                            -11                             -1

GGCGCTGATGATGTTGTTGATTCTTCTAAA TCTTTTGTGATGGAAAACTTTTCTTCGTAC CACGGGACTAAACCTGGTTATGTAGATTCC ATTCAAAAAGGTATACAAAAGCCAAAATCT
GlyAlaAspAspValValAspSerSerLys SerPheValMetGluAsnPheSerSerTyr HisGlyThrLysProGlyTyrValAspSer IleGlnLysGlyIleGlnLysProLysSer
                            10                             20                             30                             40

GGTACACAAGGAAATTATGACGATGATTGG AAAGGGTTTTATAGTACCGACAATAAATAC GACGCTGCGGGATACTCTGTAGATAATGAA AACCCGCTCTCTGGAAAAGCTGGAGGCGTG
GlyThrGlnGlyAsnTyrAspAspAspTrp LysGlyPheTyrSerThrAspAsnLysTyr AspAlaAlaGlyTyrSerValAspAsnGlu AsnProLeuSerGlyLysAlaGlyGlyVal
                            50                             60                             70                             80

GTCAAAGTGACGTATCCAGGACTGACGAAG GTTCTCGCACTAAAAGTGGATAATGCCGAA ACTATTAAGAAAGAGTTAGGTTTAAGTCTC ACTGAACCGTTGATGGAGCAAGTCGGAACG
ValLysValThrTyrProGlyLeuThrLys ValLeuAlaLeuLysValAspAsnAlaGlu ThrIleLysLysGluLeuGlyLeuSerLeu ThrGluProLeuMetGluGlnValGlyThr
                            90                            100                            110                            120

GAAGAGTTTATCAAAAGGTTCGGTGATGGT GCTTCGCGTGTAGTGCTCAGCCTTCCCTTC GCTGAGGGGAGTTCTAGCGTTGAATATATT AATAACTGGGAACAGGCGAAAGCGTTAAGC
GluGluPheIleLysArgPheGlyAspGly AlaSerArgValValLeuSerLeuProPhe AlaGluGlySerSerSerValGluTyrIle AsnAsnTrpGluGlnAlaLysAlaLeuSer
                           130                            140                            150                            160
                                                                                                      Mbo I
GTAGAACTTGAGATTAATTTTGAAACCCGT GGAAAACGTGGCCAAGATGCGATGTATGAG TATATGGCTCAAGCCTGTGCAGGAAATCGT GTCAGGCGATCAGTAGGTAGCTCATTGTCA
ValGluLeuGluIleAsnPheGluThrArg GlyLysArgGlyGlnAspAlaMetTyrGlu TyrMetAlaGlnAlaCysAlaGlyAsnArg ValArgArgSerValGlySerSerLeuSer
                           170                            180                            190                            200

TGCATAAATCTTGATTGGGATGTCATAAGG GATAAAACTAAGACAAAGATAGAGTCTTTG AAAGAGCATGGCCCTATCAAAAATAAAATG AGCGAAAGTCCCAATAAAACAGTATCTGAG
CysIleAsnLeuAspTrpAspValIleArg AspLysThrLysThrLysIleGluSerLeu LysGluHisGlyProIleLysAsnLysMet SerGluSerProAsnLysThrValSerGlu
                           210                            220                            230                            240
```

DIPHTHERIA TOXIN CODING & AMINO ACID SEQUENCE

FIG. 1-1

# RECOMBINANT METHODS FOR THE PRODUCTION OF RICIN A, RICIN B, RICIN OR DIPHTHERIA TOXIN (DT) A OR AB′ FRAGMENT, SUITABLE HOST AND VECTORS THEREFOR, AND CONJUGATES COMPRISING RICIN TOXIN A CHAIN OR DIPHTHERIA TOXIN

## Technical Field

This invention relates to immunotoxins and to the production of toxin fragments therefor using recombinant technology. More specifically, the invention relates to producing ricin and diphtheria toxin fragments for use in improved immunotoxins.

## Background Art

Bacterial and plant toxins, such as diphtheria toxin (DT), Pseudomonas aeruginosa toxin A, abrin, ricin, mistletoe, modeccin, and Shigella toxin, are potent cytocides due to their ability to disrupt a critical cellular function. For instance, DT and ricin inhibit cellular protein synthesis by inactivation of elongation factor-2 and inactivation of ribosomal 60s subunits, respectively. Bacterial Toxins and Cell Membranes, Eds. Jelajaszewicz, J. and Wadstrom, T. (1978) Academic Press, p. 291. These toxins are extremely potent because they are enzymes and act catalytically rather than stoichiometrically. The molecules of these toxins are composed of an enzymatically active polypeptide chain or fragment, commonly called an "A" chain or fragment, linked to one or more polypeptide chains or fragments, commonly called "B" chains or fragments, that bind the molecule to the cell surface and enable the A chain to reach its site of action, e.g., the cytosol, and carry out its disruptive function. The act of gaining access to the cytosol is called variously "internalization", "intoxication", or "translocation". It is believed that the A chain must be timely liberated from the B chain--frequently by reduction of a disulfide bond--in order to make the A chain functional. These natural toxins are generally not selective for a given cell or tissue type because their B chains recognize and bind to receptors that are present on a variety of cells.

## Toxin Delivery Design Strategies

Derivatives of these bacterial and plant toxins have been prepared as therapeutic agents, primarily as antineoplastic agents, that are made specific for tumor cells or other target cells by replacing the native B chain(s) of the toxin molecule with a surrogate B chain that is specific for the tumor cell or adding a B chain having such specificity to the toxin molecule. Pharmacology of Bacterial Toxins, Eds. Drews, J. and Dorner, F. Pergamon Press. Synthetic cytotoxins containing the active fragment of a bacterial or plant toxin or a cytotoxic drug are called variously "chimeric toxins", "toxin conjugates", "cytotoxic conjugates", "hybrid toxins" or, when the surrogate B moiety is an antibody, "immunotoxins". Antibodies (polyclonal, monoclonal, and antigen binding fragments), hormones, lectins and various other compounds that are recognized by receptors on tumor cell surfaces have been used as surrogate B moieties. See European patent application publication no 0044167, and US Pats Nos 4,340,535, 4,350,626, 4,357,273, 4,359,457, 4,363,758, 4,368,149, and 4,379,145.

Surrogate B moieties have been chemically linked to toxin A chains by a variety of coupling agents. Heterobifunctional agents that include a disulfide group have been used extensively. The most popular of these agents is N-succidimidyl-3-(2-pyridyldithio)propionate (SPDP). Immun Rev, 62:185-216 (1982) reports using a longer disulfide-containing coupling agent, 7-aza-8-oxo-10-(2-pyridyldithio)decanoic acid, to investigate whether greater separation between the A chain and antibody would increase activity. Increased activity was observed in an acellular system but not on intact cells, and the report concluded that the longer disulfide containing linker was not advantageous. Replacement of the disulfide bridge by a stable thioether bridge using a derivative of 6-maleimidocaproic acid as a bifunctional coupling agent for an A-antibody conjugate caused a 99% loss of activity relative to the disulfide conjugate in an intact cellular system.

The toxicity of diphtheria toxin for human lymphoblastoid cells was increased by covalent linkage to anti-lymphoblastoid (anti-CLA 4 and anti-Daudi) globulin. (Ross, W.C.J., et al, Eur J Biodiem (1980) 104:381). Thyrotropin releasing hormone derivatives have also been conjugated to DT fragments such as CRM 45 to study the translocation function (Bacha, D., et al, J Biol Chem (1983) 238:1565). DT-A conjugated using SPDP to a monoclonal antibody against guinea pig hepatocarcinoma cells showed specific cytotoxicity in vitro and in vivo (Bernhard, M.I. et al, Cancer Res (1983) 43:4420).

Dextran, polyglutamic acid, and oligopeptides containing up to four amino acid residues have been used as spacer arm bridges between cytotoxic drugs and antibodies. Nature (1975) 255:487-488, FEBS letters (1980) 119:181-186, PNAS (1982) 79:626-629, and Immun Rev, 62:1-27 (1982). These conjugates were reported to have higher toxicity in vitro than drug coupled directly to antibody.

Moolten, F.L., et al, Immun Rev, (1982) 62:47-72 conclude that A chain-antibody conjugates may be more specific than native toxins, but lack much of the potency of the native toxin. They speculate that the loss of efficacy is because the internalization function is present in native B chains and surrogate B chains are inefficient substitutes as regards internalization. Use of toxins that lack a binding function but are otherwise intact, such as the (cross reacting mutant) CRM45 of DT or toxins whose binding function is chemically or enzymatically abrogated, are suggested, but no evidence of increased efficacy is given.

In summation, the efficacy of prior toxin-antibody conjugates has been highly variable due, inter alia, to variations in immunogenicity, target cell specificity, nonspecific toxicity, serum stability, effective concentration at the target cell, binding efficiency, and internalization efficiency. The improved immunotoxins of this invention provide (1) means for improving the efficacy of toxin-antibody conjugates and (2) novel toxin conjugates that include those means, by providing an A - surrogate B geometry which permits more facile translocation of the A portion into the target cell, and a more stable mode by which antibody can be linked to the A portion. This latter property prevents premature decomposition prior to translocation of the A portion into the target cell.

## Toxin Moieties

In addition to an improved configuration/performance outcome for the conjugates per se, the invention provides means for obtaining practical amounts of toxin moieties which are subject to convenient optimization in amino acid sequence using recombinant techniques.

Ricin and diphtheria toxins have been produced by the method of the invention in recombinant form.

Both ricin toxin (RT or ricin) and diphtheria are naturally occurring toxins composed of an enzymatically active, cytotoxic "A" amino acid sequence, and a "B" sequence, which is presumed to be responsible both for attaching the "A" sequence to a target cell to be killed, and to aid in the translocation of A fragment into the cytoplasm.

The "ricin" peptides of the present invention are derived from the seeds of Ricinus communis, commonly known as castor beans. Two similar proteins (often called lectins) are extractable from these seeds: the above-mentioned ricin and Ricin communis agglutinin (RCA). Both proteins contain A and B portions which do not comprise a single peptide but are joined by a disulfide link. The A portions of both ricin and RCA are capable of catalytically inactivating the large subunit of ribosomes in vitro and the mechanism of ricin for in vivo cytotoxicity is believed to reside in this capacity for ribosome inactivation. Ricin and RCA appear to be highly homologous but differences exist. RCA is dramatically less toxic, and appears to exhibit characteristics corresponding to those expected of a dimer of ricin.

The components of ricin and of RCA have been well characterized on the basis of the extracted materials, and their properties extensively reviewed: Olsnes, S., Perspectives in Toxicology, A. W. Bernheimer, Ed (1977) J. Wiley & Sons, NY, pp 122-147; Olsnes, S., et al, Molecular Action of Toxins and Viruses, Cohen, et al, Ed (1982) Elsevier, Amsterdam, pp 51-105. Ricin has an apparent molecular weight of 58,000 daltons and consists of the A chain with a molecular weight of 32,000 daltons and a B chain of molecular weight of 34,700 daltons. RCA is a tetramer which has two A subunits of molecular weight 32,000, and two B subunits of molecular weight 36,000 each. In their native environments, the B chains are generally glycosylated. The A and B subunits of both ricin and RCA are linked only by a single disulfide bond, and not by peptide linkage unlike, for example diphtheria toxin which is found as a single chain peptide. It is also known that both ricin and RCA, though having separate peptides for A and B portions, are each derived from a single chain precursor in each case (Butterworth, H. E., et al, Eur J Biochem (1983) 137:57). The precursor is shown hereinbelow to contain a sequence of 12 amino acids between the A chain (amino terminal) and B chain (carboxy terminal) sequence. It is assumed that upon excision of the dodecameric intervening peptide, the A and B chains remain linked through the single disulfide bond.

Native ricin A exists in a number of homologous but not exactly identical forms depending on the plant variety used as source, but even protein derived from a single plant exhibits more than one primary structure. Recombinantly produced ricin A, of course, permits production of a single desired amino acid sequence, and makes possible an exploration of the structural features required for its activity.

Diphtheria toxin (DT) also contains A and B peptides and is secreted from Corynebacterium diphtheriae as a single polypeptide chain containing 535 residues, of which the amino terminal, approximately 193

residue sequence, is considered the A fragment, and the carboxyterminal, approximately 342 residue sequence, is considered the B component. The amino acid sequence of the naturally occurring diphtheria toxin, deduced from the coding sequence, is included in Figure 1. In this naturally occurring protein, the A chain is considered to end roughly after the arginine at position 193, and the B chain to pick up with the serine at position 194. It is believed that cleavage after one of the Arg residues at positions 190, 192 or 193 (trypsin is capable of thus cleaving in vitro) is required for intoxication of cells. This alteration is believed to be followed by breakage of the disulfide bond between cysteine at position 186 and cysteine at position 201 so as completely to free the A fragment from B fragment. Once released inside the cell, it is known that a single molecule of fragment A can be lethal for an individual cell (Yamaizumi, M., et al, Cell (1978) 15:245). This is believed to be attributable to the catalytic nature of the cytotoxic effect.

It is believed that the hydrophobic sequence of approximately 32 amino acids in the vicinity of residue 350 is responsible for the translocation properties of B chain. A "cross reacting mutant" secretes "CRM 45," a modified form of DT which contains approximately half of the B portion at the N-terminal end. CRM 45 is capable of insertion into artificial lipid bilayers under appropriate in vitro conditions to form ion conductive channels (Kagan, B. L., et al, Proc Natl Acad Sci (USA) (1981) 4950; Donovan, J. J., et al, Ibid - (1981) 78:172; Kayser, B. et al, Biochem Biophys Res Commun (1981) 99:358). Further, the 25 amino acid region referred to above was earlier identified by Lambott, P., et al, J Cell Biol (1980) 8:837, as especially hydrophobic and to resemble transverse lipid-associating domains found in intrinsic membrane proteins.

Figure 1 also shows that the DT protein contains a leader sequence responsible for secretion from Corynebacter, presumably initiated at the GTG codon at position -25. The invention utilizes portions of the gene cleavable at MspI (fragment 2) and MboI (fragment 1); these portions contain at least part of the control sequences native to DT, and will be deleted in most of the vectors of the invention.

The gene sequence has been disclosed by Ratti, G., et al, Nucleic Acids Res (1983) 11:6589; by Leong, D., et al, Science (1983) 220:515 and by Greenfield, L., et al, Proc Natl Acad Sci (USA)(1983) 80:6853-6857. Expression in E. coli of Mbo (fragment 1) and Msp (fragment 2) has been detected at low levels in E. coli transformed with pBR322 derived plasmids containing these inserted sequences. (Tweten, R. K., et al, J Bacteriol (1983) 156:680.)

The invention herein, by enabling the production of these toxin chains using predictable, efficient, and economic procedures which, further, permit directed modification, permits their use in practical and improved ways not before possible. Such uses include construction of immunotoxin conjugates for therapy, whereby the specific binding moiety focuses the cytotoxicity of the toxin on undesirable cells, such as tumors.

## Summary of the Invention

The present invention provides novel toxin conjugates which are peculiarly effective in recognizing target cells and in effecting their demise. It also provides toxin and other components of these conjugates. The conjugates comprise a cytotoxic component which is an enzymatically active portion of the molecule, capable of killing cells in which it is internalized, a specific binding moiety, typically an antibody or fragment of an antibody, which is capable of recognizing a specific antigenic determinant or target cell, and a spacer which provides the proper geometry between the cytotoxic component and the binding fragment. These conjugates represent an improved delivery system for naturally occuring or modified cytotoxins ("A chains") which in their natural environment are bound to a relatively non-specific binding component "B chain" (which is generally not an antibody). The naturally occuring toxins also contain, within the A-B chain fusion, sequences which are capable of cleavage intracellularly, ie which effect cleavage once the cytotoxic component has migrated to within the target cell, but are stable prior to this entry, and a translocation region which permits the desired cytotoxic A chain to enter the target cell. This intracellular cleavage exposes and labilizes the link (typically disulfide) between the A and B chain.

In the conjugates of the present invention, the non-binding portion of the molecule is constructed so as to retain the foregoing desired cytotoxic, intracellularly cleavable/extracellularly stable, and translocation properties of the natural molecule in a geometry suitable for connecting to a "binding fragment" and permitting activity. Thus, typically, the conjugates of the invention include: antibody or fragment of an antibody which is covalently linked, preferably through a bifunctional linker to a non-binding entity. The non-binding entity is an amino acid sequence which contains, to serve as the cytotoxic component, an enzymatically active site, an intracellularly cleavable/extracellularly stable site and translocation sequence and, as an extension of this amino acid sequence, a further sequence which serves as a spacer between the cytotoxic component and the binding fragment to be linked. The non-binding portion of the toxin

conuugates of the invention may be supplied using recombinant techniques.

The spacer confers the additional advantage of enhanced solubility in some instances where intermediates for desired immunotoxin may be sufficiently insoluble to interfere with their purification. By supplying a relatively more soluble portion, or by altering the conformation of the remainder of the molecule, the spacer thus permits more options in the selection of components and conjugation methods.

The invention, therefore, relates to these conjugates and to the novel components used for their construction including especially those formed by recombinant means. Both the spacer segment and the non-binding portion of the conjugate formed by fusion of the spacer with a cytotoxic component, ie, an extended spacer containing the cytotoxic component are aspects of the invention.

Thus, in one aspect, the invention relates to novel polypeptides (the spacer) consisting essentially of at least one rigid amino acid sequence bracketed by two flexible amino acid sequences--ie, components of the formula $(\text{flex-rigid})_n\text{flex}$, where "flex" represents the flexible sequence and "rigid" the rigid sequence. In a preferred embodiment "flex" is about 4 to 8 amino acids each individually selected from the group consisting of threonine, serine and glycine and "rigid" is 4 to 8 proline residues. Such novel polypeptides are useful as spacers for separating the cytotoxic component and the target cell binding component of a toxin conjugate.

In the alternative, the spacer may be described in functional terms and comprises an amino acid sequence that:

(a) is substantially stable in serum;

(b) is substantially nonhydrophobic so as not to affect adersely the water solubility of the toxin conjugate;

(c) is at least about 15 A long;

(d) has a substantially extended structure; and

(e) is sufficiently flexible to permit three dimensional movement of the cytotoxic component and the target cell binding component.

The spacer may also be a solubilization conferring sequence of amino acids, as set forth hereinbelow.

The foregoing spacer descriptions are not mutually exclusive, but are alternative characterizations of successful peptide sequences. The spacer may further contain a reactive amino acid residue proximate one of its termini so as to provide a conjugation site for the additional binding fragment.

Other aspects of the invention are fused polypeptides for use in making toxin conjugates comprising:

(a) a cytotoxic portion; and

(b) one of the above-described polypeptide spacers, as well as recombinant peptides which are useful cytotoxic moieties for the conjugates.

The invention also concerns the DNA sequences encoding the spacer, toxin fragments, or fused polypeptide (cytotoxic/spacer); expression vectors containing these DNA sequences, and cells transformed with these vectors.

Still another aspect of the invention is a toxin conjugate which comprises:

(a) a cytotoxic portion;

(b) a polypeptide spacer bound to the cytotoxic component; and

(c) a target cell binding moiety conjugated to the cytotoxic component via the polypeptide spacer.

(The "cytotoxic portion" includes a site for intracellular cleavage within a serum stable domain and an internalization facilitating domain.)

In summary, the invention is designed to provide a toxin conjugate which has the appropriate geometry for translocating the cytotoxic fragment into the target cell, the capacity to retain its binding fragment prior to such translocation, and/or the ability to solubilize the cytotoxic portion. In one aspect of the invention, the spacer is designed so as to permit the cytotoxic portion of the molecule ready access to the cell membrane. As the size of a typical antibody (binding) fragment is very much greater than that of most cytotoxic fragments, there is considerable steric hinderance of the access to the cell membrane by the cytotoxic portion imposed by the sheer bulk of the antibody or antibody fragments. Accordingly, the conjugate toxins of the invention have a geometry schematically represented in (a) rather than that given without the spacer (b).

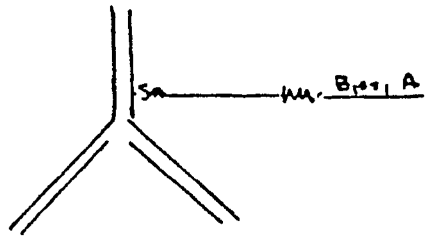

(a)

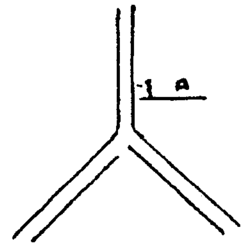

(b)

In order to effect this translocation, the spacer needs to be sufficiently flexible to allow the A portion to reach the cell membrane, and sufficiently extended to permit it to have sufficient reach.

The performance of the conjugated toxin can also be improved by securing the binding portion tightly to the remainder of the molecule with respect to a serum environment. This is done in one preferred embodiment of the invention, by utilizing a linker between the antibody and spacer which employs bonds not readily cleaved by reducing agents or by hydrolysis under extracellular conditions. The cleavage of the A-chain analog (ie the enzymatically active site) from the other end of the spacer arm can be achieved by permitting the linkage at the A end of the spacer to be more readily cleavable. This can best be done by retaining a portion of the original B chain of the native toxin in linking the A portion to the spacer. In this manner, the normal extracellular-resistant/intracellular-cleavable configuration of the A-B chain pair is retained but with the loss of the binding capacity of the original B portion. Thus, the specific binding capability conferred by the antibody on the conjugate is not lost prior to intracellular incorporation of the cytotoxic fragment. Such flexibility in the nature of the non-binding moieties available is provided through the recombinant methods of the invention.

Since the binding portion confers specific recognition of certain target cells, the conjugated toxins are useful in killing specified undesirable cells within a subject. Thus, in two other aspects, the invention relates to pharmaceutical compositions containing effective amounts of these toxins and to methods of treatment employing them.

## Brief Description of the Drawings

Figure 1 shows the nucleotide sequence of the DT gene along with the corresponding deduced amino acid sequence.

Figure 2 shows the construction of an Msp-Spacer arm clone, pMspSA2.

Figure 2 shows the construction of two Msp-Spacer fragment expression vectors wherein the coding sequence is under the control of the $P_L$ promoter: $pP_LMspSA$ and $pP_LOPMspSA2$.

Figure 4 shows the construction of pTrpSmlMbo.

Figure 5 shows the construction of pPLOPSau, an expression vector for diphtheria toxin A fragment.

Figure 6 shows the construction of pPLOPMspRT, an expression vector for the DT-A B′ fragment.

Figure 7 shows the construction of pPLOPMspCys, an expression vector for the DT-A B′ fragment with a cysteine residue at its C-terminus.

Figure 8 shows the results of polyacrylamide gel electrophoresis performed on extracts of cells transformed with expression vectors for diphtheria toxin fragments.

Figure 9 shows the nucleotide sequences of cloned inserts hybridizing to probe obtained from the cDNA library based on castor beam mRNA.

Figure 10 shows the amino acid sequence of ricin B in comparison to the sequence deduced from the ricin B encoding clone pRTB-5.

Figure 11 shows the construction of pRTB151 and pRTB601, vectors containing the ricin B encoding sequence.

Figure 12 shows junction sequences for plasmids containing the ricin B encoding fragment.

Figure 12a shows the junction region for pRTB601; figure 12b for pRTB236; and figure 12c, the junction region for pRTB514.

Figure 13 shows the construction of expression vectors for the production of ricin B fragment: pRTB514, pRTB704, and pRTB907.

Figure 14 shows the nucleotide sequence and the deduced amino acid sequence for the ricin A encoding cloning insert pRA123. The amino acid sequence determined from the protein of the ricin A fragment is shown for comparison.

Figure 15 shows the nucleotide sequence and deduced amino acid sequence derived from the inserts of pRTA115 and pRTA4 and pRTA5 which encode ricin agglutinin A. The amino acid sequence of ricin toxin A is shown for comparison.

Figure 16 shows the results of Western blot on extracts of cells transformed with plasmids encoding ricin A fragment.

Figure 17 shows the results of Western blot conducted on extracts of cells transformed with expression vectors encoding ricin B fragments.

## Modes for Carrying Out the Invention

### A. Definitions

As used herein the terms "fragment", "domain", and "region" and "portion" are interchangeable and refer to functionally but not necessarily physically distinct portions of the conjugated toxin molecule.

The term "specificity" as used to describe the target cell binding portion of the conjugated toxin means that the moiety has the ability to distinguish a target cell from other cells, typically due to the presence of a cell surface receptor that is unique to the target cells.

The term "selective" means that the cytotoxin has the ability to kill target cells preferentially, typically due to the specificity of the binding moeity or a differential in the respective quantities of receptors on target cells and other cells.

The term "target cells" means those cells which the cytotoxin is intended to kill. Although target cells will usually be tumor cells, they may be nontumourous cells whose selective destruction is desired for therapeutic or diagnostic purposes (for instance in certain assays of peripheral blood cells it is desired to selectively kill one or the other of B cells or T cells). The target cells may be present in living organisms or they may be preserved or maintained in vitro. The cells may be individual or associated to form an organ.

As used herein the term "polypeptide" or "protein" refers to an amino acid polymer. It is understood that such polymers exist in a variety of ionization states dependent on ambient pH, and that they may, further, be associated with accessory moieties--eg, glycosylated, phosphorylated or conjugated to lipids. The peptides or proteins of the invention include all such forms, including unassociated forms.

The term "intracellular" is intended to include intracytoplasmic sites and sites within vesicular compartments such as lysosomes.

"Target cell binding portion" refers to that fragment of the toxin conjugates of the invention which binds specifically to the target cells. In this invention the "binding" portion is an antibody or fragment thereof. The "non-binding" portion or fragment of the toxin conjugate comprises the remainder of the molecule. It thus includes the cytotoxic portion and the spacer.

The term "cytotoxic portion" includes the "enzymatically active polypeptide fragment" ie an A-fragment analogous sequence, the intracellularly cleavable/extracellularly stable domain and the translocation domain.

As used herein with respect to the construction of the spacer domain, "reactive amino acid residue" refers to an amino acid (or residue) which provides a site for linking or conjugation, as further described below.

"Solubilizing conferring" sequence of amino acids in the context of the invention refers to a form of spacer sequence which continues at the (preferably C) terminus of the cytotoxic portion and which results in the fused protein being soluble in aqueous media, even when the cytotoxic portion is itself insoluble or when the cytotoxic portion would otherwise be rendered insoluble by the addition of a cysteine residue.

As used herein "DT-A fragment" (diphtheria toxin-A fragment) refers to an approximately 193 residue amino acid sequence which is capable of ADP-ribosylation of EF-2, and which is substantially similar to the sequence in Figure 1 between amino acid 1 and amino acid 192 or 193.

"DT-B fragment" is an approximately 343 residue amino acid sequence which is substantially similar to the sequence approximately between amino acids 193 and 535 in Figure 1.

"DT-A-B′ fragment" is an approximately 384 residue amino acid sequence which is capable of ADP-ribosylation of EF-2, which contains both a translocation sequence and an intracellular cleavage site within an extracellularly stable domain, and which is substantially similar to the sequence shown in Figure 1 between amino acid 1 and amino acid 384.

"Msp fragment" (fragment 2) refers to an approximately 1454 bp segment which contains all of the A portion and part of the B portion--i.e, a DT-A-B′ fragment along with at least part of native promoter, ribosome binding site and secretory leader sequence as shown in Figure 1. "Msp terminator" refers to a nucleotide sequence (synthetically derived herein) which encodes one additional amino acid past the Msp cleavage point plus a stop codon. The resultant (Msp + Msp terminator) encodes a "DT-A-B′ fragment."

"Mbo fragment" (fragment 1) refers to an approximately 831 bp resultant of an Mbo digest of the DT gene. It contains the coding sequence of amino acids 1-193 (the A chain) and leader and at least partial native control sequences (see Fig. 1). "Mbo terminator" refers to a nucleotide sequence (synthetically derived herein) which encodes an additional six amino acids past the Mbo cleavage plus a stop codon. The resultant (Mbo + Mbo terminator) encodes a "DT-A fragment."

"Native" promoter, ribosome binding site, or control sequences refer to those which are normally found in association with, and operably linked to, the DT coding sequence. "Leader" sequence refers to that portion of the DNA which encodes the native pre-sequence, responsible for secretion of the mature protein.

As used herein, "ricin A" refers to a protein whose amino acid sequence is substantially similar to that of the ricin A peptide which is extractable from castor bean seeds. The ricin A of castor beans is approximately 265 amino acids in length and has a molecular weight of approximately 32,000 daltons. However, it is known that the precise sequence varies depending on the variety of bean, and, indeed that at least two slightly different forms of ricin A may be present in a single variety.

"Ricin B" refers to a protein whose amino acid sequence is substantially similar to that of the ricin B peptide which is extractable from castor bean seeds. The ricin B of castor beans is approximately 260 amino acids in length and has a molecular weight of approximately 34,700 daltons; as with ricin A, it is known that the precise sequence varies depending on the variety of bean.

"Substantially similar" means that the protein in question must be approximately the same length (arbitrarily within around 10%) but, more importantly, must retain the capacity of reference chain to carry out its native biological activity. It is well known that some small alterations in protein sequence may be possible without disturbing the functional abilities of the protein molecule, although other modifications are totally destructive. It is not currently possible to predict with any assurance into which category a particular alteration will fall. The definition herein permits any modifications which are in the first category. Such alterations could result from chance mutations in the gene sequence or from deliberate alterations thereof.

Further, as is well known, protein sequences may be modified by post-translational processing such as association with other molecules, for example, glycosides, lipids, or such inorganic ions as phosphate. The ionization status will also vary depending on the pH of the medium or the pH at which crystallization or precipitation of the isolated form occurs. Further, the presence of air may cause oxidation of labile groups, such as -SH. Included within the definition of the peptides defined herein are all such modifications of a particular primary structure--ie, eg, both glycosylated and non-glycosylated forms, neutral forms, acidic and basic salts, lipid or other associated peptide forms, side chain alterations due to oxidation or derivatization, and any other such modifications of an amino acid sequences which would be encoded by the same genetic codon sequence.

"Ricin" refers to peptides which contain both A and B chains, defined respectively as set forth herein. The peptide may mimic the native peptide in that the A and B proteins are linked only by a disulfide, or may contain the intermediate dodecamer or other short peptide sequence between the A and B chains.

"Impurities" as used in describing the proteins prepared by the method of the invention refers to materials normally associated with these proteins as produced in the castor bean seeds or in the C. diphtheriae, which are not included among the protein modifications above. Accordingly, "impurities" for ricin refers to agglutinin as well as to other castor bean cellular materials which ordinarily are associated with ricin or ricin A non-specifically; with respect to ricin A per se, "impurities" includes ricin B. "Impurities" for DT fragments refers to materials, including DNA fragments, normally occurring in C. diphtheriae.

"Operably linked" when used in describing DNA sequences refers to juxtaposition in such a way that the functionality of the sequences is preserved. Thus, for example a coding sequence "operably linked" to control sequences is positioned so that the these sequences are capable of effecting the expression of the coding sequence.

"Control sequence refers to those DNA sequences which control initiation and termination of transcription and translation. In procaryotic systems, for example, control sequences comprise promoter or promoter/operator and nucleotides encoding a ribosome binding site; in eucaryotes, promoters, terminators and enhancers appear to be involved.

"Recombinant host cells" refers to cells which have been transformed with DNA sequences constructed by recombinant techniques. Such reference includes both the cells as separated, for example by filtration or as a centrifugation pellet, and to cultures of these cells. Indeed, "cells" and "cell cultures," where the

context so permits, are used interchangeably herein. Also included in particular references to "cells" are the progeny thereof. Such progeny are either of the same genomic structure, or contain a modified genome due to inherent instability, intentional mutation, or chance alterations in the genomic structure.

## B. Components of Conjugate Toxins

Toxin conjugates have classically been conceptualized as combinations of an A fragment and a surrogate B moiety, attached through linking group that binds these fragments via a labile bridge such as a disulfide bridge. The rationale behind this conceptualization was that the function of the B moiety was primarily to bind the conjugate to the cell surface via interaction with a cell surface receptor and that the disulfide bridge provided means for joining the A fragment and B moiety tht could be broken *in vivo* to liberate the A fragment. The present invention is based on an expanded conceptualization of the mode of operation of cytotoxic conjugates and takes into account factors including:

- the spatial relationship between the cytotoxic moiety and the binding moiety,
- the role of the non-A portion of the conjugate in internalization, and
- the extracellular lability of the bond between the cytotoxic moiety and the binding moiety.

In the most preferred embodiment of the present invention with respect to toxin conjugates each of these factors is taken into account in synthesizing a novel toxin conjugate having five functional elements (which may overlap in the structure of the conjugate):

(1) an enzymatically active domain capable of the toxic activity of the A-fragment;

(2) an intracellular cleavage site within a extracellularly stable (or serum stable) domain that provides a site for liberating the enzymatically active domain from the remainder of the toxin conjugate after the toxin conjugate has been internalized;

(3) a translocation or internalization facilitating domain that acts as an adhesive to anchor the toxin conjugate molecule to the target cell membrane and/or to facilitate internalization;

(4) the novel polypeptide spacer described above; and

(5) a target cell binding moiety that recognizes and binds to a receptor on the target cell surface which by virtue of the receptor's quantity or nature causes the toxin conjugate to localize selectively on target cells relative to other cells.

In the most preferred embodiment the binding moiety is bound to the non-binding portion of the mol ecule (ie the remaining elements 1-4) by a chemical bond that is substantially resistant to cleavage *in vivo* - (either extracellularly or intracellularly) thereby preserving the specificity of the molecule once it is administered, while the intracellular cleavage site within a serum stable domain is provided at the enzymatically active or "A" end of the spacer.

In order to effect this linkage using one preferred embodiment the cytotoxic portion of the toxin needs to be provided with a free cysteine residue, capable of forming a thio-ether linkage through a suitable bifunctional linker. The presence of this cysteine may interfere with the solubility of the toxic portion, and the spacer then serves the additional function of providing solubilization. To accomplish this purpose, the detailed "spatial" requirements relating to rigidity and flexibility of the sequence are not required; all that is needed are the hydrophilic or neutral solubility properties of the chain.

### B.1. The Enzymatically Active Domain

The enzymatically active fragment of the conjugate may be the A chain of a bacterial or plant toxin or be a natural protein that has enzymatic activity similar to the A chain of a bacterial or plant toxin. As used herein the terms "enzymatically active fragment" and "A chain" are intended to include such similar acting natural proteins. Examples of such A chains are diphtheria A chain, exotoxin A chain (from *Pseudomonas aeruginosa*), a ricin A chain, abrin A chain, modeccin A chain, alpha-sarcin, *Aleurites fordii* proteins, dianthin proteins, *Phytolacca americana* proteins (PAP I, PAP II, and PAP-S), momordin, curcin, crotin, gelonin, mitogellin, restrictocin, phenomycin, and enomycin.

The derivation of an A chain from a whole natural toxin molecule involves breaking the bond(s) between the A and B chain(s) (eg, reducing the disulfide bonds(s) between the A and B chain(s) with an appropriate reducing agent, such as 2-mercaptoethanol or dithiothreitol) and isolating the A chain from the B chain(s). The isolation may be carried out chromatographically or by other conventional protein fractionation techniques. The natural proteins that have enzymatic activity similar to the A chains of the natural protein

toxins may be isolated from their sources, typically plant parts, by conventional protein extraction and isolation techniques. Alternatively, and preferably, these amino acid sequences are derived using recombinant techniques.

### B.2. The Intracellular Cleavage Site

The intracellular cleavage site domain of the cytotoxin is preferably one that functions in a way that mimics the manner in which a natural toxin liberates its A chain. Three cleavage mechanisms are postulated currently: (1) proteolysis either enzymatic or chemical (eg, pH change), (2) disulfide reduction, and most commonly (3) a combination of (1) and (2). The cleavage site(s) of such domains is substantially stable extracellularly and is labile intracellularly. In the cleavage mechanisms involving proteolysis and disulfide reduction, extracellular stability is probably due to the position of the disulfide cleavage site in the extracellular tertiary structure of the conjugate. That is, the site is not exposed to cleavage agents in the extracellular environment, but is exposed in the intracellular environment due to a change in the tertiary structure of the molecule. Cleavage sites whose lability depends on pH are stable in extracellular environments, eg, blood, having a substantially neutral pH. The lower pH of certain intracellular compartments (eg, within a lysosome or receptosome) makes the site labile. The cleavage site domain comprises a sequence of amino acids that includes residues that are susceptible to proteolysis such as by lysosomal proteases. When disulfide reduction is involved the sequence will obviously contain a disulfide bridge formed by spaced cysteine residues. When both proteolysis and reduction are involved the A chain is liberated from the remainder of the toxin conjugate by proteolysis at the sensitive residues that causes a nick or break in the polypeptide backbone of the molecule and reduction of the disulfide bond. Examples of residues that are lysosomal protease sensitive are arginine, lysine, phenylalanine, tyrosine, and tryptophan.

In a preferred embodiment, this cleavage site in a serum stable domain is proximate the enzymatically active domain and forms an extension of the C-terminus thereof. Such a configuration can be provided most conveniently by providing an extended "A" fragment from a naturally occurring toxin into a portion of the natural B chain. Recombinant techniques are best suited to this embodiment, since convenient peptide cleavage techniques specific for the desired extension may not exist for a given toxin. However, the coding sequence can be cleaved and modified so as to encode for just the desired fragment.

### B.3. The Translocation Domain

The internalization facilitating or "translocation" domain of the conjugate participates in interacting with the cell or vesicle wall whereby the wall is penetrated, opened, or disrupted to enable the conjugate to reach the intracellular compartment. The internalization facilitating domain may be identical to, or substantially similar in, amino acid content and sequence to an internalization facilitating domain of a bacterial or plant toxin or a polypeptide that is known to interact similarly with cell membranes. In the case of DT, the domain has been identified as being "hydrophobic" and located within the B fragment. The sequence of that segment possibly includes the following:
Val-Ala-Gln-Ala-Ile-Pro-Leu-Val-Gly-Glu-Leu
Val-Asp-Ile-Gly-Phe-Ala-Ala-Tyr-Asn-Phe-Val
Glu-Ser-Ile-Ile-Asn-Leu-Phe-Gln-Val-Val
Examples of polypeptides that are known to have a similar interaction with cell membranes are melittin:
Gly-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln
and delta lysine:
Met-Ala-Gln-Asp-Ile-Ile-Ser-Thr-Ile-Gly-Asp-Leu-Val-Lys-Trp-Ile-Ile-Asp-Thr-Val-Asn-Lys-Phe-Thr-Lys-Lys.
The position of the domain in the toxin conjugate molecule may vary. It will usually be located adjacent to the carboxy terminus of the A chain but may also be located adjacent to the amino terminus of the A chain. More than one internalization facilitating domain may be included in the conjugate if desired.

Since this domain is positioned adjacent the A chain, its inclusion in the toxin conjugate is most conveniently accomplished by recombinant DNA techniques. In DT, for example, the extension of the polypeptide sequence at the C terminus approximately 193 amino acids into the B chain provides such an internalization domain. Therefore, cloning and expression of the coding sequence for this portion of the DT toxin would provide the desired configuration. Alternatively, the oligonucleotides encoding known internalizing domains such as those exemplified above can be ligated to the nucleotides encoding the desired A fragment for cloning and expression.

B.4. The Spacer

The spacer comprises a sequence of amino acids that can be described in one of three non-mutually exclusive ways. In any case, the end of the spacer that attaches to the binding moiety may have a "reactive amino acid" residue at or near the terminus that provides a site for conjugating the binding moiety. For instance, if a reactive amino group at or near the end of the spacer is desired one or more lysine residues may be located near one terminus of the spacer fragment. If a reactive sulfhydryl group is desired, a cysteine residue may be situated similarly.

In one aspect, the spacer is described as having one or more extended structure portions (segments in which the peptide bond angles are enlarged) linked by segments that are flexible. Each end of the spacer terminates with a flexible segment. The spacer thus links the target cell binding moiety to the remainder of the molecule with the extended structure portion(s) serving to separate the binding moeity and the remainder of the molecule and the flexible segments permitting three dimensional movement of the binding moiety and the remainder of the molecule. Thus the spacer can be described as being formed from a series of extended structure portions in tandem with intermediate flexible regions (the flexible regions lie on either side of the extended structure regions), ie, has the general formula $(flex\text{-}rigid)_m$ flex. The extended (rigid) portion(s) of the spacer is preferably formed of a series of 4 to 8 prolines while the flexible portions are preferably composed of 4-8 amino acid residues each selected individually from the group consisting of serine, glycine, or threonine. The series of prolines for a left-handed proline II helix. These preferred spacers (less terminal reactive residue) may be represented by the formula

$(F\text{-}(Pro)_n)_mF$

wherein F represents a flexible sequence composed of amino acids each selected independently from the group consisting of serine, glycine, or threonine, n is an integer form 4 to 8 inclusive, and m is an integer from 1 to 4 inclusive. The flexible sequences may be the same or different. A particularly preferred spacer domain (less terminal reactive residue) is defined by the sequence

Gly-Thr-Gly-Ser-Gly-$(Pro)_n$-Ser-Gly-Ser-Gly-Thr where n is an integer from 4 to 8, inclusive, most preferably 6. A particularly preferred terminal reactive residue is cys.

In a second aspect, the spacer is substantially nonhydrophobic so that it has a neutral or positive effect on the water solubility of the conjugate. The spacer's hydrophobicity may be determined by summing the hydrophobicities of the individual amino acids (measured by partition coefficient tests) of which it is composed. A substantially nonhydrophobic sequence will measure neutral or hydrophilic. The hydrophilic nature of this segment will also place it on the surface of the configured molecule, thereby permitting accessibility for conjugation.

If the function of a particular spacer is merely to provide solubility during processing to a cytotoxic portion amino acid sequence, this property is, indeed, the only property required of it. Generally, the spacer is a relatively hydrophilic sequence, typically containing a cysteine residue.

In a third aspect, the spacer can also be described in functional terms as substantially stable in human serum, having a length selected such that it provides an extended structure link at least about 15 A long, preferably about 30 to about 100 A long, between the binding moiety and the remainder of the conjugate molecule, as being substantially non-hydrophobic so as not to adversely affect solubility, and having sufficient flexibility to permit three dimensional movement of the cytotoxic component with respect to the binding component.

B.5 The Target Cell Binding Moiety

The binding moiety may be any ligand that has the required target cell specificity. Antibodies, particularly monoclonal antibodies or their antigen binding fragments, are preferred binding moieties. Monoclonal antibodies against surface receptors of target cells may be made by the somatic cell hybridization procedure first described by Kohler, G. and Milstein, C., Nature, (1975) 256:495-497. The cell lines, reagents, and conditions used in this procedure are well known and have been reviewed extensively in the literature (Somatic Cell Genetics, (1979) 5:957-972). Briefly the procedure involves immunizing a host with the immunogen of interest, collecting antibody-producing cells from the immunized host, fusing the antibody-producing cells from the immunized host with an appropriate tumor cell line using a fusogen such as polyethylene glycol, growing the cells in a selective medium to eliminate unhybridized partners, identifying hybridomas that produce antibody against the immunogen, growing such hybridomas, and collecting monoclonal antibodies from the resulting culture medium (or body fluid when grown in vivo). Antigen binding fragments (Fab, Fab′, $F(ab')_2$, Fv) of the monoclonal antibodies may be made by digesting

the whole Ig with an appropriate protease, for instance papain in the case of Fab and pepsin in the case of $F(ab')_2$. Antigen binding fragments will be particularly useful in instances where it is desired that the binding moiety lack its natural effector function. Antibodies of current interest will typically be of human, rat or murine origin since rat, mouse and human tumor cell lines are available for fusion. Also, a variety of antitumor monoclonal antibody reagents are rapidly becoming available. Human monoclonal antibodies are preferred for use in making conjugates for use in human therapy because of the reduced likelihood of immunogenicity.

## C. Methods of Preparation

### C.1 The Non-binding Portion (Cytotoxic-spacer Portions)

Depending on the sizes of the four polypeptide domains that make up the nonbinding portion of the conjugate toxins, these domains may be synthesized individually or as subunits by conventional polypeptide synthesis techniques (Margolin, A. and Merrifield, R.B., Ann Rev Biochem, (1970) 39:841) and combined in sequence by known procedures.

Recombinant DNA methodology provides an alternative and preferred way of synthesizing the nonbinding portion of the conjugate, either as indi vidual subunits, or as an entire fused polypeptide. This process involves obtaining a DNA Sequence tht encodes the nonbinding portion (or a particular domain or combination of domains) inserting the DNA sequence into a suitable expression vector, transforming microorganisms or cells with the vector, growing transformants that produce the desired fragment and harvesting the desired fragment from the transformants or their growth medium. The coding sequence may be made by synthesis of DNA subunits that encode portions of the enzymatically active fragment, translocation domain, cleavage site domain, and spacer domain and assembling them by ligation techniques known in the art, or by cloning those portions of naturally occuring genes which encode the desired protein. The DNA sequence that encodes the enzymatically active fragment will normally be isolated from naturally occurring DNA; as may the sequence encoding the cleavage and translocation domains. These and the spacer encoding sequence may also be made by conventional DNA synthesis techniques, and may be reproduced by conventional DNA cloning methods. Partial structural genes of bacterial or plant toxins that lack a binding function but retain their enzymatic, cleavage and internalization functions (eg, the CRM 45 mutant of DT) may be cloned and ligated to a DNA sequence that encodes the spacer. The ligation product is inserted into suitable expression hosts and expressed to make the nonbinding portion of the conjugate.

### C.2. The Binding Portions

The binding portions of the conjugates of the invention are antibodies or fragments thereof. Use of monoclonal antibodies is preferred. Antibodies are prepared by means well known in the art, and can be isolated from serum, from spleen, or most preferably prepared and isolated from antibody secreting hybridomas. Many are commercially available (see B.5 herein).

### C.3. Conjugation of Binding and Non-binding Portions

Since the antibodies or fragments thereof are prepared by reactions of immunoglobulins isolated from serum or from hydridomas, they must be linked *in vitro* to the non-binding domains. In a preferred configuration of the non-binding fragment, the cytotoxic portion of the molecule is extended from its C terminus by a sequence providing a spacer which has been provided with a reactive amino acid residue, cysteine, located at or near its C terminus. While bifunctional reagents, such as SPDP, that result in a labile disulfide (C-S-S-C) bridge between the spacer and binding moiety may be used as coupling agents, (and have often been so used where a cleavable link was desired) bifunctional reagents that form a stable (nonreducible) bond, such as a thioether (C-S-C) link are preferred. Such bonds are not susceptible to cleavage in the environment of use, that is, they are not cleaved extracellularly *in vivo*. Several protein coupling agents that form thioether bonds are available. These agents usually contain on one end of the molecule an activated carboxyl group that will react with free amino groups, eg, an $\epsilon$-amino of a lysine of

one of the conjugation partners (in this case the binding portion) and a maleimido or haloacetyl group that will react with a sulfhydryl of the other partner (in this case the non-binding portion) on the other end. Examples of such thioether-forming agents are active esters of the following acids:
6-maleimidocaproic acid, 2-bromoacetic acid, 2-iodoacetic acid,

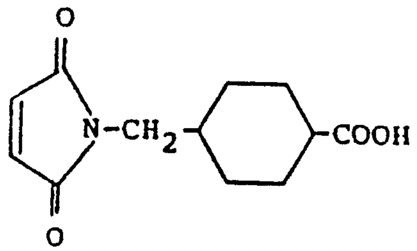

,

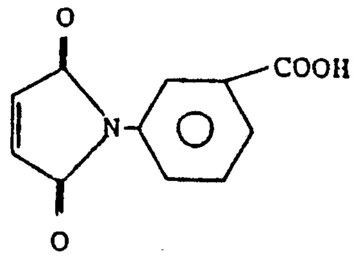

, and

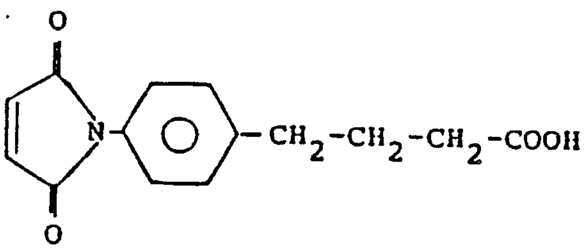

Activation of the carboxyl groups is required to permit reaction with amino groups of the chosen protein. The desired activated derivatives of the foregoing acids include most preferably the succinimidyl ester, ie

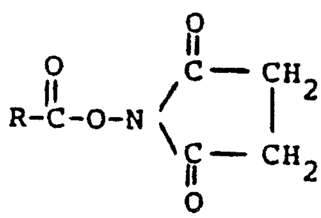

although others, especially esters, have been used, such as the water soluble ester fomed from 1-hydroxy-2-nitro-4-sulfonic acid sodium salt,

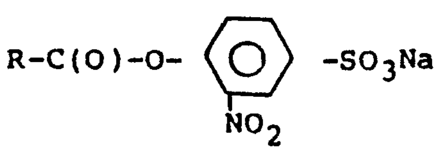

Other coupling agents that may be used are various bifunctional derivatives of imidoesters such as dimethyl adipimidate•HCl, active esters such as disuccinimidyl suberate, aldehydes such as glutaraldehyde, bis-azido compounds such as bis(p-azidobenzoyl)hexanediamine, bis-diazonium derivatives such as bis-(p-diazoziumbenzoyl)-ethylene diamine, diisocyanates such as tolylene 2,6-diisocyanate, and bis-active fluorine compounds such as 1,5-difluoro-2,4-dinitrobenzene. Heterologous permutations of such bifunctional derivatives may also be used as well as peptide bond-generating reagents such as carbodiimides.

In a typical, preferred approach, a thioether linkage is formed between a sulfhydryl on the non-binding portion of the conjugate and the coupling agent and an amide linkage is formed between an $\epsilon$-$NH_2$ of a lysine contained in the binding portion and the carboxyl of the coupling agent.

D. Mode of Administration

When used to kill target cells in vitro the conjugates will typically be added to the target cell culture medium in amounts ranging from about 1000 to about 100,000 conjugate molecules per target cell. The formulation and mode of administration for in vitro use are not critical. Aqueous formulations that are compatible with the culture or perfusion medium will normally be used.

When used in vivo for prophylaxis or therapy of humans or animals (eg, farm, laboratory, sport or pet animals) the cytotoxins ar administered to the patient in a manner and dose that induce the desired target cell reduction response. They will normally be administered parenterally, preferably intravenously. The dose and dosage regimen will depend upon the nature of the target cell and its population, the characteristics of the particular cytotoxin, eg its therapeutic index, the patient, and the patient's history. The amount of cytotoxin administered will typically be in the range of about 0.01 to about 1 mg/kg of patient weight.

For parenteral administration the cytotoxins will be formulated in a unit dosage injectable form (solution, suspension, emulsion) in association with a pharmaceutically acceptable parenteral vehicle. Such vehicles are inherently nontoxic and nontherapeutic. Examples of such vehicles are water, saline, Ringer's solution, dextrose solution, and Hank's solution. Nonaqueous vehicles such as fixed oils and ethyl oleate may also be used. Liposomes may be used as carriers. The vehicle may contain minor amounts of additives such as substances that enhance isotonicity and chemical stability, eg buffers and preservatives. The cytotoxin will typically be formulated in such vehicles at concentrations of about 1 mg/ml to 10 mg/ml.

E. Standard Methods

Recombinant techniques are preferred for construction of the non-binding portions of the conjugates. Illustrated in section F are recombinant production of ricin A, ricin B, diphtheria A, diphtheria A-B′ and diphtheria A-B′ with spacer arm attached. All of these illustrated fragments are useful, alone or conjugated to additional amino acid sequences, in construction of the toxin conjugates. In general, techniques required for such production such as those used to transform cells, construct vectors, extract messenger RNA, prepare cDNA libraries, and the like are widely practiced in the art, and most practitioners are familiar with the standard resource materials which describe specific conditions and procedures. However, for convenience, the following paragraphs may serve as a guideline.

E.1. Hosts and Control Sequences

Procaryotes may be used for both cloning and expression and most frequently are represented by various strains of E. coli. However, other microbial strains may also be used, such as bacilli, for example Bacillus subtilis, various species of Pseudomonas, or other bacterial strains. In such procaryotic systems, plasmid vectors which contain replication sites and control sequences derived from a species compatible with the host are used. For example, E. coli is typically transformed using derivatives of pBR322, a plasmid derived from an E. coli species by Bolivar, et al, Gene (1977) 2:95. pBR322 contains genes for ampicillin and tetracycline resistance, and thus provides additional markers which can be either retained or destroyed in constructing the desired vector. Commonly used procaryotic control sequences which are defined herein to include promoters for transcription initiation, optionally with an operator, along with ribosome binding site sequences, include such commonly used promoters as the beta-lactamase (penicillinase) and lactose (lac) promoter systems (Chang, et al, Nature (1977) 198:1056) and the tryptophan (trp) promoter system (Goeddel, et al Nucleic Acids Res (1980) 8:4057) and the lambda derived $P_L$ promoter and N-gene ribosome binding site (Shimatake, et al, Nature (1981) 292:128).

In addition to bacteria, eucaryotic microbes, such as yeast, may also be used as hosts. Laboratory strains of Saccharomyces cerevisiae, Baker's yeast, are most used although a number of other strains are commonly available. While vectors employing the 2 micron origin of replication are illustrated, Broach, J. R., Meth Enz (1983) 101:307, other plasmid vectors suitable for yeast expression are known (see, for example, Stinchcomb, et al, Nature (1979) 282:39, Tschempe, et al, Gene (1980) 10:157 and Clarke, L, et al, Meth Enz (1983) 101:300). Control sequences for yeast vectors include promoters for the synthesis of glycolytic enzymes (Hess, et al, J Adv Enzyme Reg (1968) 7:149; Holland, et al, Biochemistry(1978) 17:4900). Additional promoters known in the art include the promoter for 3-phosphoglycerate kinase (Hitzeman, et al, J Biol Chem (1980) 255:2073), and those for other glycolytic enzymes, such as glyceraldehyde-3-phosphate dehydrogenase, hexokinase, pyruvate decarboxylase, phosphofructokinase, glucose-6-phosphate isomerase,

3-phosphoglycerate mutase, pyruvate kinase, triosephosphate isomerase, phosphoglucose isomerase, and glucokinase. Other promoters, which have the additional advantage of transcription controlled by growth conditions are the promoter regions for alcohol dehydrogenase 2, isocytochrome C, acid phosphatase, degradative enzymes associated with nitrogen metabolism, and enzymes responsible for maltose and galactose utilization (Holland, ibid). It is also believed terminator sequences are desirable at the 3′ end of the coding sequences. Such terminators are found in the 3′ untranslated region following the coding sequences in yeast-derived genes. Many of the vectors illustrated contain control sequences derived from the enolase gene containing plasmid peno46 (Holland, M. J., et al, J Biol Chem (1981) 256:1385) or the LEU2 gene obtained from YEp13 (Broach, J., et al, Gene (1978) 8:121), however any vector containing a yeast compatible promoter, origin of replication and other control sequences is suitable.

It is also, of course, possible to express genes encoding polypeptides in eucaryotic host cell cultures derived from multicellular organisms. See, for example, Tissue Cultures, Academic Press, Cruz and Patterson, editors (1973). Useful host cell lines include VERO, HeLa cells, and Chinese hamster ovary (CHO) cells. Expression vectors for such cells ordinarily include promoters and control sequences compatible with mammalian cells such as, for example, the commonly used early and late promoters from Simian Virus 40 (SV 40) (Fiers, et al, Nature (1978) 273:113), or other viral promoters such as those derived from polyoma, Adenovirus 2, bovine papiloma virus, or avian sarcoma viruses. General aspects of mammalian cell host system transformations have been described by Axel; U.S. Patent No. 4,399,216 issued 16 August 1983. It now appears, also that "enhancer" regions are important in optimizing expression; these are, generally, sequences found upstream or downstream of the promoter region in non-coding DNA regions. Origins of replication may be obtained, if needed, from viral sources. However, integration into the chromosome is a common mechanism for DNA replication in eucaryotes.

Finally, cells from and portions of higher plants have been found useful as recombinant hosts, and appropriate control sequences are available for expression in these systems. A suitable promoter and polyadenylation signal are those of the nopaline synthese (NOS) gene derived from the 3.2 kilobase (kb) HindIII-23 DNA fragment in the T-DNA region of A. tumefaciens Ti plasmid pTiT37 (Bevan, M., et al, Nucleic Acid Res (1983) 11:369; Depicker, A., et al, J Mol Appl Genet (1982) 1:5613). Suitable plant cells include cells derived from, or seedlings of, tobacco, petunia, and cotton. Other promoters include the maize promoter for alcohol dehydrogenase-1 or alcohol dehydrogenase-2 (Gerlach, W., L., et al, Proc Natl Acad Sci (USA) (1982) 79:2981), cauliflower mosaic virus promoter (Daubert, S., et al, Virology (1982) 122:444), and wheat promoter associated with the small subunit of ribulose biphosphate carboxylase (Broglie, R., et al, Biotechnology (1983) 1:55). Other polyadenylation signals which are available presently include those found on any of the above genes, or those of Schuler, et al, (Schuler, J. S., et al, Nucleic Acid Res (1982) 10:8225).

It is to be noted that ricin A and ricin are toxic to eucaryotic cells, and thus procaryotic hosts are preferred. However, eucaryotic hosts may be used in some circumstances; indeed ricin is natively produced in eucaryotes. It may thus be appropriate to utilize eucaryotic hosts if, for example, the signal sequence is retained for the ricin A or ricin, thus permitting secretion before the toxicity is experienced by the cell.

In the alternative, certain eucaryotic environments may provide modified processing of the ricin or ricin A so as to protect the cell against their potential toxicity. Such mechnisms are not at present established; however it may prove possible to suppress toxicity by a proper folding, glycosylation, or other alterations to the tertiary and derivative structure of the subject protein.

## E.2. Transformations

Depending on the host cell used, transformation is done using standard techniques appropriate to such cells. The calcium treatment employing calcium chloride, as described by Cohen, S. N., Proc Natl Acad Sci (USA) (1972) 69:2110, or the $RbCl_2$ method described in Maniatis, et al, Molecular Cloning: A Laboratory Manual (1982) Cold Spring Harbor Press, p. 254 was used for procaryotes or other cells which contain substantial cell wall barriers. Infection with Agrobacterium tumefaciens (Shaw, C. H., et al, Gene (1983) 23:315) is used for certain plant cells. For mammalian cells without such cell walls, the calcium phosphate precipitation method of Graham and van der Eb, Virology (1978) 52:546 is preferred. Transformations into yeast are carried out according to the method of Van Solingen, P., et al, J Bact (1977) 130:946 and Hsiao, C. L., et al, Proc Natl Acad Sci (USA) (1979) 76:3829.

## E.3. Probing cDNA or Genomic Libraries

cDNA or genomic libraries are screened using the colony hybridization procedure. Each microtiter plate is replicated onto duplicate nitrocellulose filter papers (S & S type BA-85) and colonies are allowed to grow at 37°C for 14-16 hr on L agar containing 50 µg/ml Amp. The colonies are lysed and DNA fixed to the filter by sequential treatment for 5 min with 500 mM NaOH, 1.5 M NaCl, and are washed twice for 5 min each time with 5 x standard saline citrate (SSC). Filters are air dried and baked at 80°C for 2 hr. The duplicate filters are prehybridized at 42°C for 6-8 hr with 10 ml per filter of DNA hybridization buffer (5 x SSC, pH 7.0 5x Denhardt's solution (polyvinylpyrrolidine, plus Ficoll and bovine serum albumin; 1 x = 0.02% of each), 50 mM sodium phosphate buffer at pH 7.0, 0.2% SDS, 20 µg/ml Poly U, and 50 µg/ml denatured salmon sperm DNA).

The samples are hybridized with kinased probe under conditions which depend on the stringency desired. Typical moderately stringent conditions employ a temperature of 42°C for 24-36 hr with 1-5 ml/filter of DNA hybridization buffer containing probe. For higher stringencies high temperatures and shorter times are employed. The filters are washed four times for 30 min each time at 37°C with 2 x SSC, 0.2% SDS and 50 mM sodium phosphate buffer at pH 7, then are washed twice with 2 x SSC and 0.2% SDS, air dried, and are autoradiographed at -70°C for 2 to 3 days.

E.4. Vector Construction

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 µl of buffer solution; in the examples herein, typically, an excess of restriction enzyme is used to insure complete digestion of the DNA substrate. Incubation times of about one hour to two hours at about 37°C are workable, although variations can be tolerated. After each incubation protein is removed by extraction with phenol/chloroform, and may be followed by ether extraction, and the nucleic acid recovered from aqueous fractions by precipitation with ethanol followed by running over a Sephadex G-50 spin column. If desired, size separation of the cleaved fragments may be performed by polyacrylamide gel or agarose gel electrophoresis using standard techniques. A general description of size separations is found in Methods in Enzymology (1980) 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 min at 20 to 25°C in 50 mM Tris pH 7.6, 50 mM NaCl, 6 mM $MgCl_2$, 6 mM DTT and 5-10 µM dNTPs. The Klenow fragment fills in at 5′ sticky ends but chews back protruding 3′ single strands, even though the four dNTPs are present. If desired, selective repair can be performed by supplying only one of the, or selected, dNTPs within the limitations dictated by the nature of the sticky ends. After treatment with Klenow, the mixture is extracted with phenol/chloroform and ethanol precipitated followed by running over a Sephadex G-50 spin column. Treatment under appropriate conditions with S1 nuclease results in hydrolysis of any single-stranded portion.

Exonuclease III attacks double-stranded DNA, but hydrolyzes beginning at the 3′ end of the nucleotide sequence. Thus, digestion of a double-stranded DNA results in two 5′ protruding sticky ends. Hydrolysis is carried out in a buffer containing 15 mM Tris, pH 8, 10 mM NaCl, 1 mM $MgCl_2$, and 0.1 mM DTT, using approximately 2000 units per µl exonuclease III. Ordinarily, 150 units of exonuclease III were used to react with 10 µg DNA.

Synthetic oligonucleotides are prepared by the triester method of Matteucci, et al (J Am Chem Soc - (1981) 103:3185) or using commerically available automated oligonucleotide synthesizers. Kinasing of single strands prior to annealing or for labeling is achieved using an excess, e.g., approximately 10 units of polynucleotide kinase to 0.1 nmole substrate in the presence of 50 mM Tris, pH 7.6, 10 mM $MgCl_2$, 5 mM dithiothreitol, 1-2 mM ATP, 1.7 pmoles γ32P-ATP (2.9 mCi/mmole), 0.1 mM spermidine, 0.1 mM EDTA.

Ligations are performed in 15-30 µl volumes under the following standard conditions and temperatures: 20 mM Tris-Cl pH 7.5, 10 mM $MgCl_2$, 10 mM DTT, 33 µg/ml BSA, 10 mM-50 mM NaCl, and either 40 µM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 µg/ml total DNA concentrations (5-100 nM total end concentration). Intermolecular

blunt end ligations (usually employing a 10-30 fold molar excess of linkers) are performed at 1 µM total ends concentration.

In vector construction employing "vector fragments", the vector fragment is commonly treated with bacterial alkaline phosphatase (BAP) in order to remove the 5′ phosphate and prevent religation of the vector BAP digestions are conducted at pH 8 in approximately 150 mM Tris, in the presence of $Na^{+}$ and $Mg^{+2}$ using about 1 unit of BPA per µg of vector at 60°C for about one hour. In order to recover the nucleic acid fragments, the preparation is extracted with phenol/chloroform and ethanol precipitated and desalted by application to a Sephadex G-50 spin column. Alternatively, religation can be prevented in vectors which have been double digested by additional restriction enzyme digestion of the unwanted fragments.

For portions of vectors derived from cDNA of genomic DNA which require sequence modifications, site specific primer directed mutagenesis is used. This is conducted using a primer synthetic oligonucleotide complementary to a single stranded phage DNA to be mutagenized except for limited mismatching, representing the desired mutation. Briefly, the synthetic oligonucleotide is use as a primer to direct synthesis of a strand complementary to the phage, and the resulting double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The resulting plaques are hybridized with kinased synthetic primer at a temperature which permits hybridization of an exact match, but at which the mismatches with the original strand are sufficient to prevent hybridization. Plaques which hybridize with the probe are then picked, cultured, and the DNA recovered.

In more detail, approximately one pmole of the phage single stranded DNA template is mixed with approximately 10 pmoles of the synthetic oligonucleotide primer in 15 µl of 10 mM Tris, 10 mM $MgCl_2$, 90 mM NaCl. The mixture is heated to 67° for 3-5 min and then to 42° for 30 min. The mixture is then cooled on ice, and a cold solution containing the 4 dNTPs at 500 µM and 3-5 units of Polymerase I (Klenow) in sufficient buffer to bring the volume to 20-25 µl is added. The mixture is left at 0°C for 5 min and then brought to 37° for 30 min. The Klenow is then inactivated for 15 min at 75°, and the mixture transformed into an appropriate host, such as E. coli JM103 or E. coli JM105 using 1 µl reaction mixture per 300 µl cells, which are grown on yeast extract-typtone agar plates. The resulting phage plaques are transferred to filters by lifting onto nitrocellulose, and pre-hybridized in 5 ml/filter of 6 x SSC, 5 x Denhardt's, 0.1% SDS, 50 µg/ml carrier (yeast RNA salmon sperm DNA etc.) at the desired temperature for 1-2 hr.

The fixed, pre-hybridized filters are then hybridized with 2 x $10^5$ cpm/ml of kinased synthetic primer oligonucleotide (approximately 2-10 x $10^7$ cpm/µg) for 3-16 hr, and then washed in 6 x SSC once at room temperature for 5 min and then at the appropriate stringent temperature for 5 min. A simultaneous control run containing the original phage is used to verify that hybridization does not take place to the non-mutagenized strands.

E.5. Verification of Construction

In the constructions set forth below, correct ligations for plasmid construction are confirmed by first transforming E. coli strain MM294 obtained from E. coli Genetic Stock Center, CGSC #6135, or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D. B., et al, Proc Natl Acad Sci (USA) (1969) 62:1159, optionally following chloramphenicol amplification (Clewell, D. B., J Bacteriol (1972) 110:667). The isolated DNA is analyzed by restriction and/or sequenced by the dideoxy method of Sanger, F., et al, Proc Natl Acad Sci (USA) (1977) 74:5463 as further described by Messing, et al, Nucleic Acids Res (1981) 9:309, or by the method of Maxam, et al, Methods in Enzymology (1980) 65:499.

E.6. Hosts Exemplified

Host strains used in cloning and expression herein are as follows:

For cloning and sequencing, and for expression of construction under control of most bacterial promoters, E. coli (K12) strain MM294 (E. coli Genentic Stock Center CGSC #6135), Talmadge, K., et al, Gene (1980) 12:235; Meselson, M., et al, Nature (1968) 217:1110, was used as the host. For expression

under control of the $P_LN_{RBS}$ promoter, E. coli strain K12 MC1000 lambda lysogen, $N_7N_{53}cI857SusP_{80}$, ATCC 39531 (hereinafter sometimes referred to as MC1000-39531) is used.

For M13 phage recombinants, E. coli strains susceptible to phage infection, such as E. coli K12 strain DG98, JM103 or JM105 are employed. The DG98 strain has been deposited with ATCC July 13, 1984 and has accession number 39768.

## E.7. Construction of Host Vectors for Expression

The successful expression attained by the invention depends upon correct utilization of the suitable control sequences to regulate expression of the desired toxin fragment. Therefore, whatever the host, control sequences compatible with and suitable for that host are positioned operably with respect to the coding sequence, using a properly placed "start" codon at the 5′ end of the desired sequence. Any "native" control sequences are eliminated. The vectors of the invention place the coding sequence for the desired protein immediately preceded by an ATG start codon directly downstream from control systems chosen to be compatible with the particular host.

It is also important, in obtaining good production of the desired fragments, to regulate the "time" of production so as to minimize any lethal effect on the host cell. Most typically, even for procaryotes, this is done by delaying expression of the desired protein sequences until substantial growth has occurred. Accordingly, it is desirable to utilize control sequences which are subject to environmental conditions. By maintaining conditions that repress expression during growth phase, and then converting to conditions which permit expression at the desired time, the negative aspects of any potentially lethal effect can be minimized.

In two particularly preferred approaches, these regulatable control sequences are compatible with procaryotic hosts. The trp promoter is a regulatable promoter where expression of the operably linked sequence can be controlled by the level of tryptophan in the medium. By maintaining high tryptophan levels during growth, expression is repressed. Depletion or competitive inhibition of tryptophan turns on the promoter and permits expression.

Still more preferred is the $P_L$ promoter derived from lambda phage. This promoter is regulated by a protein which can be temperature sensitive. (There are mutant forms of the wild type repressor, e.g., $CI_{857}$ which have this characteristic known in the art.) When used in a host which is able to synthesize this mutant form of repressor (such as E. coli K12 strain MC1000 lysogenic for the lambda phage $N_7N_{53}CI_{857}SusP_{80}$), the $P_L$ promoter will be switched on when the temperature is raised because the higher temperature inactivates the mutant CI repressor. Thus, the host cells can be grown at low temperature without, or with, low production of the foreign protein. The temperature is then raised when growth has been attained and desired protein production is desired.

A plasmid which has temperature sensitive copy number control may also be applied. If the cells are grown at low temperatures, coding sequences contained in the plasmid are replicated at low levels; at higher temperatures, the number of such copies is increased. The amount of protein produced is thus indirectly managed by regulating the number of available copies of its coding sequence.

Several vectors which provide control sequences and replicons useful in obtaining the various conjugate non-binding sequences are described below. These include: pCS3, a temperature-sensitive, high copy number plasmid, pFC5, $pP_L322$, and $pP_LKan$, which are sources of the $P_LN_{RBS}$ control sequences, pPLOP, which contains both this cassette and the replicon sequences of pCS3, pTRP3 which has the trp control sequences available as an EcoRI/HindIII cassette, and pDG141 which contains this cassette, as well as an alternate control sequence wherein the trp control sequences are followed by an ATG start codon immediately upstream from a SacI site.

### E.7.a. Construction of pCS3

The temperature-sensitive, high copy number plasmid pCS3 was deposited with ATCC 3 June 1982 and has accession number 39142.

pCS3 is derived from pEW27 and pOP9. pEW27 is described by E. M. Wong, Proc Natl Acad Sci (USA) (1982) 79:3570. It contains mutations near its origin of replication which provide for temperature regulation of copy number. As a result of these mutations replication occurs in high copy number at high temperatures, but at low copy number at lower temperatures.

pOP9 is a high copy number plasmid at all temperatures which was constructed by inserting into

pBR322 the EcoRI/PvuII origin containing fragment from Col E1 type plasmid pOP6 (Gelfand, D., et al, Proc Natl Acad Sci (USA) (1978) 75:5869). Before insertion, this fragment was modified as follows: 50 µg of pOP6 was digested to completion with 20 units each BamHI and SstI. In order to eliminate the SstI 3′ protruding ends and "fill in" the BamHI 5′ ends, the digested pOP6 DNA was treated with E. coli DNA polymerase I (Klenow in a two-stage reaction first at 20 °C for elimination of the 3′ SstI protruding end and then at 9 °C for repair at the 5′ end. The blunt-ended fragment was digested and 0.02 pmole used to transform competent DG75 (O'Farrell, P., et al, J Bacteriology (1978) 134:645-654). Transformants were selected on L plates containing 50 µg/ml ampicillin and screened for a 3.3 kb deletion, loss of an SstI site, and presence of a newly formed BamHI site.

One candidate, designated pOP7, was chosen and the BamHI site deleted by digesting 25 g of pOP7 with 20 units BamHI, repairing with E. coli DNA polymerase I fragment (Klenow), and religating with T4 DNA ligase. Competent DG75 was treated with 0.1 µg of the DNA and transformants selected on L plates containing 50 µg/ml ampicillin. Candidates were screened for the loss of the BamHI restriction site. pOP8 was selected. To obtain pOP9 the AvaI(repaired)/EcoRI $Tet^R$ fragment from pBR322 was prepared and isolated and ligated to the isolated PvuII(partial)/EcoRI 3560 bp fragment from pOP8.

Ligation of 1.42 kb EcoRI/AvaI(repair) $Tet^R$ (fragment A) and 3.56 kb EcoRI/PvuII $Amp^R$ (fragment B) used 0.5 µg of fragment B and 4.5 µg of fragment A in a two-stage reaction in order to favor intermolecular ligation of the EcoRI ends.

Competent DG75 was transformed with 5 µl of the ligation mixture, and transformants were selected on ampicillin (50 µg/ml) containing plates. pOP9, isolated from $Amp^R$ $Tet^R$ transformants showed high copy number, colicin resistance, single restriction sites for EcoRI, BamHI, PvuII, HindIII, 2 restriction sites for HincII, and the appropriate size and HaeIII digestion pattern.

To obtain pCS3, 50 µg pEW27 DNA was digested to completion with PvuII and the EcoRI. Similarly, 50 µg of pOP9 was digested to completion with PvuII and EcoRI and the 3.3 kb fragment was isolated.

0.36 µg (0.327 pmoles) pEW27 fragment and 0.35 µg (0.16 pmoles) pOP9 fragment were ligated and used to transform E. coli MM294. $Amp^RTet^R$ transformants were selected. Successful colonies were initially screened at 30 °C and 41 °C on beta-lactamase assay plate and then for plasmid DNA levels following growth at 30 °C and 41 °C. A successful candidate, designated pCS3, was confirmed by sequencing.

E.7.b Vectors Supplying the $P_L$ Control Sequence

Three plasmids were constructed which can serve as sources for the EcoRI (or PstI)/HindIII $P_LN_{RBS}$ cassette. For each of these plasmids, the DNA sequence containing $P_L$ λ phage promoter and the ribosome binding site for the N-gene ($N_{RBS}$) is obtained from a derivative of pKC30 described by Shimatake and Rosenberg, Nature (1981) 292:128. pKC30 contains a 2.34 kb fragment from λ phage cloned into the HindIII/BamHI vector fragment from pBR322. The $P_L$ promoter and $N_{RBS}$ occupy a segment in pKC30 between a BglII and HpaI site. The derivative has the BglII site converted to an EcoRI site.

The BglII site immediately preceding the $P_L$ promoter was converted into an EcoRI site as follows: pKC30 was digested with BglII, repaired with Klenow and dNTPs and ligated with T4 ligase to an EcoRI linker (available from New England Biolabs) and transformed into E. coli K12 strain MM294 $Lambda^+$. Plasmids were isolated from $Amp^R$ $Tet^S$ transformants and the desired sequence confirmed by restriction analysis and sequencing. The resulting plasmid, pFC3, was double-digested with PvuI and HpaI to obtain an approximately 540 bp fragment framing the desired sequence. This fragment was partially digested with HinfI and the 424 bp fragment isolated and treated with Klenow and dATP, followed by S1 nuclease, to generate a blunt-ended fragment with the 3′ terminal sequence -AGGAGAA, where the -AGGAGA portion is the $N_{RBS}$. This fragment was restricted with EcoRI to give a 347 base pair DNA fragment with 5′-EcoRI (sticky) and HinfI(partial repair S1 blunt)-3′ termini.

## Preparation of pFC5

Pβl-Z15, deposited 13 January 1984, ATCC No. 39578, was prepared by fusing a sequence containing ATG plus 140 bp of β-IFN fused to lac-Z into pBR322. In pβl-Z15, the EcoRI site of pBR322 is retained, and the insert contains a HindIII site immediately preceding the ATG start codon of β-IFN. pβ1-Z15 was restricted with HindIII, repaired with Klenow and dNTPs, and then digested with EcoRI. The resulting EcoRI/HindIII (repaired) vector fragment was ligated with the EcoRI/HinfI (repaired) fragment above, and the

ligation mixture used to transform MC1000-39531. Transformants containing the successful construction were identified by ability to grow on lactose minimal plates at 34° but not at 30°. (Transformations were plated on X-gal-Amp plates at 30° and 34° and minimal-lactose plates at 30° and 34°. Transformants with the proper construction are blue on X-gal-Amp plates at both temperatures, but on minimal lactose plates, grow only at 34°.) The successful construct was designated pFC5.

Preparation of $pP_L322$

In the alternative, pBR322 may also be used as the cloning vector to carry the desired EcoRI/HindIII $P_L$-$N_{RBS}$ cassette. pBR322 was digested with HindIII, repaired with Klenow and dNTPs, and then further digested with EcoRI. The vector fragment was then ligated to the EcoRI/HinfI(repaired) fragment prepared above, and the ligation mixture transformed into MC1000-39531. Successful transformants were identified as $Amp^R$ $Tet^S$ colonies. Plasmids were isolated from succesful transformants and a successful ligation was confirmed by sequencing, and designated $pP_L322$.

Preparation of $pP_LKan$

The third host plasmid vector used to obtain the cassette was pDG144, deposited January 13, 1984, ATCC No. 39579. pDG144 is extensively described in another application and is not part of the invention. It is an altered pBR322 containing an intact $Amp^R$ gene, and a coding sequence for a protein capable of conferring resistance to kanamycin ($Kan^R$). The $Kan^R$ coding sequence is preceded by a synthetic polylinker. Since pDG144 contains neither a promoter nor a ribosome binding site preceding the coding sequence, $Kan^R$ is not expressed, and cells harboring pDG144 are sensitive to Kan and to structurally related antibiotics. The polylinker sequence immediately preceding the ATG start codon for the kanamycin gene can be removed by digestion with EcoRI and HindIII and $P_LN_{RBS}$ inserted.

Accordingly, pDG144 was digested with HindIII, blunt-ended with Klenow and dNTPs, and then digested with EcoRI. The vector fragment was ligated with the above-prepared EcoRI/HinfI(repaired) fragment and transformed into MC1000-39531. $Amp^R$ $Kan^R$ colonies were selected, plasmids isolated and the correct sequence construction verified by restriction analysis and sequencing. One plasmid containing the correct sequence was designated $pP_LKan$.

Each of the above resulting vectors, pFC5, $pP_L322$, and $pP_LKan$, may be used to clone and provide the EcoRI/HindIII $P_LN_{RBS}$ cassette. The cassette can then conveniently be placed behind an ATG start codon which contains a HindIII site immediately preceding it.

E.7.c. Completion of pPLOP

pCS3 was then modified to provide the $P_L$ and $N_{RBS}$ control sequences. pCS3 was digested with HindIII, and then digested with EcoRI. The vector fragment was ligated with an isolated EcoRI/HindIII from pFC5 containing the $P_LN_{RBS}$ and transformed into E. coli MM294. The correct construction of isolated plasmid DNA was confirmed by restriction analysis and sequencing and the plasmid designated pPLOP. pPLOP was deposited with ATCC 18 December 1984 and has accession number 39947.

E.7.d. Construction of pTRP3

To construct the host vector containing the trp control sequences behind a HindIII site, the trp promoter/operator/ribosome binding site sequence, lacking the attenuator region, was obtained from pVH153, obtained from C. Yanofsky, Stanford University. Trp sequences are available in a variety of such plasmids known in the art. pVH153 was treated with HhaI (which cuts leaving an exposed 3′ sticky end just 5′ of the trp promoter) blunt-ended with Klenow, and partially digested with TaqI. The 99 bp fragment corresponding to restriction at the TaqI site, 6 nucleotides preceding the ATG start codon of trp leader was isolated, and then ligated to EcoRI (repair)/ClaI digested, pBR322 to provide pTRP3. pTRP3 was deposited with ATCC 18 December 1984 and has accession number 39946.

E.7.e. Construction of pDG141

pDG141 contains the trp control sequences immediately upstream from an ATG start codon. It was deposited with the ATCC January 24, 1984, and given the accession number 39588. The sequence downstream of the ATG provides a SacI cleavage site which cuts between the G and the succeeding bp. In the construction of pDG141, a derivative of pBR322, pTRP3, is used to provide a trp (PstI/HindIII) cassette and pBW20 to provide the ATG and SacI site.

Construction of pBW20

pBW20 contains a synthetic ATG-containing dodecamer cloned into the HindIII/PvuII vector fragment from pBR322. The dodecamer, TATGAGCTCATA, contains SstI (or SacI) sites. To prepare pBW20, pBR322 was digested with HindIII, repaired with Klenow and the four dNTPs, and then digested with PvuII. The vector fragment was ligated with the self-complementary dodecamer and transformed into *E. coli* MM294 and the correct construction confirmed by plasmid isolation and sequencing.

pBR322 dodecamer pBR322

TCGATA|AGCTTATGAGCT|CATACTG

HindIII SacI

12 ng of pTRP3 restricted with PstI and HindIII was ligated with 1.34 ng of similarly restricted pBW20. The ligation mixture was subsequently digested with BamHI to linearize any ligation products which contained the HindIII/PstI unwanted vector fragment from pTRP3. The ligation mixture was used to transform *E. coli* MM294, and the desired colonies selected on plates of L-Broth containing 50 µg/ml ampicillin pre-spread with 500 µg tryptophan. Correct construction was confirmed by sequencing.

F. Diphtheria Toxin Components and Conjugates

An illustrative and preferred embodiment of the invention comprises the components and intermediates in a specific conjugated toxin. In this toxin, the binding fragment consists of anti-Daudi antibodies which are linked by reaction with the succinimidyl ester of m-maleimidobenzoic acid to a spacer portion of the formula

$$\text{Gly-Thr-Gly-Ser-Gly(Pro)}_6\ \text{Ser-Gly-Ser-Gly-Thr-Cys}$$

spacer — reactive amino acid

which is in turn a C terminal extension of a portion of the naturally occurring diphtheria toxin. Thus, the conjugate toxin here exemplified can be represented by the formula:

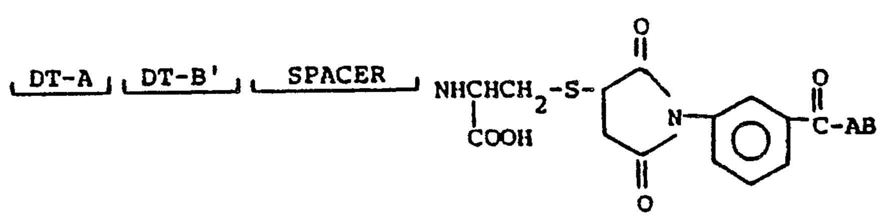

wherein DTA represents the enzymatically active portion, or "A" chain of diphtheria toxin, DT-B′ represents the first approximately 190 amino acids of the diphtheria B chain, and AB represents the anti-Daudi antibody.

In this embodiment, three of the elements of the non-binding portion of the conjugate toxin are derived from diphtheria toxin; the enzymatically active domain, the cleavage site domain and the translocation domain. The mature diphtheria toxin molecule including both A and B chains contains 535 amino acid residues of which the A chain (the amino terminal fragment) contains 193 residues and the B chain (the carboxy terminal fragment) contains 342 residues. It appears that the intracellular cleavage site between the A and B chains in the native toxin is after one of the three Arg residues. Such cleavage readily takes place in vitro catalyzed by trypsin-like enzymes. It is thought that a similar cleavage takes place in vivo, thus exposing the disulfide link between the cysteine at position 186 and that at 201 for reductive intracellular cleavage. It is known that the amino terminal 193/342 of the B fragment contains sequences that cause the toxin to insert into artificial lipid bilayers under appropriate conditions forming ion conductive channels (Kagan, B.L., et al, Proc Natl Acad Sci (USA), (1981) 78:4950; Donovan, J.J., et al Proc Natl Acad Sci, (1972) 78:172 (1972); Kaiser, G., et al Biochem Biophys Res Commun, (1981) 99:358; FEBS Letters (1983) 160:82). Thus the portions of the diphtheria toxin which are embodied in the illustrative conjugate toxin contain the intracellular cleavage/extracellularly stable connection between the A and B chain and at least a portion of the hydrophobic domain responsible for translocation of the cytotoxic portion into the cytosol.

Fig. 1 shows the sequence of the diphtheria toxin gene and the flanking regions, along with the deduced amino acid sequence. The deduced sequence is in reasonable agreement with the previously reported primary amino acid sequence data (Delange, R.J., et al, Proc Natl Acad Sci (USA) (1976) 73:69; Delange, R.J., et al, J Bio Chem (1979) 254:5827; Drazin, R.E., et al, (ibid) 5832 (1979); Delange, R.J., et al, (ibid) 5838 (1979); Falmagne, P., et al, Biochim Biophys Acta (1978) 535:54; Falmagne, P., et al, Toxicon - (1979) 17:supp 1 46; Lambotte, P., et al, J Cell Biol (1980) 87:837; Capiau, C., et al, Arch Phys (1982) 90:B-96; Falmagne, P., et al, Toxicon (1982) 20:243). The deduced sequence assumes a leader sequence as shown, consistent with the fact that DTB is secreted from the natural source, C. Diphtheriae and with the fact that the sequence in this region strongly resembles known signal peptides (Michaelis, S., et al, Ann Rev Microbiol (1982) 36:435). It is presently believed that the GTG codon at position -25 serves as a start codon and encodes methionine rather than the valine there shown.

The entire toxin gene sequence is carried by bacteriophage-$\beta$ and can be isolated from the phage by restriction with Xba 1 and EcoR 1. A shorter MspI fragment within this sequence (see Fig 1) comprises most of the sequence used in the illustrative construct herein, this fragment results from Msp1 restriction about 300 bp preceding the first amino acid codon, and at the site shown at the codon encoding amino acid 382, approximately in the middle of the B fragment.

The construction of the illustrated conjugate toxin may be summarized as follows:

the Msp portion of the gene containing the codons for the A chain and approximately half of the B chain are ligated to synthetic DNA encoding the desired spacer with its reactive amino acid (cysteine) terminus. The resulting oligonucleotide is then modified to delete the promoter and ribosome binding regions as well as the codons for the leader sequence. It is then ligated into an operably linked position with respect to the $P_L$ promoter and N-gene ribosome binding site in suitable expression vector, along with an ATG start codon immediately preceding the glycine residue at position 1. Bacteria transformed with this expression vector produced the entire non-binding region of the molecule. The transformed cells are sonicated and the non-binding portion recovered from the sonicate. The non-binding portion is then linked to the anti-Daudi antibody in vitro using an activated m-maleimidobenzoic ester as linker.

Additional non-binding fragments are also recombinantly derived as illustrated below, and used to obtain alternate conjugates.

## F.1. Isolation and Cloning of the Msp1 Fragment from the DT Gene

DNA was isolated from corynephage $\beta^{Tox+}$ grown on Corynebacterum diphtheriae $C7^{(-)tox-}$. (The host and phage are obtainable from J. Collier, University of California, Los Angeles; see Tweten, R.K., et al, J Bacteriol (1983) 156:680.

To prepare DNA, high-titered $\beta$ phage stocks were prepared in "TYE" medium: (15 g/l bactotryptone, 10 g/l yeast extract, 5 g/l NaCl supplemented with 1 mM $CaCl_2$), by the method of Holmes, R.K., et al J Virology (1969) 38:586. Upon completion of lysis, debris was removed by centrifugation at 13,000 x g for 5 min, and NaCl added to 0.5 M, followed by PEG to 100 g/l, and the mixture was stirred overnight at 4° C. The phage were concentrated by centrifugation at 13,000 x g for 15 min and resuspended in 100 mM Tris HCl pH 7.5, 100 mM NaCl, 20 mM EDTA. Pronase was added to 1 mg/ml and the mixture was incubated at 37° C for 2 hr. After removal of PEG by addition of potassium phosphate (dibasic:monobasic/2:1) to 23% and centrifugation at 6,000 x g for 5 min, the lower phase was extracted with phenol, ethanol precipitated

and the DNA purified by banding in a CsCl-EtBr gradient.

Approximately 500 µg of the phage DNA (MW = 22 x $10^6$ daltons) was treated with EcoRI and XbaI and the resulting mixture run on 1.7 liters 1% agarose gel at 90 volts for 35 hr. The XbaI/EcoRI fragment (1.5 x $10^6$ daltons) containing the toxin gene was cut out, run through a syringe, and electroeluted in 1/10 TBE for 4 hrs at 500 volts onto a spectropore dialysis membrane. The DNA was retreived from the membrane using 0.25% SDS in 1/10 TBE, phenol extracted, ether extracted, and ethanol precipitated.

The resulting DNA was further restricted with MspI, the DNA resolved on 5% PAGE, and the two MspI fragments isolated by the crush and soak method. The large Msp fraction (see Fig 1) which contained control sequences, leader, A, and partial B sequences from the toxin was cloned by ligating approximately 5 ng of the fragment with 2 µg of ClaI-restricted, BAPed, pBR322. The ligation mixture was transformed into E. coli MM294, and the desired clones determined by isolation of plasmids, restriction analysis and sequencing. The desired cloning vector was designated pMsp. Although this cloning was accomplished as above, constructions to provide the non-binding portion were obtained using phage directly as the source of Msp fragment.

F.2. Synthesis and Cloning of Spacer Coding Sequence

A DNA fragment encoding the amino acid sequence

Gly-Thr-Gly-Ser-Gly-(Pro)$_6$-Ser-Gly-Ser-Gly-Thr-Cys

and flanked by sequence defining convenient restriction sites and a stop codon was designed and synthesized by conventional DNA synthesis procedures.

The sequence,

```
AG CTT CCA GGC ACT GGA TCT GGC-
           Gly Thr Gly Ser Gly-

CCG CCG CCA CCG CCG CCT TCT GGA TCC GGT ACC TGC TGA G
Pro Pro Pro Pro Pro Pro Ser Gly Ser Gly Thr Cys Stop
```

and its complement were prepared using the triester method of Matteucci (supra) and annealed and kinased to give the double stranded sequence:

```
P-AGCTTCCAGGC-----------ACCTGCTGAG
      AGGTCCG-----------TGGACGACTCAGCT-P

HindIII                                 SalI
```

The annealed sequence was cloned as follows: pBR322 (25.72 µg) was restricted with SalI and HindIII, BAPed, phenol extracted and desalted over a one cc Sephadex G-50 column. The kinased annealed, double stranded spacer encoding sequence (0.2 pmoles) was ligated with 1 µg of the plasmid vector fragment, and the ligation mixture was used to transform E. coli strain MM294. $Amp^RTet^S$ colonies were screened for plasmid size. The desired plasmid, pSA1 was confirmed by restriction analysis and sequencing.

F.3 Cloning of Spacer onto Msp1 Fragment

The plasmid, pMspSA2 which contains the MspI fragment coding sequence ligated to the spacer coding sequence was contructed as outlined in Fig. 2.

pSA1 plasmid DNA (77 µg) was restricted with SalI and ClaI, run on a 12% polyacrylamide gel and the fragment containing the spacer arm sequence isolated by the crush and soak method. One half the sample was further restricted with AluI to give "fragment A". As shown in Fig. 2, the Alu cleavage results in a blunt

end 6 bp upstream from the glycine codon.

The large MspI fragment (10 ng) isolated from phage as in F.1 was blunt ended by filling in with Klenow fragment and dNTPs. The mixture was run over a Sephadex G-50 column, treated with HindIII, and re run over a 1 cc Sephadex G-50 column to give "fragment B". As seen from Fig. 1, HindIII restriction deletes a portion of the DT control sequences, but probably leaves at least a portion of the promoter and ribosome binding site, and the entire leader sequence.

For the vector, 77 µg pSA1 was restricted with HindIII and SalI, treated with BAP, and the vector DNA fragment purified with a 1 cc Sephadex G-50 column to give "fragment C".

The ligation mixture consisted of 4 µg of fragment C, 3 ng of fragment B, and 20 ng of fragment A under standard ligation conditions. Following ligation overnight at 12° C, the mixture was transformed into E.coli strain MM294, and $Amp^R Tet^S$ colonies screened for plasmid size. The desired construct was identified by restriction analysis and confirmed by Maxam-Gilbert sequencing. This plasmid, pMspSA2 contains, between the HindIII and SalI sites of pBR322, a portion of the DT control sequence, leader sequence, A fragment, B fragment through the codon for amino acid 382, and the spacer arm codons.

F.4. Deletion of the DT Promoter and Leader Sequences

The preparation of pATGMspSA is outline in Fig 3.

pTrpSmIMbo (55µg) was double digested with AccI and ClaI and the short fragment spanning the ATG start codon and a portion of the A fragment isolated. (See Fig 4 for relevant sequences in pTrpSmIMbo and ¶F.4.a below for its construction.)

A vector fragment was prepared by digesting 25 µg of pMspSa with ClaI, and treating with BAP. The missing portions of the Msp toxin fragment were supplied by a digest of 50 µg of pMspSA with AccI and ClaI and isolating the 764 bp fragment between these sites in the coding sequence.

A ligation mixture containing 250 ng of the ATG-partial A fragment from pTrpSMIMbo, 700 ng of the partial A- partial B fragment from pMspSA and 2 µg of the spacer-vector fragment from pMspSA was transformed into E.coli MM294 and $Amp^R Tet^S$ colonies selected. The correct construction was confirmed by restriction analysis.

F.4.a Constructioin of pTrpSmIMbo

pTrpSmIMbo contains the DT-A fragment coding sequence followed by the Mbo terminator sequence (supra) under the control of the trp promoter. The DT-A and Mbo terminator) and pDG141, which contains the trp promoter (see Fig 4)

To construct pTS12, the oligonucleotide

GA TCT GTT GGC TCG AGT TGA
Arg Ser Val Gly Ser Ser Term

which encodes the amino acid sequence subsequent to the Mbo cleavage site for six additional amino acids prior to a termination codon was synthesized using the triester method of Matteucci, et al (supra); kinased and hybridized to the complementary synthetic fragment to give

5' HO GATCTGTTGGCTCGAGTTGA
ACAACCGAGCTCAACTAGCT P
BglII SalI

One pmole of the double-standard oligonucleotide was then placed in a three way ligation mixture with 1.4 pmoles (0.8 µg) of Mbo fragment 1 and the vector fragment formed from 1 µg pBR322 which had been treated with BamHI, SalI and BAP. The mixture was ligated overnight before transforming into E. coli MM294. $Amp^R Tet^S$ colonies were selected and the desired construction confirmed by DNA isolation, restriction analysis and sequencing. The correct plasmid was designated pTS12.

pTS12 (53.5 µg) was restricted with HhaI blunt-ended with Klenow, 18 µg of the resulting fragments

ligated to 3.15 nmoles of the oligomeric linker CCCCGGGG, and then treated with SmaI. The resulting sequence at the 5′ terminus was thus modified to give the sequence GGGGCTGA which encodes the peptide sequence beginning with amino acid 1 of the DT-A fragment, (See Fig. 4). The 3′ end of the ligation product terminates in the first HhaI site of pBR322 following the SalI site, and the fragment contains the entire coding sequence along with in reading frame with terminator for the small Mbo fragment. The desired 654 bp fragment was isolated using 6% PAGE, and elution by crush and soak.

One picomole of this modified prepared fragment was ligated with 0.7 µg of pDG141 which had been restricted with SacI, blunt-ended with Klenow, and BAPed (the preparation of pDG141 is described above). The pDG141 derived fragment has an ATG start codon operably linked to the trp promoter. The resulting ligation mixture was transformed into E.coli MM294, and resistant colonies grown in 10 ml TYE′ medium containing 1009 µg/ml ampicillin and screened for plasmid size. Those colonies which contained plasmids larger than pDG141 wee screened for expression of the DT-A fragment.

The cells were grown to log phase in 10 ml of the TYE′-Amp 100 medium at 37° for 4 hr. To demonstrate expression, one ml of the culture was centrifuged and the pellet resuspended in 20 µl buffer containing 625 mM Tris, pH 6.8, 3% SDS. After heating at 95°C for 5 min, samples were run in 12.5% SDS-PAGE with a 3% stacking (Laemmli, et al, Nature (1970) 227:680). Two clones which showed an additional protein band at the expected molecule weight were confirmed by the EF-2 ADP-ribosylation assay according to the procedure of Chung, D.W., et al, Infect Immun (1977) 16:832. These colonies, designated ptrpSmlMbo, produced 20 µg of DT-A per ml of culture. The anti-genicity and molecular weight of the product were confirmed by Western Blot.

F.5. Preparation of Expression Vectors $pP_L$MspSA and $pP_L$OPMspSA for Expression of the DT A-B′ Toxin-Spacer Construct.

One expression vector, $pP_L$MspSA was constructed by inserting the appropriate portions of pATGMspSA behind the $P_L$ promotor. The construction is shown in Fig. 3. pATGMspSA was digested with HindIII, PstI, (EcoRI to prevent religation) and the large vector fragment containing the ATG start codon, the A and B′ DT toxin and spacer coding sequences used in subsequent ligation. This fragment was then ligated with a PstI, HindIII, BAPed preparation of pFC5 which fragment corresponds to the portion of pFC5 containing the $P_L$ promoter and N-gene ribosome binding site. The ligation mixture was then used to transform MC1000-39351 and transformants selected by $Amp^R$. The correct construction of the desired plasmid $pP_L$MspSA was confirmed by restriction analysis.

A second vector, $pP_L$OPMspSA was constructed by a two-way ligation of a fragment obtained by restricting pCS3 with EcoRI, SalI followed by BAP treatment and a fragment obtained from $pP_L$MspSA by restriction with EcoRI, SalI and PstI (see Fig 3). The ligation mixture was then used to transform MC1000-39351 and transformants selected by $Amp^R$. The correct construction of the desired plasmid $pP_L$OPMspSA was confirmed by restriction analysis and Maxam-Gilbert sequencing.

The colonies transformed with each of the foregoing plasmids were grown at 30°C in TYE medium containing 100 µg/ml ampicillin, and at the end of log phase the temperature raised to 42°C. After 1.5 hr the cells were centrifuged and sonicated and the sonicate assayed by the assay of Chung, D.W., et al, Infect Immun (1977) 16:832 for enzymatic activity. Activity corresponding to DTA-B′-spacer at levels of 1-10 µg/ml medium was found when $pP_L$MspSA was used and 20-150 µg/ml when $pP_L$OPMspSA was used. Production of the desired DT A-B′-spacer was confirmed by Western Blot.

F.6. Preparation of Expression Vectors for DT Fragments Without Spacer

F.6.a. $pP_L$OPSau (Expression Vector for DT-A)

The construction is outlined in Figure 5.

To provide a properly terminated DT-A fragment, the synthetic oligonucleotide

**GA TCT GTT GGC TCG AGT TGA**

**Arg Ser Val Gly Ser Ser Term**

which encodes the six amino aids normally found sequential to the Arg at position 193 of native DT and a stop codon, was kinased and annealed to a complementary sequence to give the double stranded sequence:

```
5'P GATCTGTTGGCTCGAGTTGA
               ACAACCGAGCTCAACTAGCT OH5'
    BglII                    SalI
```

as shown in Figure 5. The fragment has a phosphorylated BglII site and a non-phosphorylated SalI site. This fragment was ligated with SalI digested pCS3 to obtain pCS3DTerm which results from ligation of an inverted duplication of the synthetic oligonucleotides (formed by annealing the BglII ends of two of the synthetic oligonucleotides) to insert a symmetrically duplicated termination sequence pair. The vector fragment from EcoRI/XhoI digestion of pCS3DTerm (see Fig. 5) thus provides that portion of the termination sequence downstream from the XhoI site. The thus digested BAPed vector from pDC3DTerm was ligated with a fragment containing the $P_LN_{RBS}$ cassette operably linked to the DT-A fragment and the upstream XhoI portion of the termination sequence. This latter fragment is obtained by EcoRI/XhoI (PstI to prevent religation) digestion of $P_LP_{trp}$ Switch A (see below). The resulting plasmid, $pP_LOPSau$, includes the $P_LN_{RBS}$ sequence immediately preceding the ATG start codon for DT-A linked to mature protein encoding sequence of Mbo with its terminator.

$pP_LP_{trp}$ SwitchA is prepared from pTrpSmIMbo and pFC5 as follows:

55 µg pTrpSmIMbo was restricted with EcoRI, HindIII and PstI, and the vector portion treated with BAP. 5 µg of the vector fragment was ligated to 5 µg of HindIII/PstI digested pFC5. 0.3 µg of the ligation mixture DNA was transformed into MC1000-39531 cells, $Amp^R$ clones selected and the desired construction confirmed by restriction analysis, and designated $pP_LP_{trp}$ SwitchA. (Growth of these transformants on TYE medium containing 100 µg/ml ampicillin at 30° for 7 hr, followed by heating to 60°C to raise quickly the medium temperature to 42° and growth at 42°C for 1 hr resulted in production of DT-A at a concentration of 5-20 µg/ml as assayed by the procedure of Chung & Collier, Infect Immun (1977) 16:832.)

F.6.b. Preparation of pPLOPMspRT (DT A-B′ Toxin)

pPLOPMspRT is constructed as shown in Figure 6. EcoRI, SalI digested/BAPped pCS3 was ligated to EcoRI/SalI (PstI to prevent religation) digested $pP_LMspRT$ (see below). The resulting ligation mixture was transformed into MC1000-39531 and the $Amp^R$ $Tet^S$ transformants analyzed by restriction analysis to confirm the correct construction of $pP_LOPMspRT$ which contains properly started and terminated DT-A-B′ under the control of the $P_LN_{RBS}$ cassette.

pPLMspRT derives from pATGMspRT as the source of the protein coding sequence with a properly placed start codon preceding the first amino acid of the native DT-A, and pFC5 as the source of the $P_LN_{RBS}$ cassette. It was constructed by taking advantage of the portability of the $P_LN_{RBS}$ cassette. A PstI/HindIII digest of pFC5, was mixed with a PstI/HindIII (EcoRI to prevent religation) digest (BAPed) of pATGMspRT to give the desired $pP_LMspRT$. The ligation mixture was used to transform MC1000-39531 and $Amp^R$, $Tet^S$ colonies selected. Correct construction of $pP_LMspRT$ was confirmed by plasmid isolation and restriction.

pATGMspRT, in turn, contains the coding sequence for DT-A-B′ (with the termination sequence) immediately preceded by the ATG start codon. It is constructed in a 3-way ligation from pTrpSmIMbo, pBR322, and pMspRT, as shown in Figure 6 (pMspRT contains the Msp fragment and the synthetic terminator cloned into pBR322).

pTrpSmIMbo was restricted with Sau3AI and HindIII, and the 586 bp fragment containing the ATG start codon, A fragment codons and Mbo terminator isolated. (The Sau3AI site is immediately upstream of the XhoI sequence of the terminator.) pMspRT, used as a source of the B′ fragment along with its terminator, was treated with Sau3AI, and the 750 bp fragment containing the above-mentioned portions isolated. This latter fragment was then restricted with SalI. The two fragment preparations were then ligated into the vector fragment from HindIII/SalI double digested, BAPed pBR322 and transformed into E. coli MM294 and the desired construction pATGMspRT verified.

The construction of pMspRT is also shown in Figure 6. An Msp fragment, isolated as above, was digested with HindIII, and ligated into HindIII/SalI digested, BAPed vector fragment of pBR322 in a three-way ligation, along with kinased and annealed Msp terminator (synthetically derived as shown in Figure 6). The ligation mixture was transformed into E. coli MM294, plasmids isolated from $Amp^R$ $Tet^S$ colonies, and

the correct construction confirmed by sequencing.

F.6.c. Construction of $pP_LOPMspCys$ (DT-A-B′ with Cys

In order to provide a cysteine residue for use in linkage to form conjugate toxins, the DT-A-B′ fragment was modified by adding a cysteine residue. In so doing, of course, the fragment maintains its DT-A-B′ fragment status as set forth in the definition section above.

By providing a cysteine residue at the carboxy terminus, the DT-A-B′ fragment is now capable of forming a thioether linkage with a suitable reactive linker molecule which can, in turn, be covalently bound to a suitable antibody or antibody fragment to confer specificity on the DT-A-B′ fragment. Suitable linker molecules which form thioether linkages at one end and ester linkages or amide linkages at the other include 6-maleimidocaproic acid, 2-bromoacetic acid, 2-iodoacetic acid, all of them in their acyl activated derivatized forms such as, the succinimidyl ester, as set forth above.

The construction contains the desired coding sequence under the control of $P_LN_{RBS}$ on a high copy number plasmid analogous to the previous constructions for DT-A and DT-A-B′ above. It is outlined in Figure 7.

pCS3 was cleaved with EcoRI/SalI and BAPed to provide the vector fragment providing for high copy number. This vector fragment was ligated with $pP_LMspCys$ (see below), which had been restricted with EcoRI, SalI (and PstI to prevent self ligation). Successful $Amp^RTet^S$ transformants into MC1000-39351 were isolated, and the desired construction confirmed by plasmid isolation and restriction analysis. A successful construction was designated $pP_LOPMspCys$.

The construction $pP_LMspCys$ is also outlined in Figure 7. The vector fragment and the $pP_LNRBS$ segments are provided by a HindIII/SalI (BamHI to prevent religation) restriction digest of $pP_L322$. The coding sequences for DT-A and the B′ fragment up to the Msp restriction site are provided by an MspI/HindIII digest of pATGMspRT. The 1151 bp fragment is isolated from this double digest for ligation. The terminating sequences for the cys-containing construct are provided by the synthetic double-stranded oligonucleotide

```
5'HO CGGGGTGCTGACCCGGG
              CCCACGACTGGGCCCAGCT P5'
     BglII                  SalI
```

shown in Figure 5, which is an MspI-SalI fragment. These three fragments were ligated under standard conditions and the ligation mixture used to transform MC1000-39531. Successful transformants ($Amp^R$ $Tet^S$) were selected, plasmid DNA isolated, and the correct construction, $P_LMspCys$ confirmed by sequence analysis.

F.7. Production of Recombinant Proteins

Fresh overnights on transformants of $pP_LOPSau$, $pP_LOPMspRT$, $pP_LOPMspSA$ and pPLOPMspCys were inoculated into N8-2 media (per 500 mls: 20 mM $NH_4Cl$, 44 mM $KH_2PO_4$, 56.2 mM $Na_2HPO_4$, 18 mM $K_2SO_4$, 0.4 mM $MgSO_4$, 6 $\mu$M $ZnSO_4$, 6 $\mu$M $MnSO_4$, 0.2 $\mu$M $CuSO_4$, 0.4% glucose, 0.002% thiamine) supplemented with 5 g/l casamino acid, 5 g/l glucose, 100 $\mu$g/ml ampicillin and 10 $\mu$M $FeSO_4$. The cells were grown at 30°C to an $OD_{680}$ of 0.150, and induced at 60°C for 45 sec followed by growth at 42°C for 5 hour. To assess the level of production, 0.5 ml of cells were centrifuged and the pellet resuspended in 20 $\mu$l 3% SDS, 62.5 mM Tris HCl, pH 6.8. Following heating at 95°C for 5 minutes the samples were run on 12.5% SDS polyacrylamide 3% stacking gel. The results are shown in Figure 8.

The quantity of DT-A or DT-A-B′ protein produced was assayed in each case by measuring quantitatively the intensity of the relevant protein band illustrated in Figure 8 upon staining with Coomassie Blue. These intensities corresponded to values of approximately 150 $\mu$g/ml cell culture for hosts transformed with any of $pP_LOPMspSA$, $pP_LOPSau$, $pP_LOPMspRT$ or with $pP_LOPMspCys$.

The identity of each of the stained bands to the desired DT fragment was confirmed in each case by the EF-2 ADP-ribosylation assay of Chung (supra), and by Western Blot.

10-20 g cells transformed with pPLOPMspRT were resuspended in 20 ml 50 mM Tris, 1 mM EDTA pH 8.2 and sonicated. Following centrifugation, the supernatant was diluted approximately 5 fold in 5 mM Na

phosphate, pH 6.8, 0.5 M NaCl and applied to a phenylsepharose column. The desired peptide eluted in approximately 5 mM Na phosphate, pH 6.8, giving a fraction of greater than 80% purity.

A similar procedure attempted on the cells harboring $pP_LOPMspCys$ did not result in any soluble peptide. The desired peptide remained with the pelleted cells during centrifugation, and was not recoverable in the supernatant.

Sixteen grams of cells transformed with $pP_LOPMspSA$ were resuspended in 20 ml 50 mM Tris, pH 8.2, 1 mM EDTA, 10 mM DDT and sonicated. The supernatant from centrifugation was diluted 5-10 fold in 5 mM Na phosphate, pH 6.8, 10 mM DTT and applied to a DEAE Sephacel column. The protein was eluted using a 0-300 mM NaCl gradient and 5 mM Na phosphate, pH 6.8, 10 mM DTT. The fractions containing the desired peptide were pooled, dialyzed against 5 mM Na phosphate, pH 6.8, 10 mM DTT and loaded onto an NAD-Agarose (P.L Biochemical TYPE1) column. Following elution using a 0-1 M NaCl gradient in the same buffer, desired fractions were pooled, concentrated, and run over a Sephacryl S-200 sizing column and the resulting fractions estimated to be 80% pure.

A similar procedure is used to recover the recombinant protein from cells transformed with pPLOPSau to obtain the DT-A fragment.

## G. Recombinant Ricin Fragments

Portions of the ricin toxin protein were also prepared using recombinant techniques. Both A and B chains have been cloned and expressed, as set forth below.

### G.1. Isolation of Messenger RNA

50 g of immature castor beans (Rininus communis) were placed in 100 ml homogenizing buffer (150 mM NaCl, 50 mM Tris, pH 8.3, 5 mM EDTA and 50 mM freshly added β-mercaptoethanol) to which was added 12 ml 0.2 M vanadium-ribonucleoside complex, * 30 mg proteinase K, and 15 ml 20% SDS. The mixture was homogenized by blending at high speed for 3-4 min in a Waring blender and then incubating 2-3 hr at room temperature with occasional blending.

The suspension was centrifuged for 15 min at 8000 x g at 5° C and the pellet discarded. The supernatant was strained through cheesecloth to remove lipids and the filtrate extracted sufficiently with phenol:$CHCl_3$:isoamyl alcohol, 24:24:1 containing 1% hydroxy-quinoline, to remove vanadium salts and protein, and the aqueous layer brought to 0.4 M with NaCl and 10 mM with EDTA. 2.5 x volume absolute ethanol was added to precipitate nucleic acids, and the mixture stored at -20° C overnight.

The precipitate was centrifuged for 15 min at 7000 x g at 2° C, and the pellet resuspended in 9.5 ml aqueous solution 0.025 M NaCl, 0.025 M Tris, pH 8, plus 9.5 ml phosphate buffer (2.5 M total phosphate, $K_2HPO_4$:33% $H_3PO_4$, 20:1), plus 9.5 ml 2-methoxyethanol. The mixture was shaken and chilled on ice for 3-5 min with occasional mixing, and then centrifuged at 2000 x g for 5 min at 2° C. The upper layer was removed and to this was added 10 ml 0.2 M sodium acetate, and 5 ml 1% cetyl trimethylammonium bromide (CTAB) and the mixture chilled on ice for 10 min. The resulting white precipitate was harvested by centrifugation at 2000 x g for 10 min at 2° C. The precipitate was washed by addition of 70% ethanol in 0.1 M sodium acetate and by re-centrifuging at 2500 x g 10 min at 4° C.

After removal of the supernatant, the pellet was resuspended in 2 ml G-100 column starting buffer (20 mM Tris, pH 8,1 mM EDTA, 0.5% SDS), and then adjusted to contain 0.5 M NaCl. Solids were removed by centrifugation at 2000 xg for 5 min at room temperature and the supernatant applied to a Sephadex G-100 column (1.5 cm x 40 cm) and the column eluted using buffer similar to that applied to the column but lacking SDS. The eluate was assayed by monitoring $OD_{260}$. Desired messenger RNA was obtained in the flow through volume, leaving behind oxidized phenolic compounds present in the plant extract. (These compounds are known to behave similarly to polyA RNA on dT columns, inhibit protein synthesis, and thus

*The vanadium ribonucleoside solution is prepared as follows: 893 mg $VOSO_4 \cdot 3H_2O$ was added to 2 ml water and the mixture boiled to dissolve the vanadium salt. A solution containing the 4 ribonucleosides was prepared by dissolving 1 mmole each of adenosine, cytidine, guanosine, and uridine in 17 ml water. Heat is required. 1 ml fo the foregoing $VOSO_4$ solution was then added to the ribonucleoside solution and the resultant titrated to pH 6.5 with 10 N NaOH, and finally to pH 7 with 1 N NaOH while stirring in a boiling water bath. Formation of the complex is indicated by a change in color from bright blue to green-black. The solution was finally diluted to 20 ml with water.

interfere with the assay for mRNA.

The initial peak containing mRNA was treated with formamide, a denaturant, to destroy ribosomal RNA complexing. To do this, the mRNA containing fractions were pooled, precipitated in ethanol, and the precipitates redissolved in a minimum volume of water. To this solution was added 9 volumes of deionized formamide containing 20 mM PIPES (piperazine-N,N-bis(2-ethanesulfonic acid), pH 6.5-7.0.

The mixture was then warmed to 37° C for 5 min, and 10 volumes of dT column buffer (0.5 M NaCl, 10 mM Tris, pH 7.5, 1 mM EDTA) added. The presence of formamide dissociates the polyA RNA from any ribosomal RNA present.

The denatured mixture was then run over an oligo dT column according to procedures well established in the art, and approximately 100 µg polyA RNA recovered upon elution.

### G. 2. Preparation of a cDNA Library and Isolation of Ricin B Clones

#### G.2.a. cDNA Preparation

A portion of the polyA RNA was treated under appropriate buffer conditions with reverse transcriptase and then treated with base to destroy the remaining mRNA. The resulting single-stranded cDNA was repaired using *E. coli* Polymerase I (Klenow fragment) in the presence of the 4 dNTPs and the resulting "hairpin" then ligated using T4 ligase to a SalI linker (obtained from New England BioLabs). After treating with S1 nuclease and repairing with Klenow, the blunt end was ligated to an EcoRI linker using $T_4$ ligase. After then digesting with EcoRI and SalI, the resulting double-stranded cDNA fragments, which are bounded by EcoRI and SalI restriction sites, were ligated into a EcoRI/SalI digested, BAP treated preparation of pUC13 obtained and freely available from J. Messing, the University of Minnesota. pUC13 is a modification of pBR322 capable of conferring Amp resistance ($Amp^R$), and bearing a lac promoter control sequence upstream of linkers bearing convenient restriction sites, including EcoRI and PstI sites downstream from the SalI site used in the insertion; sites which are identical to those found in the analogous M13 phage cloning vectors. The resulting ligation mixture was used to transform *E. coli* MM294, and $Amp^R$ strains selected.

#### G.2.b. Clones Containing RTB Sequence

Successful colonies were transferred onto nitrocellulose plates, and probed using the procedure of Grunstein & Hogness (supra), with the mixture of 16 synthetic oligonucleotides

5′ CC($^{A}_{G}$)TC($^{A}_{G}$)TT($^{C}_{T}$)TT($^{A}_{G}$)AACATCC 3′

which is kinased with $^{32}P$. This mixture represents the anti-sense strand complementary to the codons for the amino acid sequence Trp-Met-Phe-Lys-Asn-Asp-Gly. Of about 5000 colonies probed about 1% were found which hybridized to the probe. Plasmids were isolated from several representations of these colonies, and analyzed by restriction analysis and Maxam-Gilbert sequencing. Three plasmids, pTRB4, pRTB5, and pRTA115 were sequenced in the insert region, and the results are shown in Figure 9.

Figure 10 shows a comparison of the ricin B amino acid sequence published by Funatsu, G., et al, *Agric Biol Chem* (1979) 43:2221, with that deduced from RTB5. The amino acid sequence deduced from the pRTB5 base sequence shows a high level of correspondence to ricin B, although some discrepancies exist. These are due to possible errors in the published sequence and to varietal differences in the ricin B proteins represented. The entire coding sequence for ricin B is present in pRT5 except for codons for the first 11 amino acids. pRTB5 was used as the source of the bulk of the coding sequence in the expression vectors. The pRTA115 insert contains the upstream coding regions of the ricin B gene. Although pRTA115 is believed associated with the RCA precursor protein, the amino acid sequence deduced from pRTA115 for RCA matches that of ricin B for the 11 amino acids needed to complete the N-terminus. These sequences were therefore used as models for the construction of oligonucleotides encoding the missing 11 N-terminus codons and also permit the deduction of the amino acid sequence of the 12 amino acid peptide in the single peptide precursor of RCA and, presumably, of ricin A and B.

#### G.2.c. Construction of pRTB601:RTB as a HindIII Cassette

The construction of pRTB601, as well as its intermediate plasmid pRTB151, from pRTB5 is shown in

Figure 11.

The coding sequences of pRTB5 were disposed so as not to be expressible under the control of the lac promoted as inserted into pUC13. Therefore, pRTB5 was cut with EcoRI and PstI and the vector cleaved into several fragments with BstNI. The insert fragment was ligated under standard conditions using T4 ligase with an EcoRI/PstI digest of pUC8, another modified pBR322 vector obtained from and freely available from Messing, J., at the University of Minnesota. pUC8 has EcoRI and PstI sites which place an EcoRI/PstI insert under lac promoter control as a fusion protein with the initial 5-8 amino acids of β-galactosidase. It also contains a HindIII site immediately downstream from the PstI site. The ligation mixture was transformed into E. coli MM294, and transformants selected for ampicillin resistance. Plasmid DNA was isolated from successful transformants in several colonies, and analyzed by restriction site mapping. Colonies showing the appropriate restriction patterns were selected. One colony, designated pRTB151, was tested for expression of the gene for the fusion protein. On Western Blot no protein band corresponding to the desired molecular weight was found, although cross-reacting proteins were produced. It was assumed that the reading frame might be improper, since this plasmid was designed to have the β-galactosidase and ricin B sequences in different phases.

Ten μg of pRTB151 DNA was digested to completion with EcoRI, dissolved in 60 μl S1 buffer and digested for 4 min at room temperature under conditions which remove about 1 base pair of duplex DNA per min. DNA recovered from the foregoing buffer was dissolved in 60 μl exonuclease III buffer and digested for 4 min at room temperature. Subsequent analysis showed that the plasmid DNA had lost approximately 120 bp from each 3′ end, leaving 5′ ends available for hybridization. DNA recovered from the Exonuclease III buffer was dissolved in 50 μl water and 20 μl used in the ligation/repair reaction below.

Thus, 20 μl sample (2 pmoles) was mixed with 20 pmoles each of the synthetic oligonucleotides:

```
                          Oligo 2
5'- GACCATGATAAGCTTATGGCTGATGTTTGTATGGATCC and
          HindIII     3'TACCTAGGACTCGGGTATCACGCATAGCATCC-5'
                                            Oligo 1
```

which have complementary sequences as shown, and wherein Oligo-2 encodes a HindIII site upstream of an ATG start codon as shown in Figure 12a. The 5′ end of Oligo-1 is complementary to 15 bases at the 5′ end of the pRTB151 cDNA sequence as there shown and is complementary to the contiguous missing codons of the ricin B sequence. The 5′ end of Oligo-2 is complementary to the 5′ sticky end of the vector residue of the exonuclease III treated pRTB151.

The mixture was heated to 60° for 5 min in order to denature completely complementation of single-stranded DNA, cooled to 37° for 5 min to hybridized complementary strands, and then chilled on ice. The soluton was brought to polymerase I (Klenow) buffer conditions and reacted for 2 hr at 12° in the presence of the 50 μM each of the 4 dNTPs, 0.1 mM NAD, 0;3 units/μl Klenow, and 0.08 units/μl E. coli DNA ligase. The ligation mixture was used directly to transform competent E. coli MM294 and several thousand $Amp^R$ colonies found. Several hundred of these were replicated and grown on nitrocellyulose filters and subjected to standard colony hybridization using $P^{32}$ kinased Oligo-2 as probe. Two clones which hybridized with the probe were analyzed by restriction analysis and sequenced, and a correct construction designated pRTB601. pRTB601 thus contains the ricin B coding sequence as a HindIII cassette. The upstream HindIII site is introduced immediately upstream of the ATG codon in Oligo-2; the downstream HindIII site arises from the pUC8 vector plasmid.

### G.3. Construction of Ricin B Expression Vectors

#### G.3.a. Trp Promoter Control

pRTB236 is a precursor to target vector pRTB514 and it places the codons of pRTB5 under the control of lac Z promoter in proper reading frame so as to produce a fusion protein containing the initial 5 residues of β-galactosidase at the amino terminus followed by the peptide encoded by the cDNA insert in pRTB151. Thus, the resulting protein is a sequence begun with the β-galactosidase N-terminus and completed with the portion of ricin B lacking the first 11 amino acids.

To construct pRTB236, pRTB151 was modified to the correct reading frame as follows: pRTB151 was digested with EcoRI and treated with S1 nuclease to remove the EcoRI protruding ends. The reaction mixture was then religated using T4 DNA ligase, and transformed into *E. coli* MM294. $Amp^R$ colonies were selected, and a number of colonies sequenced. pRTB was found to be in the correct reading frame (the determined relevant sequence is shown in Figure 12b), and this transformed strain was capable of producing ricin B protein as shown by Western Blot.

pRTB514 contains the entire coding sequence of ricin B chain preceded by an ATG start codon under control of the trp promoter/operator system. The trp promoter operator system is derived from plasmid pDG141.

3 µg of pRTB601 was digested with HindIII to excise the ricin B coding sequence. The restriction digest was treated with FnuDII to destroy the $Amp^R$ region of the vector remainder, and ligated using T4 DNA ligase with a HindIII digest of 1 µg pDG141. The ligation mixture was transformed into *E. coli* MM294, and $Amp^R$ colonies selected. The desired plasmid pRTB514 was isolated from one colony and the correct construction confirmed by Maxam-Gilbert sequencing. The relevant sequence is shown in Figure 12.

### G.3.b. $P_L$ Promoter Control

pRTB704 is analogous to pRTB514 except that the coding sequence is under the control of the $P_L$-$N_{RBS}$ cassette. The construction is identical to that for pRTB514 except that pFC5 rather than pDG141 is used as a source of the HindIII digested plasmid which provides the promoter/operator and ribosome binding site encoding sequences. Alternatively, rather than pFC5, $pP_L322$ or $pP_LKan$ can also be used.

pRTB907 is analogous to pRTB704 except that the plasmid vector is a temperature sensitive high copy number plasmid. pRTB907 is derived from pRTB704 by excising the control and the coding sequences as an EcoRI/SalI digest, cleaving the pRTB704 vector portion with HhaI, and ligating this segment into EcoRI/SalI digested pCS3 described above. The ligation mixture was then used to transform MC1000-39531 and transformants selected by $Amp^R$. The correct construction of the desired plasmid was confirmed by restriction analysis.

The constructions of pRTB514, pRTB704 and pRTB907 are shown in Figure 13.

### G.4. Preparation of a cDNA Library and Isolation of Ricin A Clones

The polyA mRNA prepared as in the preceding paragraph was used to obtain a cDNA library according to the method of Maniatis, et al (supra). Briefly, a portion of the polyA RNA is treated under appropriate buffer conditions with reverse transcriptase in the presence of the primer and then treated with base to destroy the remaining mRNA. A poly dC oligomer is added to the 3′ end of the single strand using terminal transferase under standard conditions. The resulting single-stranded cDNA is converted to the double stranded form using oligo-dG as primer, employing reverse transcriptase. The double stranded cDNa is then inserted into the PstI site of pBR322 by tailing the cDNA with oligo-dC and the cleaved vector with oligo-dG, and annealing. The resulting mixture was used to transform *E. coli* MM294, and 5000 $Amp^R$ strains obtained.

### G.4.b. Preparation of Ricin A Chain Clones

Successful colonies were transferred onto nitrocellulose plates, and probed using the procedure of Grunstein % Hogness (supra), with the mixture of four synthetic oligonucleotides:

A
T
5'-GCATCTTCTTGGTTGTCCGGATGAAAGAAATAGGC-3'
G

which are kinased with $^{32}P$. This mixture represents the anti-sense strand complementary to the codons for the amino acid sequence contained in ricin A with most codon ambiguities resolved by sequencing cDNA synthesized using a mixtures of primers to a portion of the codon sequence and employing the experimentally determined sequence of the synthesized cDNA. Plasmids were isolated from several representative positively hybridizing colonies, and analyzed by restriction analysis. Two groups thus obtained appeared to correspond to ricin A and to agglutinin A. Two plasmids, one from each group, pRA123 and pRA45 were sequenced in the insert region.

Figures 14 and 15 show the results of this sequencing. Figure 14 shows the sequence of the insert in pRA123. The base sequence permits the deduction of an amino acid sequence presented immediately below the nucleotide sequence in the figure. It can be compared to that of isolated protein, labeled RTA in the figure; only six residues differ. These difference may be due to errors in the published seqence and/or to varietal differences in the ricin A proteins represented. The entire coding sequence for ricin A is present, as well as codons for the 12 amino acids joining the A to B chain, and for the signal sequence. pRA123 was used as the source of the the coding sequence in the expression vectors described below after modification to provide correct start and stop codons. pRA123 was deposited with ATTC on 17 August 1984 and has deposit no. 39799.

Figure 15 shows the sequence deduced for ricin agglutinin A from a combination of sequences in pRTA115 (see Figure 9) and of pRTA4 and pRTA5. In that figure, the bae sequence for this composite is shown, and the line immediately below it represents the deduced amino acid sequence. To show the differences from ricin toxin A, that sequence, labeled RTA, is also included in the figure. The cysteine residues at positions 84 and 156 in the agglutinin sequence represent major differences from the toxin sequences obtained.

### D.3. Modification of pRA123

pRA123 which contains the entire ricin A coding sequence was modified so that this sequence would be obtainable as a HindIII/BamHI cassette with a termination codon in the proper position after amino acid 267, and a start codon in the position immediately preceding the mature sequence. pRA123 was digested with BamHI, and the approximately 896 bp fragment isolated and subcloned into M13mp18 in an anti-sense orientation relative to the lac promoter in the M13 vector. The phage single stranded DNa was subjected to primer directed mutagenesis using the sequences shown as superscripts 2 & 3 in Figure 14 as primers. The oligonucleotide shown near the beginning of the A chain sequence (2) imparts modifications which insert an ATG start codon immediately preceding the A toxin N-terminal amino acid and a HindIII site in turn, immediately upstream of the ATG. The primer placed near the C-terminal end of the toxin coding sequence (3) replace the serine codon with a TAA termination codon. The resulting modified phage were identified after each mutagenesis using the appropriate above primers as probes. The desired constructs were double digested with HindIII and BamHI and the appropriate ricin A encoding fragments isolated.

If vectors are to be prepared for expression of the complete ricin sequence, the mutation directed by primer 3 which alters the serine residue to a terminantion codon is omitted. If secretion of either ricin A or ricin is desired, the mutation directed by primer 2 is omitted, and that directed by primer labeled 1 in Figure 14, in the region of the start codon for the signal sequence is substituted. This modification results in provision of a HindIII site immediately preceding the ATG codon of the signal sequence. Thus, in addition to the above described modifications which enable construction of vectors for ricin A production intracellularly, sequences can be provided for construction of vectors which result in secretion of ricin A, or of intracellular production or secretion of the entire ricin sequence. The vectors designed for secretion must, of course, be constructed for expression in appropriate hosts, as set forth above.

### G.5. Preparation of Expression Vectors for Ricin A

The HindIII/BamHI fragment prepared in the previous paragraph was ligated into the host expression vectors pTRP3 and pPLOP digest with HindIII/BamHI.

Ligation products with pTRP 3 were transformed into E. coli MM294 to $Amp^R$. Plasmids were isolated from selected colonies. Two of these plasmids pRAT7 and pRAT1 (CMCC 2115) were shown to contain the entire RTA sequence.

Ligation products with pLOP were transformed into E. coli MC1000 lambda lysogen, and plasmids isolated from two of the $Amp^R$ colonies were designated pRAL6 (CMCC 2114) and pRAL7. These were also

shown to contain the complete RTA coding insert.

In a similar manner, plasmids designed to express secreted ricin A are constructed using the HindIII/BamHI fragment prepared from pRA123 which had been subjected to primer directed mutagenesis using primers 1 and 3 in Fig 14. These plasmids contain the coding sequence for ricin A along with the signal sequence in operable linkage to suitable eucaryotic control sequences. In an appropriate vector/host system, ie, eg, yeast, plant or mammalian cells, expression of the coding portions of these plasmids results in the secretion of ricin A, rather than intracellular production and retention.

## G.6. Expression Plasmids for Whole Ricin

Similarly, plasmids pRABT and pRABL which express complete ricin chains, can be prepared from intermediates made by using the pRA123 sequences modified by mutagenesis with primer 2 only and primer 1 only respectively. Into these intermediates the coding sequences for the B portion of ricin is inserted. The intermediate plasmids in each case, obtained as described above, are treated with BamHI and SalI followed by BAP to obtain a vector fragment which will frame the B-portion coding region and a portion of the B-donor vector sequences. pRTB704 is used as the donor of the B-portion containing fragment.

To obtain the "B" fragment, pRTB704 is digested with SalI, then partially with BamHI, and the fragments are separated by agarose gel electrophoresis. The large fragment containing the B portion sequence from the BamHI site immediately 3′ of the N-terminal amino acid, to the SalI site in the pUC vector fragment is thus isolated. This fragment is then ligated with the BAPed vector, and the ligation mixture transformed into *E. coli* and selected for $Amp^R$. Successful transformants are grown, plasmid DNA isolated, and used to transform the suitable corresponding host for expression of the complete ricin chain.

## G.7. Summary

Thus, in summary, representative vectors applicable to procaryotic host expression include:

| Vector | pRA123 modified w/primer | expression vector for | promoter/host |
|---|---|---|---|
| pRAT1 | 2,3 | intracellular ricin A | trp/MM124 |
| pRAL6 | 2,3 | intracellular ricin A | $P_L$/MC100 λlysogen |
| pRABT* | 2 | intracellular ricin | trp/MM124 |
| pRABL* | 2 | intracellular ricin | $P_L$/MC100 λlysogen |

*These represent vectors completed by a BamHI/SalI fragment insert from pRTB704.

If the ricin A or ricin is to be secreted, pRA123 will be modified using primers 1 and 3 or solely 1 respectively, and the modified sequences ligated to eucaryotic control systems.

## G.8. Expression of Recombinant Ricins

### G.8.a. Ricin A

Figure 16 shows Western blots performed by the method of Erlich, H. A., et al, *Infect Immunol* (1983) 41: 683, of cell extracts from suitable *E. coli* hosts transformed with pRAL6, pRAL7, pRAT1 and pRAT7. Immunoreactivity was obtained with antibodies raised against natural ricin A in rabbits. The mobility of the protein product noted as 'rec a' in the figure is consistent with that of a non-glycosylated form of ricin A relative to the mobilities of the native, glycosylated form, denoted ricin $A_1$ and $A_2$ in the figure. No immunoreactivity, except for acceptable background, was noted in extracts from cells cultured under identical conditions which contained the vectors, pLOP and pTRP3, plasmids which do not bear ricin sequences. Analysis of Coomassie Blue stained SDS-polyacrylamide gels and of radioautographs of parallel Western blots permits estimates of production levels. For the pRAT1 transformants the recombinant RTA

represents approximately 0.5% of total cell protein. For the pRAL transformants, the RTA can be approximately 5% of total cell protein.

To obtain purified, active protein, cultures of the foregoing transformants were lysed by sonication and the insoluble material recovered. This material was treated with a chaotropic agent, 8M urea containg 0.5% SDS, to solubilize it and disperse protein aggregates. The resulting suspension was centrifuged to pellet residual insolubles and the supernatant applied to a Sephacryl S-200 (Pharmacia Co.) column to fractionate the protein components. Fractions containing approximately 80% homogeneous ricin A protein were identified using polyacrylamide gel analysis and these fractions assayed for enzymatic activity associated with ricin A, i.e., the ability to inhibit protein synthesis in a rabbit reticulocyte in vitro translation system (a commercially available system obtainable, e.g., from Bethesda Research Laboratories, Rockville, Maryland). The purified protein was biologically active in this assay.

G.8.b. Ricin B

E. coli MC1000-39531 transformed with either of the plasmids, pRTB704 and pRTB907, were grown at 30 °C in TYE′ medium (15 g/l bactotryptone, 10 g/l yeast extract, 5 g/l NaCl supplemented with 1 mM $CaCl_2$) containing 100 µg/ml ampicillin. While in logarithmic growth phase, the temperature was increased to 42 °C, and after 1.5 hr the cells were centrifuged and extracted with SDS. The extract was assayed by Western Blot as described above. The results are shown in Figure 17, and contrasted with the results obtained with the analogous pRTB514 (using trp promoter) and with precursor and control plasmids. The lanes depicted are as follows: Lane 1, "native" ricin B, lanes 2-9, extracts of transformants with, respectively, pUC8, pRTB5, pRTB151, pRTB221 (a modified pRTB151), pRTB236, pRTB514, pRTB704, and pRTB907.

H.1. Assay for Cytotoxicity

The DT-A-B′ spacer was conjugated with an antibreast monoclonal antibody 260F9, (hybridomao deposited at the ATCC on 27 January 1984 under accession number HB8488. Ricin toxin A chain (RTA) or diphtheria toxin A chain (DTA) which contain free sulfhydryl groups for analogous conjugation with the antibody were thus conjugated. These conjugates were assayed for immunotixicity.

To form the cnjugate, breast monolconal antibody 260F9 or other antibodies as specified below were first derivatized with SPDP or

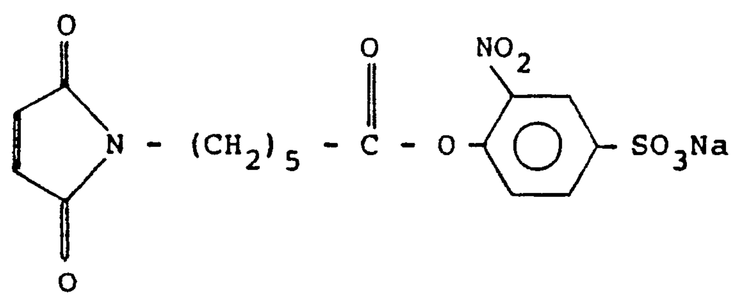

(mal-sac-HNSA). The antibodies derivatized to SPDP were used to form disulfide links to the free cysteine sulfhydryls of DT-A-B′-spacer DTA or RTA. Those derivatized with mal-sac-HNSA were used to form thioether linkages with DT-A-B′-spacer.

For SPDP, a 10-20 fold molar excess of SPDP was added to a solution containing 20 mg/ml of antibody in PBS and incubated at room temperature for 1 hr, and then dialyzed against PBS to remove unreacted SPDP. It was calculated that approximately 2-5 pyridyl-disulfide moieities were introduced into each antibody using this procedure.

To complete the conjugation with SPDP to give a disulfide linkage to the cytotoxic portions, DT-A-B′-spacer solution or solution of RTA or DTA containing 1-2 mg/ml which had been stored in reducing agent in 4 °C was passed over a Sephadex G-25 column equilibrated in PBS to remove the reducing agent, and the DT-A-B′-spacer or other cytotoxic portion was mixed with derivatized antibody in 2-4 molar excess cytotoxic portion. Conjugation was confirmed by spectrophotometric determination of released pyridine-2-thiol and by SDS-PAGE.

For mal-sac-HNSA, approximately 0.2 ml of mal-sac-HNSA solution containing 1 mg/ml was added to 1 ml antibody solution containing 3-8 mg/ml in PBS. The mixture was kept at room temperature and monitored until 5 mal-sac-HNSA moieties were incorporated per antibody. The reaction was then stopped by desalting the mixture on a G-25 column equilibrated in 0.1 M Na phosphate, pH 6.

The DT-A-B′-spacer, stored in reducing agent at 4° C, was passed over PBS-equilbiated Sephadex G-25 to remove reducing agent, and the protein (1-2 mg/ml) mixed in 2-4 molar excess with the derivatized antibody. Conjugation was confirmed by SDS-PAGE.

In a typical protocol, breast tumor cells (MCF-7) were seeded in 8 ml glass vials and dilutions of the immunoconjugates were added. Following incubation for 22 hr at 37° C, the medium was removed and replaced with medium containing $35_S$ methionine. Following a 2-hr pulse, the medium was aspirated, the monolayer was washed twice with 10% trichloroacetic acid containing 1 mg/ml methionine and the vials were dried. Following the addition of 3 ml of 4ad scintillation fluid containing 20% (v/v) Triton X-100, the vials were counted. Toxicity ws expressed as the concentration of protein required to inhibit protein synthesis by 50% ($TCID_{50}$).

The results of these assays are shown in Table 1 both for 260F9, and other antibody partners.

Table 1

| Monoclonal Antibody | $TCID_{50\%}$ (nM) | | |
|---|---|---|---|
| | Ab-DTA | Ab-DT-A-B′-spacer | Ab-RTA |
| ATR[1] | 10 | 2 | 0.1 |
| 260F9 | 30 | 0.3 | 0.1 |
| 106A10 | > 100 | 7 | 1 |
| 208D11[2] | > 100 | 40 | 4 |
| 245E7[3] | > 100 | 50-100 | 10 |
| MOPC21[4] | > 100 | > 100 | > 100 |

1. positive control, anti-transferrin receptor antibody
2. monoclonal antibreast antibody secreted by hybridoma deposited 1/27/84 at ATCC, number HB8487
3. monoclonal antibreast antibody secreted by hybridoma deposited 1/27/84 at ATCC, number HB8489
4. negative control, purchased from Zymed Labs

The foregoing assay was run as set forth above, using the 260F9 conjugate but substituting alternate cell lines for MCF-7. The results are shown in Table 2.

Table 2

| Cell Line | $TCID_{50\%}$ (nM) | | |
|---|---|---|---|
| | RTA | DT-A-B′-spacer | |
| | Disulfide | Disulfide | Thioether |
| MCF-7 | 0.1 | 0.3 | ND |
| CAMA-1 | 0.4 | 1.0 | 0.5 |
| BT-20 | 9 | 1.6 | 1.4 |
| SKBR3 | 0.06 | 0.6 | 0.2 |
| CC95 | > 100 . | > 100 | ND |

The cell lines shown, CAMA-1, BT-20, and SKBR-3 are other breast tumor cell lines; a normal fibroblast cell line CC-95 was also used. The DT-A-B′-spacer was comparably active with respect to the alternative breast tumor lines, but relatively inactive against the normal cells.

The materials listed listed below were deposited with the American Type Culture Collection, Rockville, MD, USA (ATTC). The deposits were made under the provisions of the Budapest Treaty on the International

Recognition of the Deposit of Microorganisms for the Purposes of Patent Procedure and the Regulations thereunder (Budapest Treaty). Maintenance of a viable culture is assured for 30 years from date of deposit. The organism will be made available by ATCC under the terms of the Budapest Treaty, and subject to an agreement between Applicants and ATCC which assures unrestricted availability upon issuance of the pertinent US patent. Availability of the deposited strain is not to be construed as a license to practice the invention in contravention of the rights granted under the authority of any government in accordance with its patent laws.

| Plasmid | Deposit Date | CMCC# | ATCC# |
|---|---|---|---|
| p$\beta$1-Z15 | 13 Jan 1984 | 1948 | 39578 |
| pDG144 | 13 Jan 1984 | -- | 39579 |
| pFC5 | 14 Sept 1984 | 1935 | 39864 |
| pCS3 | 3 June 1982 | -- | 39142 |
| pTRP3 | 18 Dec 1984 | 1731 | 39946 |
| pPLOP | 18 Dec 1984 | 2118 | 39947 |
| pDG141 | 24 Jan 1984 | -- | 39588 |
| pRA123 | 17 Aug 1984 | 2108 | 39799 |
| pRTB704 | 14 Sept 1984 | 1951 | 39865 |
| pRAL6 | 4 Sept 1984 | 2114 | 39833 |
| E. coli K12DG98 | 13 July 1984 | 1965 | 39768 |

## Claims

1. A replicable, recombinant expression vector effective in expressing the gene encoding Ricin A, Ricin B or Ricin in recombinant host cells.

2. A vector as claimed in claim 1 which, when the gene encodes for Ricin A, is pRAT 1 or pRAL 6 or, when the gene encodes for Ricin B, is pRTB 514, pRTB 704 or pRTB 907.

3. Recombinant host cells transformed with a vector as claimed in claim 1 or claim 2.

4. A method of producing Ricin A, Ricin B or Ricin in recombinant host cells which comprises culturing a cell as defined in claim 3 transformed with the respective vector and recovering the Ricin A, Ricin B or Ricin respectively from the culture.

5. A recombinant expression vector, effective in expressing a DNA sequence encoding diphtheria toxin (DT) A or AB′ fragment which comprises a first DNA sequence encoding DT-A or DT-AB′ fragment operably linked to a second control DNA sequence compatible with a transformant host cell, and which does not contain, between the first and second DNA sequence, the native DT control sequence leader encoding sequence or portions thereof and wherein the second, control DNA sequence is a control sequence different from, and other than, the native DT control sequence.

6. A vector of claim 5 wherein the first DNA sequence encodes a DT-A or DT-AB′ fragment which has the sequence Met-Gly-Ala-Asp at its N-terminus.

7. A vector of claim 6 wherein the second control DNA sequence comprises the trp promoter or the $P_L$ promoter.

8. Recombinant host cells transformed with a vector as claimed in any one of claims 5 to 7.

9. A method of producing DT-A or DT-AB′ fragment which method comprises culturing the recombinant cells of claim 8.

10. A conjugate comprising a recombinantly produced cytotoxic portion which comprises Ricin toxin A chain or Diphtheria toxin and a binding moiety which is a monoclonal antibody or an Fab, Fab′2 or Fc fragment thereof, said binding moiety permitting binding specifically to a cell.

```
                                                  Msp I                                                     Mbo I
                                                  CCGGCGTTGCG TATCCAGTGGCTACACTCAGGTTGTAATGA TTGGGATGATGTACCTGATCTGAGAGCGAT

TAAAAACTCATTGAGGAGTAGGTCCCGATT GGTTTTTGCTAGTGAAGCTTAGCTAGCTTT CCCCATGTAACCAATCTATCAAAAAAGGGC ATTGATTTCAGAGCACCCTTATAATTAGGA

TAGCTTTACCTAATTATTTTATGAGTCCTG GTAAGGGGATACGTTGTGAGCAGAAAACTG TTTGCGTCAATCTTAATAGGGGCGCTACTG GGGATAGGGGCCCCACCTTCAGCCCATGCA
                     TerValLeu ValArgGlyTyrValValSerArgLysLeu PheAlaSerIleLeuIleGlyAlaLeuLeu GlyIleGlyAlaProProSerAlaHisAla
                           -31                            -21                            -11                             -1

GGCGCTGATGATGTTGTTGATTCTTCTAAA TCTTTTGTGATGGAAAACTTTTCTTCGTAC CACGGGACTAAACCTGGTTATGTAGATTCC ATTCAAAAAGGTATACAAAAGCCAAAATCT
GlyAlaAspAspValValAspSerSerLys SerPheValMetGluAsnPheSerSerTyr HisGlyThrLysProGlyTyrValAspSer IleGlnLysGlyIleGlnLysProLysSer
                            10                             20                             30                             40

GGTACACAAGGAAATTATGACGATGATTGG AAAGGGTTTTATAGTACCGACAATAAATAC GACGCTGCGGGATACTCTGTAGATAATGAA AACCCGCTCTCTGGAAAAGCTGGAGGCGTG
GlyThrGlnGlyAsnTyrAspAspAspTrp LysGlyPheTyrSerThrAspAsnLysTyr AspAlaAlaGlyTyrSerValAspAsnGlu AsnProLeuSerGlyLysAlaGlyGlyVal
                            50                             60                             70                             80

GTCAAAGTGACGTATCCAGGACTGACGAAG GTTCTCGCACTAAAAGTGGATAATGCCGAA ACTATTAAGAAAGAGTTAGGTTTAAGTCTC ACTGAACCGTTGATGGAGCAAGTCGGAACG
ValLysValThrTyrProGlyLeuThrLys ValLeuAlaLeuLysValAspAsnAlaGlu ThrIleLysLysGluLeuGlyLeuSerLeu ThrGluProLeuMetGluGlnValGlyThr
                            90                            100                            110                            120

GAAGAGTTTATCAAAAGGTTCGGTGATGGT GCTTCGCGTGTAGTGCTCAGCCTTCCCTTC GCTGAGGGGAGTTCTAGCGTTGAATATATT AATAACTGGGAACAGGCGAAAGCGTTAAGC
GluGluPheIleLysArgPheGlyAspGly AlaSerArgValValLeuSerLeuProPhe AlaGluGlySerSerSerValGluTyrIle AsnAsnTrpGluGlnAlaLysAlaLeuSer
                           130                            140                            150                            160
                                                                                                      Mbo I
GTAGAACTTGAGATTAATTTTGAAACCCGT GGAAAACGTGGCCAAGATGCGATGTATGAG TATATGGCTCAAGCCTGTGCAGGAAATCGT GTCAGGCGATCAGTAGGTAGCTCATTGTCA
ValGluLeuGluIleAsnPheGluThrArg GlyLysArgGlyGlnAspAlaMetTyrGlu TyrMetAlaGlnAlaCysAlaGlyAsnArg ValArgArgSerValGlySerSerLeuSer
                           170                            180                            190                            200

TGCATAAATCTTGATTGGGATGTCATAAGG GATAAAACTAAGACAAAGATAGAGTCTTTG AAAGAGCATGGCCCTATCAAAAATAAAATG AGCGAAAGTCCCAATAAAACAGTATCTGAG
CysIleAsnLeuAspTrpAspValIleArg AspLysThrLysThrLysIleGluSerLeu LysGluHisGlyProIleLysAsnLysMet SerGluSerProAsnLysThrValSerGlu
                           210                            220                            230                            240
```

DIPHTHERIA TOXIN CODING & AMINO ACID SEQUENCE

FIG. 1-1

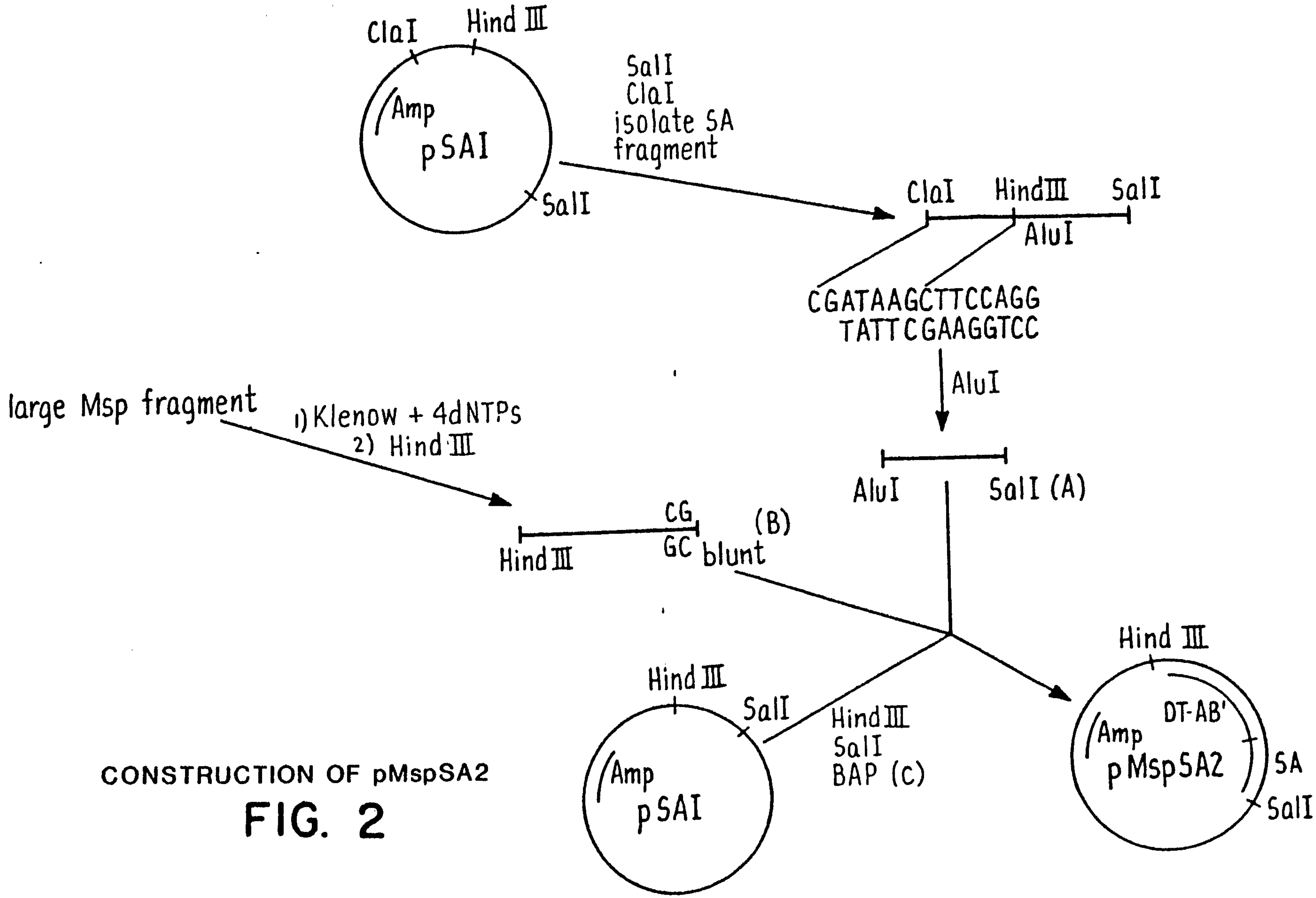


CONSTRUCTION OF pMspSA2

FIG. 2

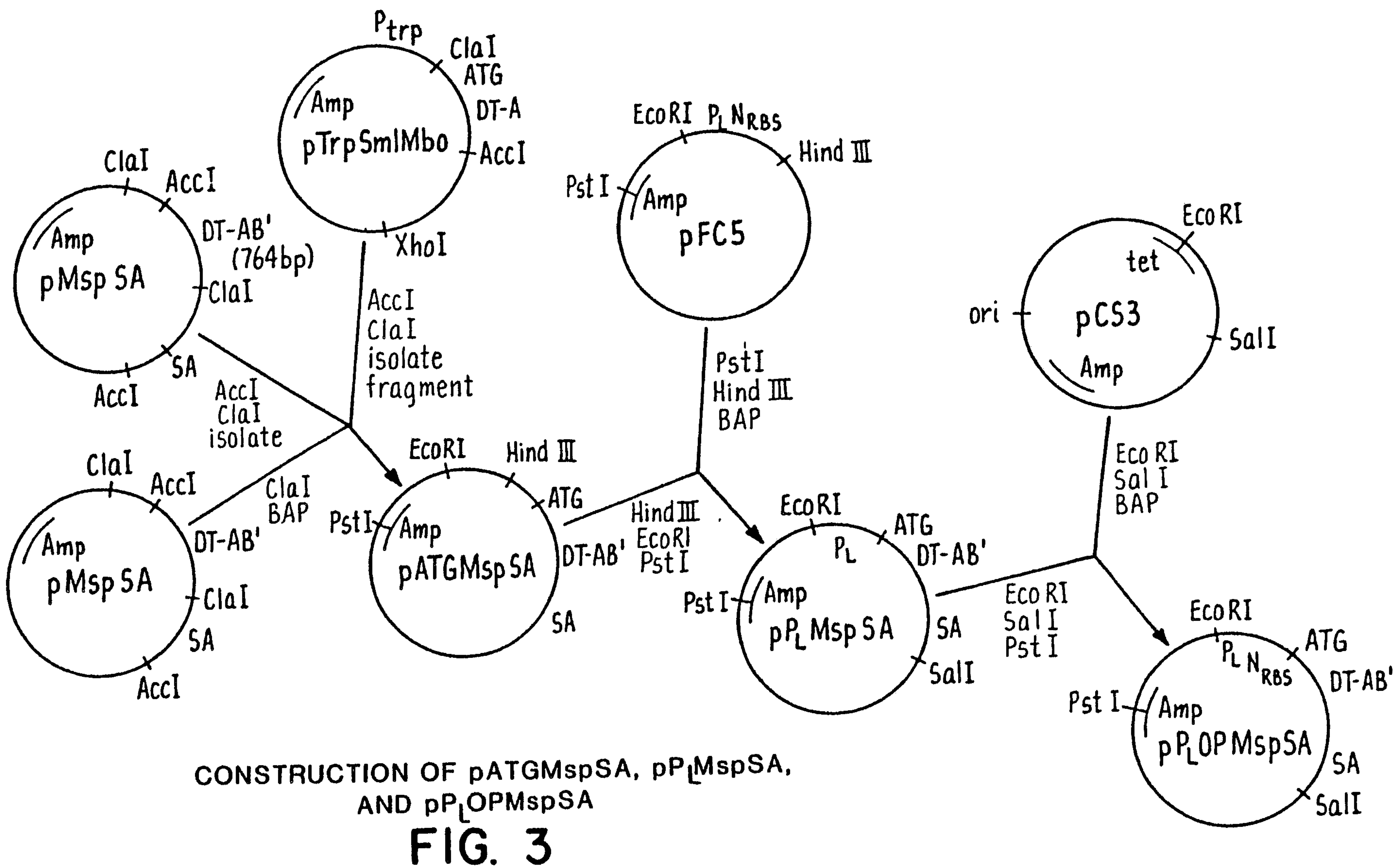


CONSTRUCTION OF pATGMspSA, $pP_LMspSA$, AND $pP_LOPMspSA$

FIG. 3

# FIG. 4

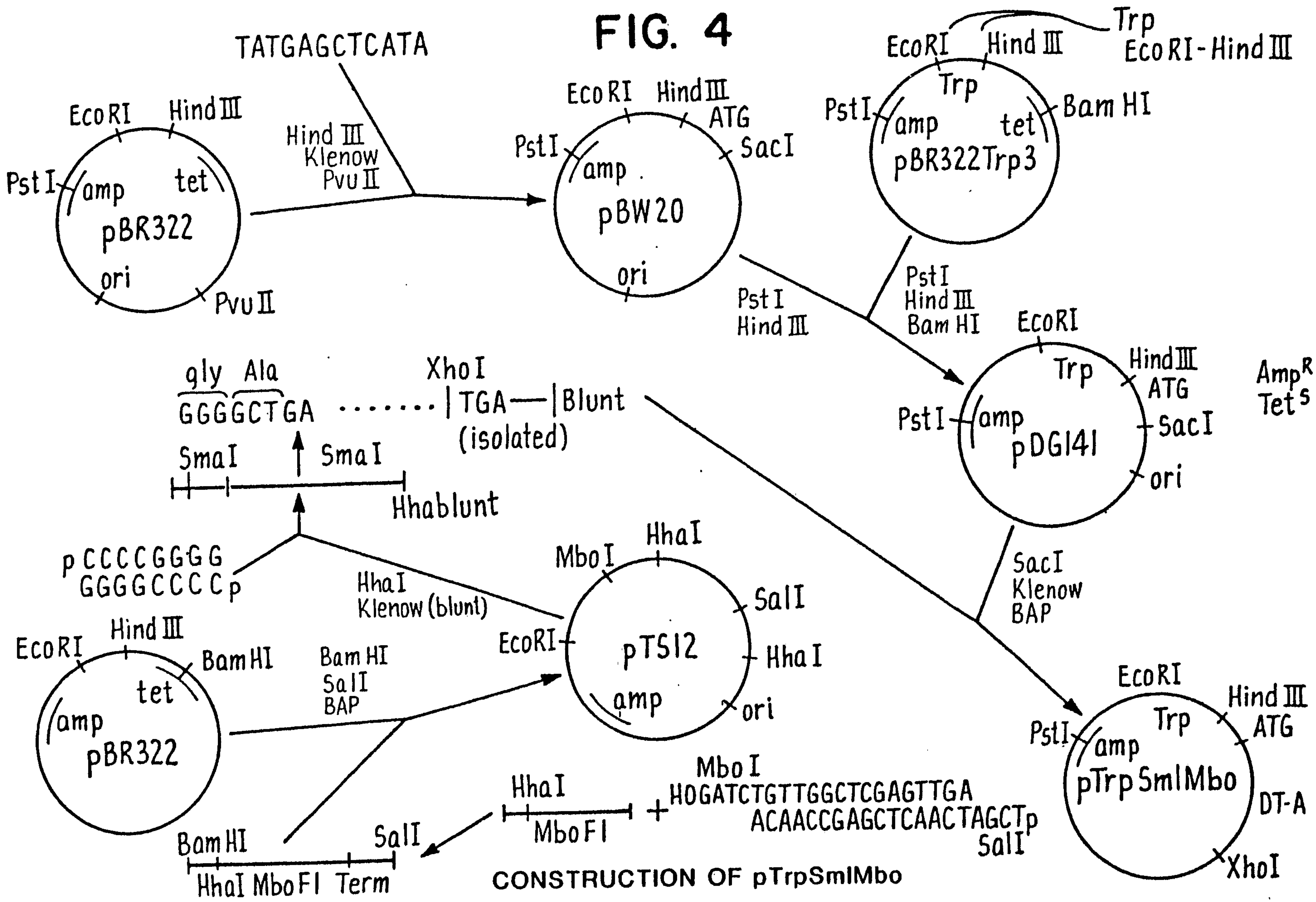


CONSTRUCTION OF pTrpSmlMbo

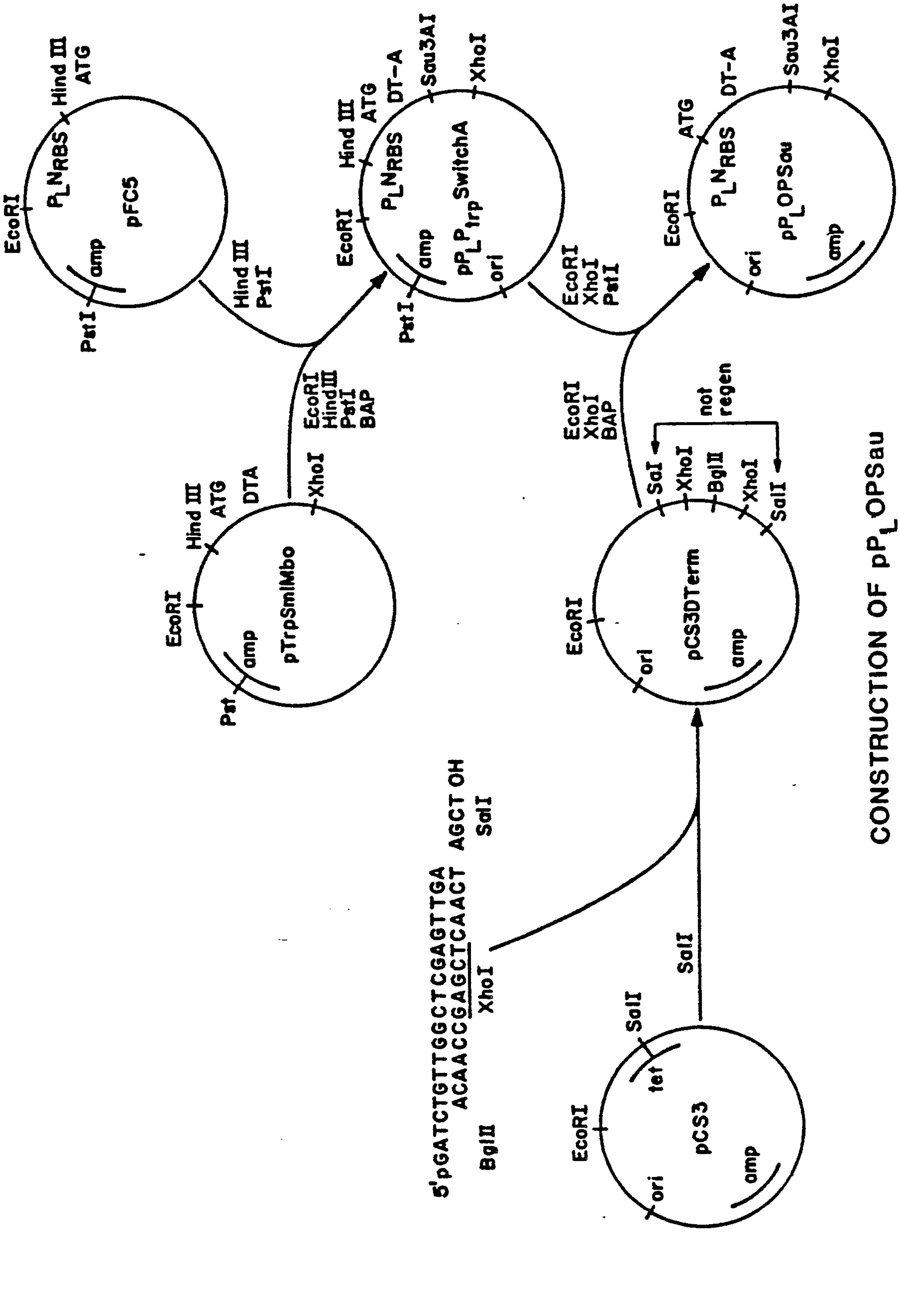


CONSTRUCTION OF $pP_LOPSau$

FIG. 5

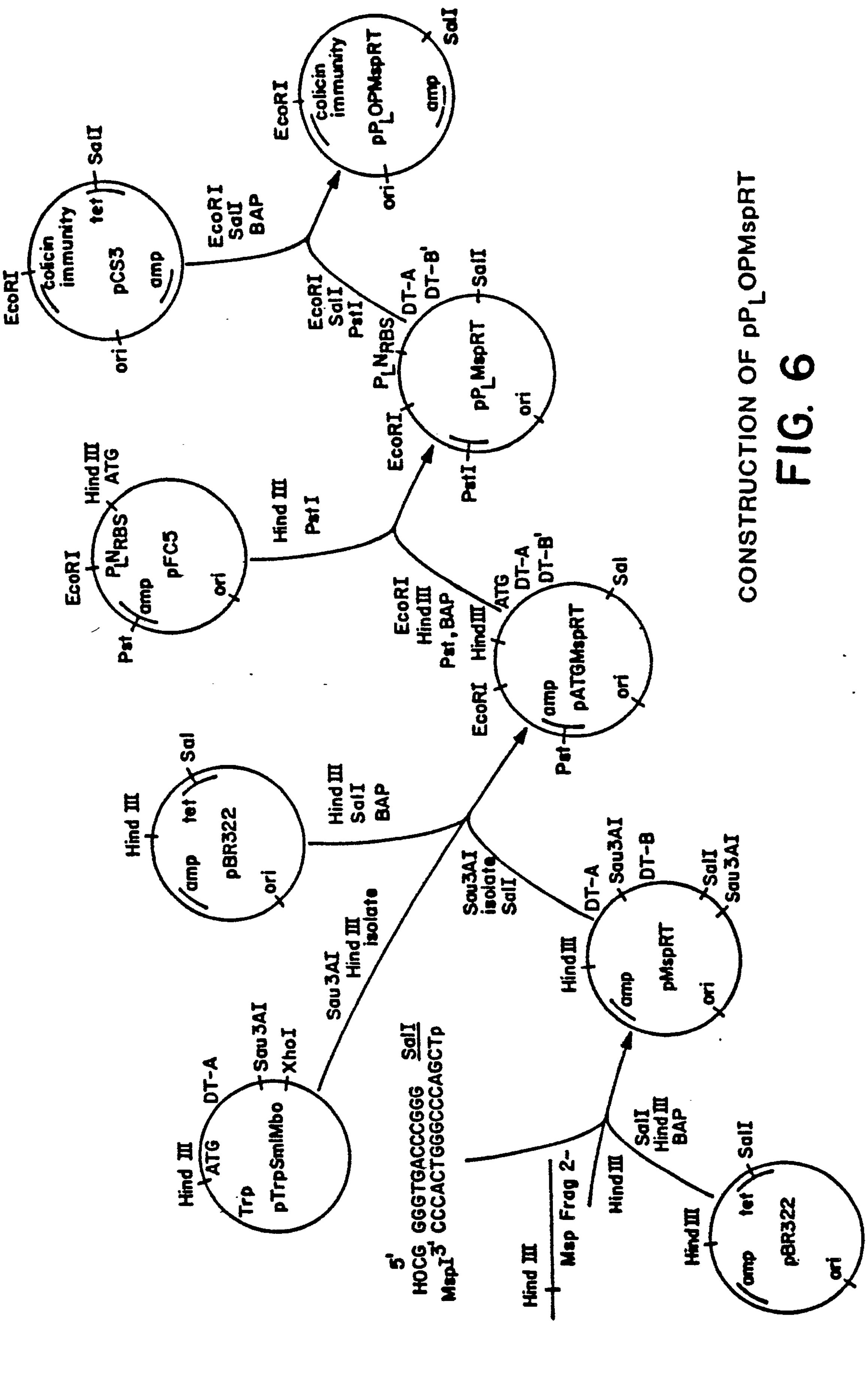


CONSTRUCTION OF pP$_L$OPMspRT

FIG. 6

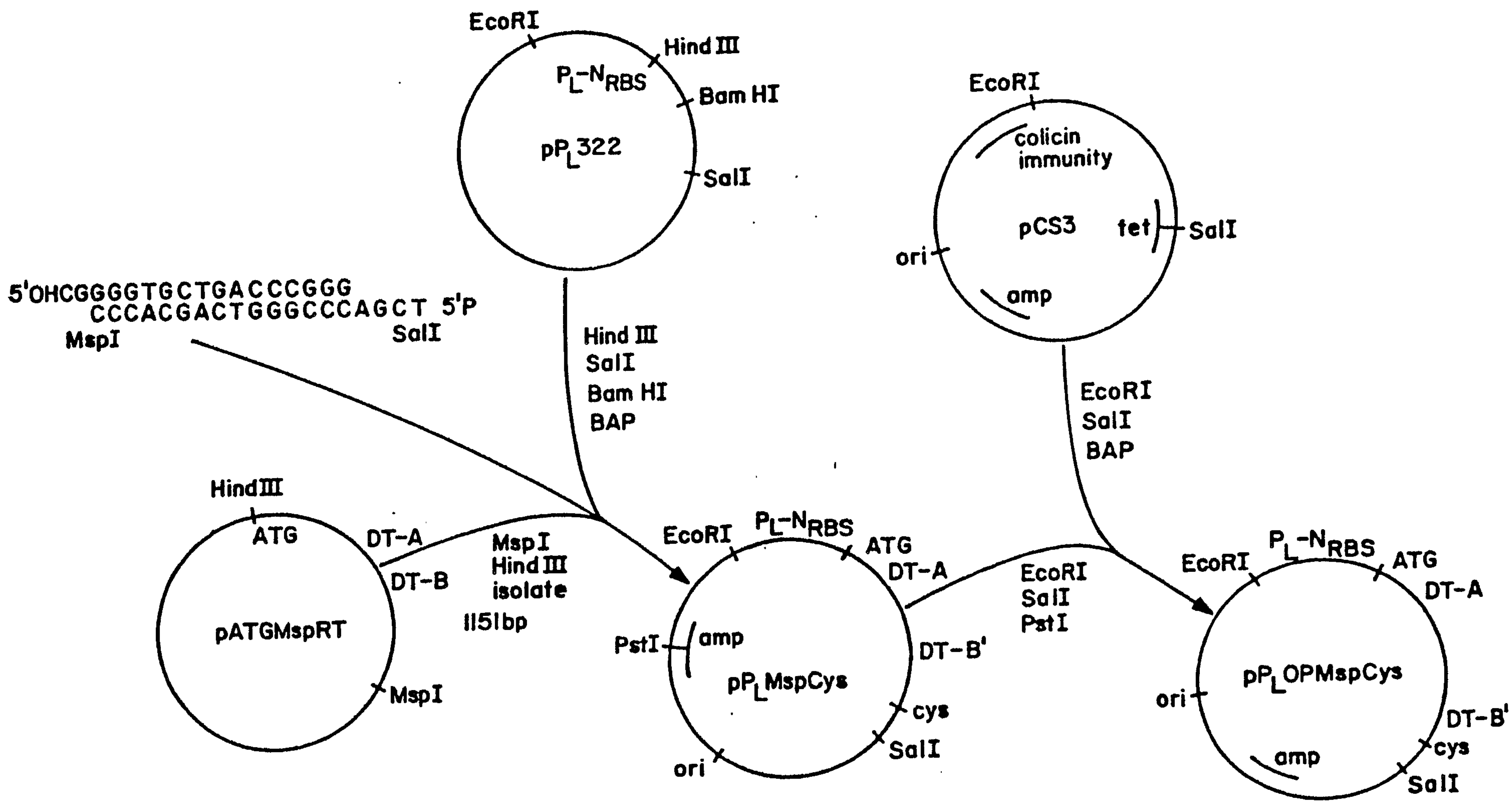


CONSTRUCTION OF $pP_L$OPMspCys

FIG. 7

FIG. 8

Results of Polyacrylamide Gel Electrophoresis of Extracts of Transformed Cells

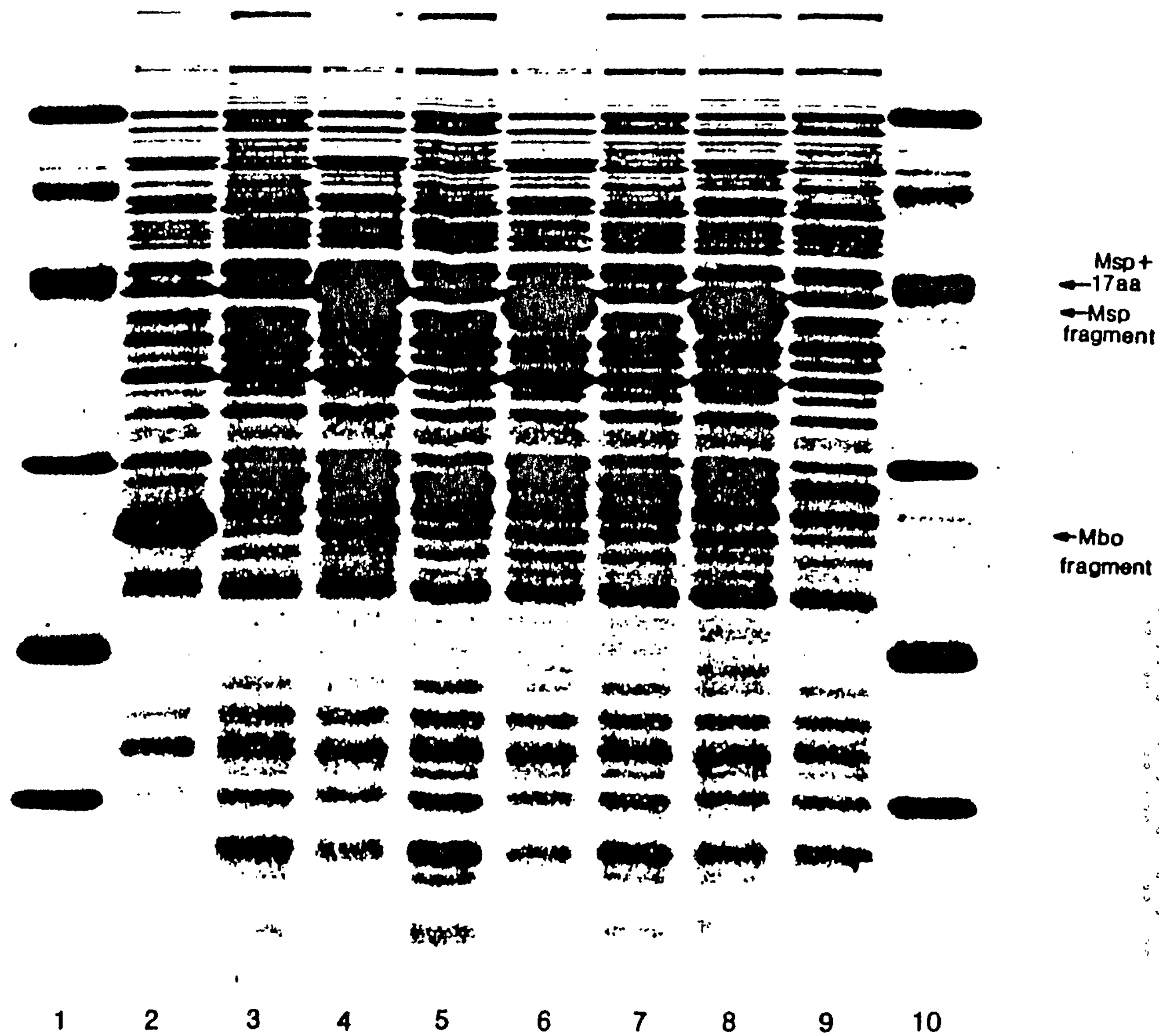


Lanes 2-9 represent extracts of MC1000-39531 cultures transformed with the plasmids of the invention as follows:

Lanes 2 and 3: $p^{P}_{L}$OPSau

Lanes 4 and 5: $p^{P}_{L}$OPMspSA

Lanes 6 and 7: $p^{P}_{L}$OPMspRT

Lanes 8 and 9: $p^{P}_{L}$OPMspCys

Lanes 2, 4, 6 and 8 are extracts of cells which were induced by an increase in medium temperature to 42° as described, Lanes 3, 5, 7 and 9 are extracts from uninduced cells.

Sequences of pRTA-115, pRTB-4 and pRTB-5 Cloned Inserts

115-TATTAATCCCTATCATAGCTCTCATGGTGTATAGATGCGCACCTCCACCGTCGTCACAGTT

115-TTCTTTGCTTATAAGGCCAGTGGTGCCAAATTTTAATGCTGATGTTTGTATGGATCCTGAGC
115 --(EcoRI)-- 4
115-CCATAGTGCGTATCGTAGGTCGAAATGGTCTATGTGTTGATGTTACAGGTGAAGAATTCTAC
5-gaattccGCGTATCGTAGGTCGAAATGGTCTATGTGTTGATGTTAGGGATGGAAGATTCCAC

4-GATGGAAACCCAATACAATTGTGGCCTTGCAAATCTAATACAGACTGGAATCAGTTATGGA
5-AACGGAAACGCAATACAGTTGTGGCCATGCAAGTCTAATACAGATGCAAATCAGCTCTGGA

4-CTTTGAGAAAAGACGGTACAATTCGATCTAATGGCAAGTGTTTGACCATTTATAAGTCCAG
5-CTTTGAAAAGAGACAATACTATTCGATCTAATGGAAAGTGTTTAACTACTTACGGGTACAG

4-TCTAGGAAAGCATGTGATGATATATAATTGTACTACCGCTACAGTTGGTGCCACCCGTTGG
5-TCCGGGAGTCTATGTGATGATCTATGATTGCAATACTGCTGCAACTGATGCCACCCGCTGG

4-CAAATATGGGACAACCGAACCATCATAAATCCCATATCTGGTTTAGTTTTGGCAGCCACAT
5-CAAATATGGGATAATGGAACCATCATAAATCCCAGATCTAGTCTAGTTTTAGCAGCGACAT

4-CAGGAAACAGTGGTACCACACTTACAGTGCAAACCAACATTTATGCCGTTAGTCAAGGTTG
5-CAGGCAACAGTGGTACCACACTTACAGTGCAAACCAACATTTATGCCGTTAGTCAAGGTTG

4-GCTTCCTAGTAATAATACACAACCTTTTGTGACATCCATTGTTGGGCTAAATGATCTCTGT
5-GCTTCCTACTAATAATACACAACCTTTTGTGACAACCATTGTTGGGCTATACGGTCTGTGC

4-TTACAAGCAAATACTGGAAAAGTATGGTTAGACGAGTGTACAAGTGAAAAGGCTGAACAAC
5-TTGCAAGCAAATAGTGGACAAGTATGGATAGAGGACTGTAGCAGTGAAAAGGCTGAACAAC

4-AATGGGCGCTTTATGCAGATGGTTCAATACGGCCTCAGCAAAACCAAGATAACTGCCTTAC
5-AGTGGGCTCTTTATGCAGATGGTTCAATACGTCCTCAGCAAAACCGAGATAATTGCCTTAC

4-AAGTGATGCTAATATACGAGAAACAATTGTCAAGACCCTCTCTTGCAGCACTGCATCCTCC
5-AAGTGATTCTAATATACGGGAAACAGTTGTCAAGATCCTCTCTTGTGGCCCTGCATCCTCT

4-GGCCAGCGATGGATGTTCAAGAATGATGGAACCATTTGGAATTTGTATAATGGATTGGTGT
5-GGCCAACGATGGATGTTCAAGAATGATGGAACCATTTTAAATTTGTATAGTGGGTTGGTGT

4-TAGATGTGAAGCGATCGGATCCGACCCTTAAACAAATCATTATTTACCCTTTCCATGGAAA
5-TAGATGTGAGGGCATCGGATCCGAGCCTTAAACAAATCATTCTTTACCCTCTCCATGGTGA

4-CCCAAACCAAATATGGTTTCCACTATTTTGATAGACTAATTACCCTCTTGCAGTGTATGTA
5-CCCAAACCAAATATGGTTACCATTATTTTGATAGACAGATTACTCTCTTGCAGTGTGTATG

4-TGTCCTACCATGAACATAGTTG CTTAAATAAAAAGGACATTGTAAATTAAAAAAA...
5- TCCTGCCATGAAAATAGATGGCTTAAATAAAAAGGACATTGTAAATTTTGTAACTGAAA

5-GGACAGCAAGTTATTGCAGTCCAGTATCTAATAAGAGCACAACTATTGTCTTGTGCAAAAAA...

FIG. 9

# FIG. 10

Comparison of Ricin-B Sequence with RTB-5

```
R-AlaAspValCysMetAspProGluProIleValArgIleValGlyArgAsnGlyLeuCys
                                     ArgIleValGlyArgAsnGlyLeuCys
                                gaattccGCGTATCGTAGGTCGAAATGGTCTATGT

R-ValAsnValArgAspGlyArgPheAsnHisGlyAsnAlaIleGlnLeuTrpProCysLys
  ValAspValArgAspGlyArgPheHisAsnGlyAsnAlaIleGlnLeuTrpProCysLys
  GTTGATGTTAGGGATGGAAGATTCCACAACGGAAACGCAATACAGTTGTGGCCATGCAAG

R-SerAsnThrAspAlaAsnGlnLeu   ThrLeuLysArgAspAsnThrIleArgSerAsn
  SerAsnThrAspAlaAsnGlnLeuTrpThrLeuLysArgAspAsnThrIleArgSerAsn
  TCTAATACAGATGCAAATCAGCTCTGGACTTTGAAAAGAGACAATACTATTCGATCTAAT

R-GlyLysCysLeuThrThrTyrGlyTyrProSerGlyValTyrValMetIleTyrAspCys
  GlyLysCysLeuThrThrTyrGlyTyrSerProGlyValTyrValMetIleTyrAspCys
  GGAAAGTGTTTAACTACTTACGGGTACAGTCCGGGAGTCTATGTGATGATCTATGATTGC

R-AsnThrAlaAlaThrThrAlaAspArg   GluIleTrpAsnAsnGlyThrIleIleAsnPro
  AsnThrAlaAlaThrAspAlaThrArgTrpGlnIleTrpAspAsnGlyThrIleIleAsnPro
  AATACTGCTGCAACTGATGCCACCCGCTGGCAAATATGGGATAATGGAACCATCATAAATCCC

R-ArgSerSerLeuValLeuAlaAlaThrSerGlyAsnSerGlyThrThrLeuThrValGlnThr
  ArgSerGlyLeuValLeuAlaAlaThrSerGlyAsnSerGlyThrThrLeuThrValGlnThr
  AGATCTAGTCTAGTTTTAGCAGCGACATCAGGCAACAGTGGTACCACACTTACAGTGCAAACC

R-AsnIleTyrAlaValSerGlnGlyProLeuPheThrAsnAsnThrGlnProTrpValThr
  AsnIleTyrAlaValSerGlnGlyTrpLeuProThrAsnAsnThrGlnProPheValThr
  AACATTTATGCCGTTAGTCAAGGTTGGCTTCCTACTAATAATACACAACCTTTTGTGACA

R-ThrIleValGlyLeuTyrGlyLeuCysLeuGlnAlaAsnSerGlyGlnValValIleGlu
  ThrIleValGlyLeuTyrGlyLeuCysLeuGlnAlaAsnSerGlyGlnValTrpIleGlu
  ACCATTGTTGGGCTATACGGTCTGTGCTTGCAAGCAAATAGTGGACAAGTATGGATAGAG

R-AspSerCysSerGluLysAlaGluGlnGlnTrpAlaLeuTyrAlaSerGlyAsnIleAsn
  AspCysSerSerGluLysAlaGluGlnGlnTrpAlaLeuTyrAlaAspGlySerIleArg
  GACTGTAGCAGTGAAAAGGCTGAACAACAGTGGGCTCTTTATGCAGATGGTTCAATACGT

R-ProGlnGlnArgArgAspAsnCysLeuThrSerAspSerAsnIleArgGluThrValVal
  ProGlnGlnAsnArgAspAsnCysLeuThrSerAspSerAsnIleArgGluThrValVal
  CCTCAGCAAAACCGAGATAATTGCCTTACAAGTGATTCTAATATACGGGAAACAGTTGTC

R-LysIleLeuSerCysGlyProAlaSerSerGlyGluArgTrpMetPheLysAsnAspGly
  LysIleLeuSerCysGlyProAlaSerSerGlyGlnArgTrpMetPheLysAsnAspGly
  AAGATCCTCTCTTGTGGCCCTGCATCCTCTGGCCAACGATGGATGTTCAAGAATGATGGA

R-ThrIleLeuAsnLeuTyrSerGlyLeuValLeuAspValArgAlaSerAspProSerLeu
  ThrIleLeuAsnLeuTyrSerGlyLeuValLeuAspValArgAlaSerAspProSerLeu
  ACCATTTTAAATTTGTATAGTGGGTTGGTGTTAGATGTGAGGGCATCGGATCCGAGCCTT

R-LysGlnIleIleLeuTyrProLeuTrpGlyHisAspProAsnGlnLeu   IleLeuProPhe
  LysGlnIleIleLeuTyrProLeu   HisGlyAspProAsnGlnIleTrpLeuProLeuPheTerTer
  AAACAAATCATTCTTTACCCTCTC   CATGGTGACCCAAACCAAATATGGTTACCATTATTTTGATAG

  ACAGATTACTCTCTTGCAGTGTGTATGTCCTGCCATGAAAATAGATGGCTTAAATAAAAA
  GGACATTGTAAATTTTGTAACTGAAAGGACAGCAAGTTATTGCAGTCCAGTATCTAATAA
  GAGCACAACTATTGTCTTGTGCAAAAAA....
```

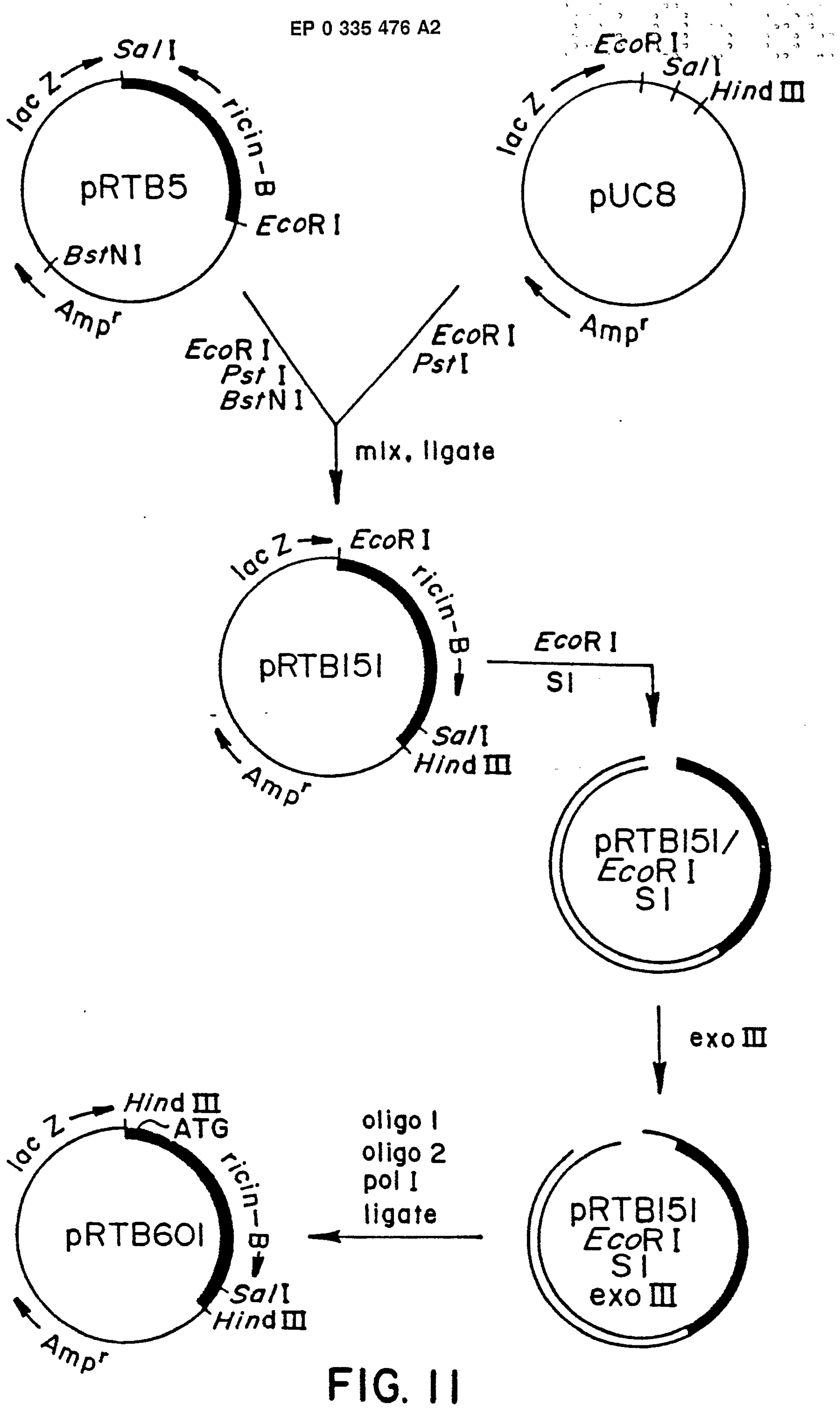
lac Z
Sal I
ricin-B
pRTB5
EcoR I
BstN I
Amp^r
lac Z
EcoR I
Sal I
Hind III
pUC8
Amp^r
EcoR I
Pst I
BstN I
EcoR I
Pst I
mix, ligate
lac Z
EcoR I
ricin-B
pRTB151
Sal I
Hind III
Amp^r
EcoR I
S1
pRTB151/
EcoR I
S1
exo III
lac Z
Hind III
ATG
ricin-B
pRTB601
Sal I
Hind III
Amp^r
oligo 1
oligo 2
pol I
ligate
pRTB151
EcoR I
S1
exo III

FIG. 11

FIG. 12a

Junction Region for the Construction of pRTB601

```
                                                      |-----   pRTB-151   --->
          Hind III |------------- ricin-B ------------------------------->
               MetAlaAspValCysMetAspProGluProIleValArgIleValGlyArgAsnGlyLeu...
GACCATGATAAGCTTATGGCTGATGTTTGTATGGATCC            GCGTATCGTAGGTCGAAATGGTCTA...
                              TACCTAGGACTCGGGTATCACGCATAGCATCC        ACCAGAT...
          (oligo 2)                          (oligo 1)
```

FIG. 12b

Fusion of lacZ and Ricin-B in pRTB236

```
  <-----  lacZ  ----------|  |----------  ricin-B  ---------------->
              MetThrMetIleThrProArgIleValGlyArgAsnGlyLeuCysValAspValArgAsp...
...CAGGAAACAGCTATGACCATGATTACGCCGCGTATCGTAGGTCGAAATGGTCTATGTGTTGATGTTAGGGAT...
  (RBS)
```

FIG. 12c

Junction Region in pRTB514

```
                                  |----------  ricin-B  ---------------->
  <-----   pDG141   -------||-------------  pRTB601  ------------------>
                                MetAlaAspValCysMetAspProGluProIleValArgIle...
...AGTTCACGTAAAAAGGGTATCGATAAGCTTATGGCTGATGTTTGTATGGATCCTGAGCCCATAGTGCGTATC...
               (RBS)     Hind III
```

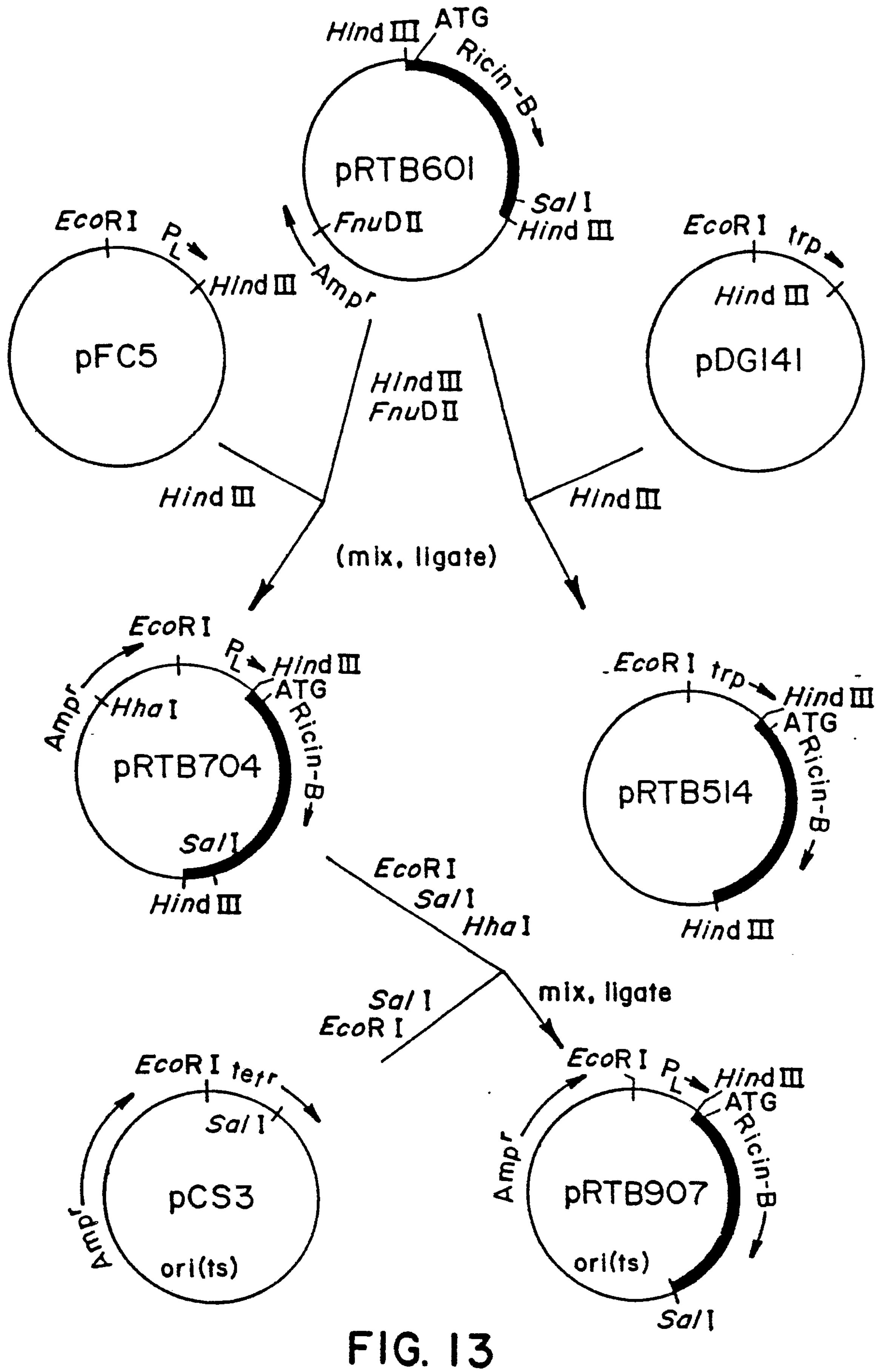
Hind III
ATG
Ricin-B
pRTB601
Sal I
Hind III
FnuD II
Amp^r
EcoR I
P_L
Hind III
pFC5
EcoR I
trp
Hind III
pDG141
Hind III
FnuD II
Hind III
Hind III
(mix, ligate)
EcoR I
P_L
Hind III
ATG
Amp^r
Hha I
Ricin-B
pRTB704
Sal I
Hind III
EcoR I
trp
Hind III
ATG
Ricin-B
pRTB514
Hind III
EcoR I
Sal I
Hha I
Sal I
EcoR I
mix, ligate
EcoR I
tet^r
Sal I
Amp^r
pCS3
ori(ts)
EcoR I
P_L
Hind III
ATG
Ricin-B
Amp^r
pRTB907
ori(ts)
Sal I

FIG. 13

```
                                          (HindIII)
                          (1) ccaagaattgctgcaaaagcttatgaaaccggg
TCTTCCTCAGCTGCTCACTTTCCAATAAAATTCCAAGAATTGCTGCAATCAAAGATGAAACCGGGAGGAAATACT
                                                         METLysProGlyGlyAsnThr

                                             BamH1          (2) ctttcacattagag
ATTGTAATATGGATGTATGCAGTGGCAACATGGCTTTGTTTTGGATCCACCTCAGGGTGGTCTTTCACATTAGAG
IleValIleTrpMETTyrAlaValAlaThrTrpLeuCysPheGlySerThrSerGlyTrpSerPheThrLeuGlu
(HindIIIMET)                                         --- <------(leader)<-----
aagcttatgatattccccaaac
GATAACAACATATTCCCCAAACAATACCCAATTATAAACTTTACCACAGCGGGTGCCACTGTGCAAAGCTACACA
AspAsnAsnIlePheProLysGlnTyrProIleIleAsnPheThrThrAlaGlyAlaThrValGlnSerTyrThr
RTA-<-------IlePheProLysGlnTyrProIleIleAsnPheThrThrAlaGlyAlaThrValGlnSerTyrThr

AACTTTATCAGAGCTGTTCGCGGTCGTTTAACAACTGGAGCTGATGTGAGACATGAAATACCAGTGTTGCCAAAC
AsnPheIleArgAlaValArgGlyArgLeuThrThrGlyAlaAspValArgHisGluIleProValLeuProAsn
RTA-AsnPheIleArgAlaValArgGlyArgLeuThrThrGlyAlaAspValArgHisGluIleProValLeuProAsn

AGAGTTGGTTTGCCTATAAACCAACGGTTTATTTTAGTTGAACTCTCAAATCATGCAGAGCTTTCTGTTACATTA
ArgValGlyLeuProIleAsnGlnArgPheIleLeuValGluLeuSerAsnHisAlaGluLeuSerValThrLeu
RTA-ArgValGlyLeuProIleAsnGlnArgPheIleLeuValGluLeuGlnAsnHisAlaGluIleSerValThrLeu

GCGCTGGATGTCACCAATGCATATGTGGTAGGCTACCGTGCTGGAAATAGCGCATATTTCTTTCATCCTGACAAT
AlaLeuAspValThrAsnAlaTyrValValGlyTyrArgAlaGlyAsnSerAlaTyrPhePheHisProAspAsn
RTA-AlaLeuSerValThrAsnAlaTyrValValGlyTyrArgAlaGlyAsnSerAlaTyrPhePheHisProAspAsn

CAGGAAGATGCAGAAGCAATCACTCATCTTTTCACTGATGTTCAAAATCGATATACATTCGCCTTTGGTGGTAAT
GlnGluAspAlaGluAlaIleThrHisLeuPheThrAspValGlnAsnArgTyrThrPheAlaPheGlyGlyAsn
RTA-GlnGluAspAlaGluAlaIleThrHisLeuPheThrAspValGlnAsnArgTyrThrPheAlaPheGlyGlyAsn

TATGATAGACTTGAACAACTTGCTGGTAATCTGAGAGAAAATATCGAGTTGGGAAATGGTCCACTAGAGGAGGCT
TyrAspArgLeuGluGlnLeuAlaGlyAsnLeuArgGluAsnIleGluLeuGlyAsnGlyProLeuGluGluAla
RTA-TyrAspArgLeuGluGlnLeuAlaGlyAsnLeuArgGluAsnIleGluLeuGlyAsnGlyProLeuGluGluAla

ATCTCAGCGCTTTATTATTACAGTACTGGTGGCACTCAGCTTCCAACTCTGGCTCGTTCCTTTATAATTTGCATC
IleSerAlaLeuTyrTyrTyrSerThrGlyGlyThrGlnLeuProThrLeuAlaArgSerPheIleIleCysIle
RTA-IleSerAlaLeuTyrTyrTyrSerThrGlyGlyThrGlnLeuProThrLeuAlaArgSerPheIleIleCysIle

CAAATGATTTCAGAAGCAGCAAGATTCCAATATATTGAGGGAGAAATGCGCACGAGAATTAGGTACAACCGGAGA
GlnMETIleSerGluAlaAlaArgPheGlnTyrIleGluGlyGluMETArgThrArgIleArgTyrAsnArgArg
RTA-GlnMetIleSerGluAlaAlaArgPheGlnTyrIleGluGlyGluMetArgThrArgIleArgTyrAsnArgArg

TCTGCACCAGATCCTAGCGTAATTACACTTGAGAATAGTTGGGGGAGACTTTCCACTGCAATTCAAGAGTCTAAC
SerAlaProAspProSerValIleThrLeuGluAsnSerTrpGlyArgLeuSerThrAlaIleGlnGluSerAsn
RTA-SerAlaProAspProSerValIleThrLeuGluAsnSerTrpGlyArgLeuSerThrAlaIleGlnGluSerAsn

CAAGGAGCCTTTGCTAGTCCAATTCAACTGCAAAGACGTAATGGTTCCAAATTCAGTGTGTACGATGTGAGTATA
GlnGlyAlaPheAlaSerProIleGlnLeuGlnArgArgAsnGlySerLysPheSerValTyrAspValSerIle
RTA-GlnGlyAlaPheAlaSerProIleGlnLeuGlnArg   AspGlySerLysPheSerValTyrAspValSerIle
                                                                   (TER)
                                                   (3) cacagttttaattgcttataagg
TTAATCCCTATCATAGCTCTCATGGTGTATAGATGCGCACCTCCACCATCGTCACAGTTTTCTTTGCTTATAAGG
LeuIleProIleIleAlaLeuMETValTyrArgCysAlaProProProSerSerGlnPheSerLeuLeuIleArg
RTA-LeuLeuProIleIleAla   MetValTyrArgCysAlaProProProSerSerGlnPhe(<--A-chain)

                                           BamH1
CCAGTGGTACCAAATTTTAATGCTGATGTTTGTATGGATCCTGAGCCCATAGTGCGTATCGTAGGTCGAAATGGT
ProValValProAsnPheAsnAlaAspValCysMETAspProGluProIleValArgIleValGlyArgAsnGly
RTB-        (B-chain-> )AlaAspValCysMetAspProGluProIleValArgIleValGlyArgAsnGly
```

FIG. 14

## Estimated Ricin Agglutinin A Sequence

```
RTA-IlePheProLysGlnTyrProIleIleAsnPheThrThrAlaGlyAlaThrValGlnSerTyrThrAsnPheIle

        CTGTGCGCAGTCATTTAACAACTGGAGGTGATGTGAGACATGAAATACCAGTGTTGCCAAACAGAGTTGGT
          ValArgSerHisLeuThrThrGlyGlyAspValArgHisGluIleProValLeuProAsnArgValGly
RTA-ArgAlaValArgGlyArgLeuThrThrGlyAlaAspValArgHisGluIleProValLeuProAsnArgValGly

    TTGCCTATAAGCCAACGGTTTATTTTAGTTGAACTCTCAAATCATGCAGAGCTTTCTGTTACATTAGCACTGGAT
    LeuProIleSerGlnArgPheIleLeuValGluLeuSerAsnHisAlaGluLeuSerValThrLeuAlaLeuAsp
RTA-LeuProIleAsnGlnArgPheIleLeuValGluLeuGlnAsnHisAlaGluIleSerValThrLeuAlaLeuSer

    GTCACCAATGCATATGTGGTCGGCTGCCGCGCTGGAAATAGCGCCTATTTCTTTCATCCTGACAATCAAGAAGAT
    ValThrAsnAlaTyrValValGlyCysArgAlaGlyAsnSerAlaTyrPhePheHisProAspAsnGlnGluAsp
RTA-ValThrAsnAlaTyrValValGlyTyrArgAlaGlyAsnSerAlaTyrPhePheHisProAspAsnGlnGluAsp

    GCAGAAGCAATCACTCATCTTTTCACGGATGTTCAAATT????????????GCTTTTGGTGGTAATTATGATAGA
    AlaGluAlaIleThrHisLeuPheThrAspValGlnIle????????????AlaPheGlyGlyAsnTyrAspArg
RTA-AlaGluAlaIleThrHisLeuPheThrAspValGlnAsnArgTyrThrPheAlaPheGlyGlyAsnTyrAspArg

    CTTGAACAACTTG?AGGT   CTTGAGAGAAATATTGAGTTGGGAACTGGTCCATTAGAGGACGCTATCTCAGCG
    LeuGluGlnLeu???Gly   LeuGluArgAsnIleGluLeuGlyThrGlyProLeuGluAspAlaIleSerAla
RTA-LeuGluGlnLeuAlaGlyAsnLeuArgGluAsnIleGluLeuGlyAsnGlyProLeuGluGluAlaIleSerAla

    CTTTATTATTATAGTACTTGTGGCACTCAGATTCCAACTCTGGCTCGTTCCTTTATGGTTTGCATCCAAATGATT
    LeuTyrTyrTyrSerThrCysGlyThrGlnIleProThrLeuAlaArgSerPheMETValCysIleGlnMETIle
RTA-LeuTyrTyrTyrSerThrGlyGlyThrGlnLeuProThrLeuAlaArgSerPheIleIleCysIleGlnMetIle

    TCAGAAGCAGCAAGATTCCAGTACATTGAGGGAGAAATGCGCACGAGAATTAGGTACAACCGAAGATCTGCACCA
    SerGluAlaAlaArgPheGlnTyrIleGluGlyGluMETArgThrArgIleArgTyrAsnArgArgSerAlaPro
RTA-SerGluAlaAlaArgPheGlnTyrIleGluGlyGluMetArgThrArgIleArgTyrAsnArgArgSerAlaPro

    GATCCTAGCGTAATTACACTTGAGAATAGTTGGGGGAGACTTTCCACTGCAATTCAAGAGTCTAACCAAGGAGCC
    AspProSerValIleThrLeuGluAsnSerTrpGlyArgLeuSerThrAlaIleGlnGluSerAsnGlnGlyAla
RTA-AspProSerValIleThrLeuGluAsnSerTrpGlyArgLeuSerThrAlaIleGlnGluSerAsnGlnGlyAla

    TTTGCTAGTCCAATTCAACTGCAAAGACGTAACGGTTCCAAATTCAATGTGTACGATGTGAGTATATTAATCCCT
    PheAlaSerProIleGlnLeuGlnArgArgAsnGlySerLysPheAsnValTyrAspValSerIleLeuIlePro
RTA-PheAlaSerProIleGlnLeuGlnArg   AspGlySerLysPheSerValTyrAspValSerIleLeuLeuPro

    ATCATAGCTCTCATGGTGTATAGATGCGCACCTCCACCGTCGTCACAGTTTTCTTTGCTTATAAGGCCAGTGGTG
    IleIleAlaLeuMETValTyrArgCysAlaProProProSerSerGlnPheSerLeuLeuIleArgProValVal
RTA-IleIleAla   MetValTyrArgCysAlaProProProSerSerGlnPhe
                                                (A chain <- )

    CCAAATTTTAATGCTGATGTTTGTATGGATCCTGAGCCCATAGTGCGTATCGTAGGTCGAAATGGTCTATGTGTT
    ProAsnPheAsnAlaAspValCysMETAspProGluProIleValArgIleValGlyArgAsnGlyLeuCysVal
RTA-            AlaAspValCysMetAspProGluProIleValArgIleValGlyArgAsnGlyLeuCysVal
               ( -> B chain)

    GATGTTACAGGTGAAGAATTC
    AspValThrGlyGluGluPhe
RTB-AsnValArgAspGlyArgPhe
```

This is a composite derived from the inserts of pRTA115 and pRA45.
? = undetermined sequence

FIG. 15

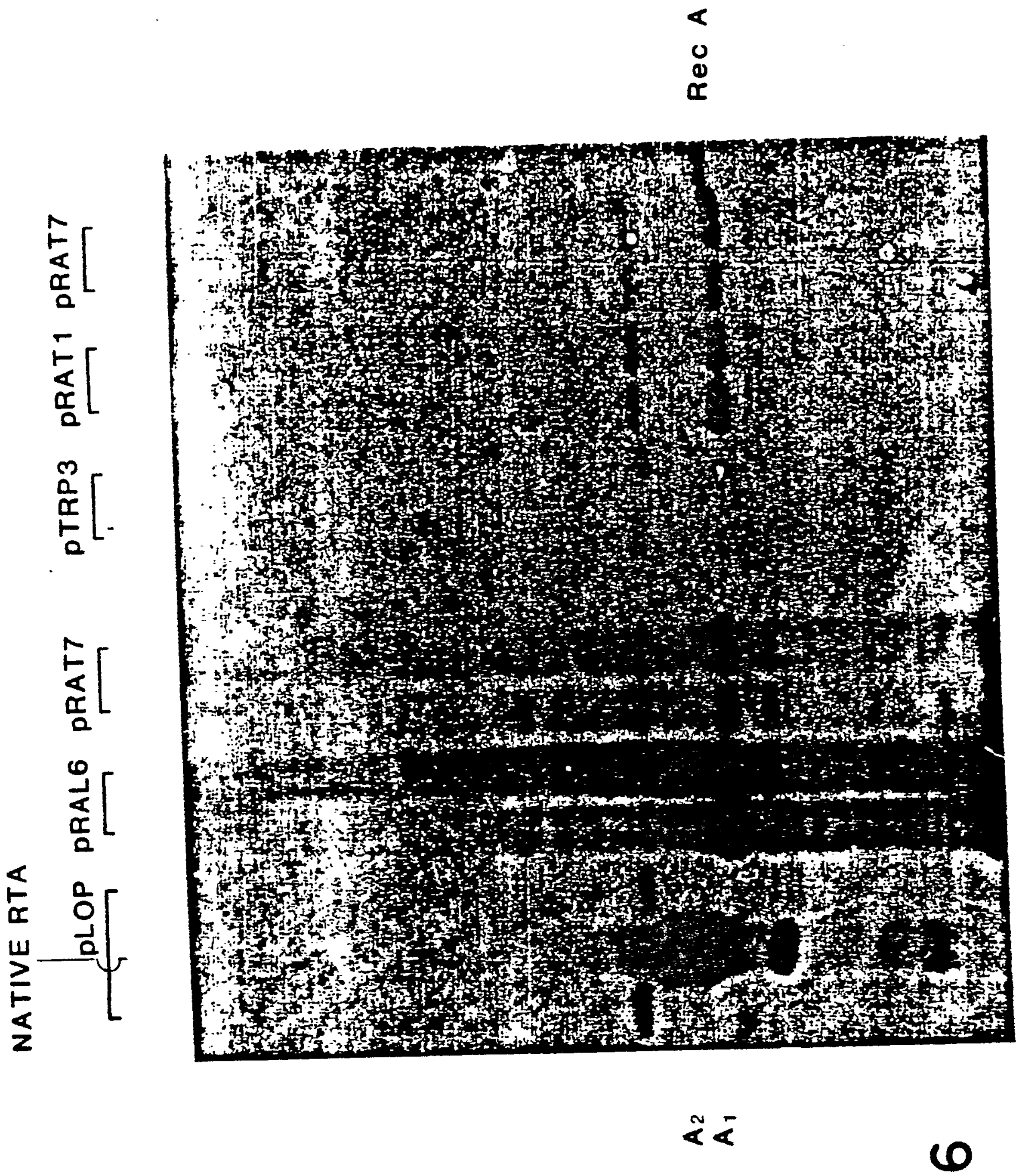


FIG. 16

# FIG. 17

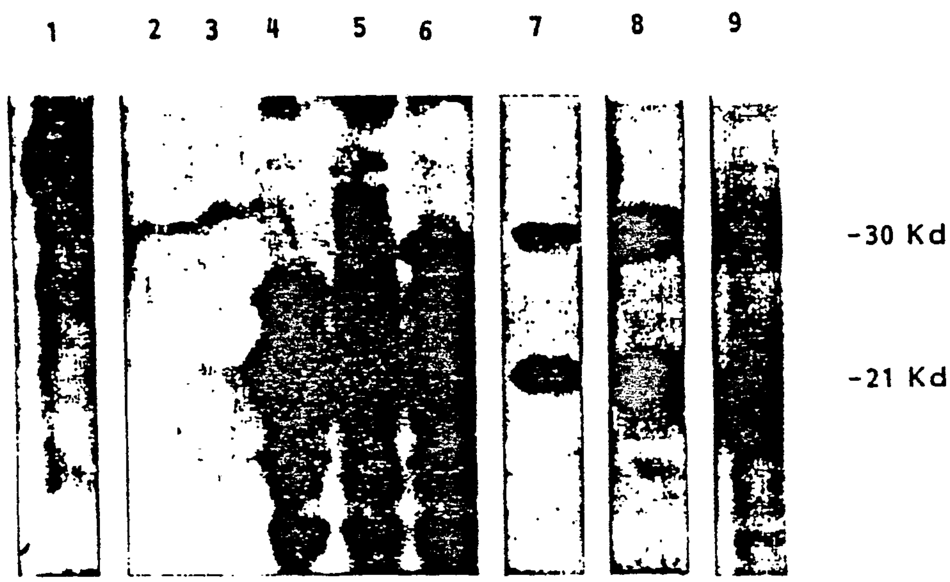


Key

1 natural ricin-B

2 pUC8

3 pRTB5

4 pRTB151

5 pRTB221

6 pRTB236

7 pRTB514

8 pRTB704

9 pRTB907